(19) Europäisches Patentamt
European Patent Office
Office européen des brevets

(11) EP 4 076 410 B1

(12) EUROPEAN PATENT SPECIFICATION

(45) Date of publication and mention of the grant of the patent:
**10.09.2025 Bulletin 2025/37**

(21) Application number: **20901388.7**

(22) Date of filing: **18.12.2020**

(51) International Patent Classification (IPC):
***A61K 31/135** (2006.01)* ***A61K 9/00** (2006.01)*
***A61P 25/24** (2006.01)* ***A61K 47/02** (2006.01)*
***A61K 47/10** (2017.01)* ***A61K 47/26** (2006.01)*
***A61K 47/34** (2017.01)* ***A61K 47/38** (2006.01)*

(52) Cooperative Patent Classification (CPC):
**A61K 9/0019; A61K 31/135; A61K 47/02; A61K 47/10; A61K 47/26; A61K 47/34; A61K 47/38; A61P 25/24**

(86) International application number:
**PCT/CN2020/137496**

(87) International publication number:
**WO 2021/121366 (24.06.2021 Gazette 2021/25)**

(54) **LONG-ACTING INJECTABLE FORMULATIONS OF KETAMINE PAMOATE SALTS**

LANGWIRKENDE INJIZIERBARE FORMULIERUNGEN VON KETAMINPAMOATSALZEN

FORMULATIONS INJECTABLES À ACTION PROLONGÉE DE SELS DE PAMOATE DE KÉTAMINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.12.2019 US 201962951061 P**

(43) Date of publication of application:
**26.10.2022 Bulletin 2022/43**

(73) Proprietor: **Alar Pharmaceuticals Inc.**
**Taichung City, Taiwan 40763 (TW)**

(72) Inventors:
- **LIN, Tong-Ho**
  **Taichung City 40763 (TW)**
- **WEN, Yung-Shun**
  **Taichung City 40763 (TW)**
- **CHEN, Chia-Hsien**
  **Taichung City 40763 (TW)**
- **LIU, Ying-Ting**
  **Taichung City 40763 (TW)**
- **HOU, Rui-Zhi**
  **Taichung City 40763 (TW)**
- **WU, Zhi-Rong**
  **Taichung City 40763 (TW)**

(74) Representative: **Papula Oy**
**P.O. Box 981**
**00101 Helsinki (FI)**

(56) References cited:
**WO-A1-2018/122626 WO-A1-2019/073408**
**WO-A1-2019/172920 WO-A1-2019/186357**
**WO-A1-2020/143762 US-A1- 2014 275 276**

- **SOTA HIRANO, MICHELE BOVI, ALESSANDRO ROMEO, FLAVIA GUZZO, CRISTIANO CHIAMULERA & MASSIMILIANO PERDUCA: "Ketamine nano-delivery based on poly-lactic-co-glycolic acid (PLGA) nanoparticles", APPLIED NANOSCIENCE, vol. 8, 11 April 2018 (2018-04-11), pages 655 - 663, XP009528594, ISSN: 2190-5509, DOI: 10.1007/s13204-018-0765-1**
- **LAEL REINSTATLER , NAGY A. YOUSSEF: "Ketamine as a Potential Treatment for Suicidal Ideation: A Systematic Review of the Literature.", DRUGS IN R&D, vol. 15, no. 1, 10 February 2015 (2015-02-10), pages 37 - 43, XP055822524, ISSN: 1174-5886, DOI: 10.1007/s40268-015-0081-0**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 076 410 B1

## Description

TECHNICAL FIELD

The present disclosure relates generally to formulations of ketamine pamoate salts. In particular, the present disclosure relates to a sustained-release pharmaceutical composition comprising *R, S*-ketamine pamoate salt, *S*-ketamine pamoate salt or *R*-ketamine pamoate salt, and its uses in anesthesia, analgesia, or treating anti-inflammatory and central nervous system diseases.

BACKGROUND

Ketamine, 2-(2-chlorophenyl)-2-(methylamino)cyclohexan-1-one, is an arylcyclohexylamine derivative and a racemic mixture containing equal amounts of *S*-ketamine and *R*-ketamine. The molecular weight (MW) of ketamine is 237.73 and that of ketamine hydrochloride (HCl) is 274.19. According to clinical studies in humans, the initial distribution phase of intravenous ketamine from the central compartment (plasma) to peripheral tissue compartments occurs with a half-life of 7 to 11 minutes, and the elimination phase occurs with a half-life of between 2 and 3 hours (Way. et al., 1982). Ketamine is distributed rapidly and has short duration of action. Therefore, ketamine is usually administered as an immediate-release dosage form.

As an antagonist of N-methyl D-aspartate (NMDA) receptors, ketamine is indicated for the use of anesthetic; for example, ketamine HCl has been marketed since 1970 as Ketalar injection (intravenous or intramuscular). In addition, *S*-ketamine HCl is used as an antidepressant, Spravato (nasal spray), which has been approved by FDA in 2019, for the treatment of treatment-resistant depression (TRD).

Marketed products of ketamine have two limitations. Firstly, ketamine formulation generally shows short half-life in clinical studies. Specifically, the ketamine elimination half-life of Ketalar is 2.5 hours. Further, the esketamine concentration of Spravato declines rapidly, and the mean terminal half-life ranges from 7 to 12 hours. Secondly, adverse events may occur after administration of ketamine formulation, such as dissociation, dizziness, nausea, sedation, vertigo, hypoesthesia, anxiety, lethargy, increased blood pressure, vomiting, and feeling drunk, such that the patient must be observed for a few hours during the recovery period. The long-acting ketamine formulation is thus developing.

PCT Patent Publication Nos. WO 2018/122626 A1 and WO 2019/186357 A1 issued to Cellix Bio Ltd. disclose ketamine salts (ketamine and -RH), wherein RH represents pamoic acid, and the ratio of ketamine to pamoic acid is 1: 1 in the formula.

PCT Patent Publication No. WO 2005/016261 A2 issued to Alkermes Controlled Therapeutics Inc. describes a pharmaceutical composition comprising a pamoate salt of an active agent selected from the group consisting of haloperidol and aripiprazole. The composition releases an effective amount of the active agent over a period of at least about 48 hours.

In previous studies, Han et al. (Int. J. Pharm. (2020), 581, 119291) discloses a sustained-release ketamine nanoparticle, which comprises poly(ethylene glycol) (PEG)-block-poly(lactic-co-glycolic acid) (PLGA). The formulation shows a sustained-release profile for more than 5 days after intravenous injection in mice, and the $C_{max}$ of ketamine is between 1000 and 10000 ng/mL.

PCT Patent Publication No. WO 2017/003935 Al issued to Shenox Pharmaceuticals LLC. describes transdermal delivery devices SHX-001 comprising ketamine for the treatment of major depressive disorder (MDD) and pain. The transdermal delivery device provides ketamine plasma concentrations below 100 ng/mL for 8 hours to 7 days, which reduces adverse side effects of ketamine. The pharmacokinetic profiles are predicted by known convolution methodology using *in vitro* transdermal permeation data and *in vivo* intravenous plasma concentration data.

PCT Patent Publication No. WO 2019/073408 Al issued to Douglas Pharmaceuticals Ltd. describes an oral extended release ketamine HCl tablet, which comprises polyethylene oxide (PEO), for the treatment of treatment-resistant depression (TRD), treatment-resistant anxiety, and phobia. The ketamine plasma concentrations show a sustained-release profile for 2 days after single doses of 60 mg, 120 mg and 240 mg ketamine tablets in clinical studies, and the $C_{max}$ of ketamine is about 12 to 42 ng/mL. The oral formulation has no dissociative side effects after 60 mg to120 mg doses, and minimal dissociative side effects at 240 mg.

SUMMARY

In view of the foregoing, the present disclosure relates a sustained-release pharmaceutical composition comprising a crystal or an amorphous form of a ketamine pamoate salt, and a pharmaceutically acceptable carrier thereof, wherein the ketamine pamoate salt is selected from the group consisting of R, S-ketamine pamoate salt, S-ketamine pamoate salt, R-ketamine pamoate salt, and any combination thereof. The ketamine pamoate salt is a crystal form or an amorphous form of a ketamine pamoate salt having a stoichiometry of 2:1 of ketamine to pamoate, a ketamine

pamoate salt having a stoichiometry of 1:1 of ketamine to pamoate, or a combination thereof. The pharmaceutically acceptable carrier is selected from the group consisting of N-methyl-2-pyrrolidone, ethyl acetate, ethanol, butanol, 2-butanol, isobutanol, isopropanol, glycerin, benzyl benzoate, dimethyl sulfoxide, N,N-dimethylacetamide, propylene glycol, dimethyl glycol, benzyl alcohol, polyethylene glycol 4000 (PEG4000), polysorbate 80 (Tween 80), sodium carboxymethyl cellulose, poly lactic acid, poly(lactic-co-glycolic) acid, and any combination thereof.

In accordance with embodiments of the present disclosure, the ketamine pamoate salt may be *R, S*-ketamine pamoate salt having a stoichiometry of 2:1 of ketamine to pamoate (Formula I), *S*-ketamine pamoate salt having a stoichiometry of 2:1 of ketamine to pamoate (Formula II), *R*-ketamine pamoate salt having a stoichiometry of 2:1 of ketamine to pamoate (Formula III), *R*- or *S*-ketamine pamoate salt having a stoichiometry of 1:1 of ketamine to pamoate (Formula IV), or any combination thereof:

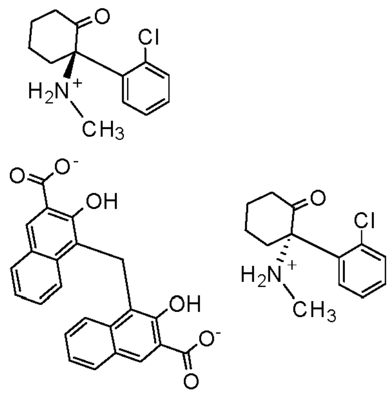

Formula I (*R*, *S*-ketamine pamoate salt, ratio 2:1),

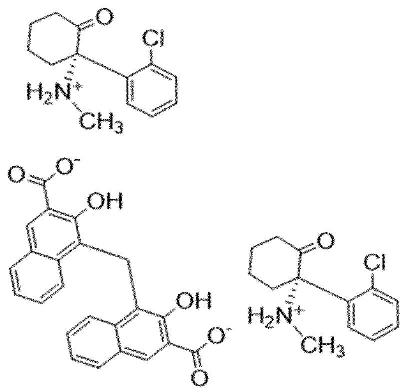

Formula II (*S*-ketamine pamoate salt, ratio 2:1),

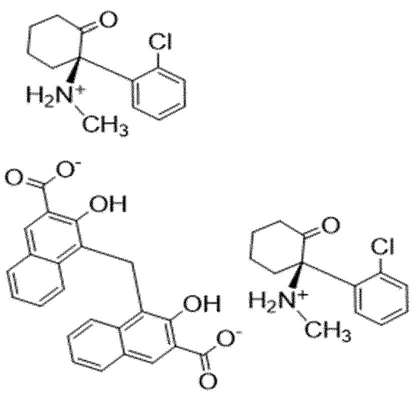

Formula III (*R*-ketamine pamoate salt, ratio 2:1),

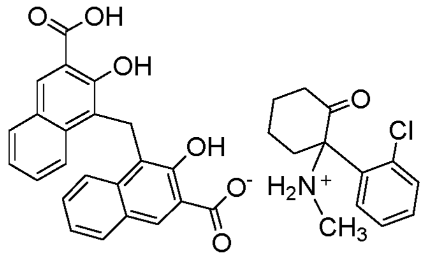

Formula IV (*R*- or *S*-ketamine pamoate salt, ratio 1:1).

In accordance with embodiments of the present disclosure, the crystal form of a ketamine pamoate salt is represented by an x-ray powder diffraction (XRPD) pattern comprising one or more 2θ values selected from 6.0, 10.7, 11.6, 12.0, 13.0, 14.7, 15.0, 22.2, 25.2 and 30.3 (± 0.2 2θ).

In some embodiments of the present disclosure, the ketamine pamoate salt is *R, S*-ketamine pamoate salt (ratio 2:1) in a crystal form represented by an XRPD pattern comprising one or more 2θ values selected from 6.0, 8.6, 10.7, 11.6, 12.0, 13.0, 14.7, 15.0, 15.3, 17.9, 18.6, 19.6, 20.0, 21.1, 21.6, 22.2, 23.3, 24.4, 25.2, 25.9, 26.9, 28.6, 29.7, 30.3, 32.4, 34.0 and 36.6 (± 0.2 2θ).

In some embodiments of the present disclosure, the ketamine pamoate salt is *S*-ketamine pamoate salt (ratio 2:1) in a crystal form represented by an XRPD pattern comprising one or more 2θ values selected from 6.0, 10.8, 11.7, 12.0, 12.6, 13.1, 14.6, 15.1, 18.2, 19.2, 19.7, 20.1, 22.0, 22.8, 23.3, 23.7, 24.1, 24.7, 25.2, 27.3, 30.1, 31.6, 45.4, 56.4 and 75.2 (± 0.2 2θ).

In some embodiments of the present disclosure, the ketamine pamoate salt is *R*-ketamine pamoate salt (ratio 2:1) in a crystal form represented by an XRPD pattern comprising one or more 2θ values selected from 6.0, 10.8, 11.7, 12.0, 12.6, 13.1, 14.6, 15.0, 18.2, 19.3, 19.7, 20.6, 22.0, 22.9, 23.6, 24.1, 24.7, 25.2, 25.9, 27.3, 30.1, 31.6, 45.4, 56.4 and 75.2 (± 0.2 2θ).

In some embodiments of the present disclosure, the ketamine pamoate salt is *R*- or *S*-ketamine pamoate salt (ratio 1:1) in a crystal form represented by an XRPD pattern comprising one or more 2θ values selected from 6.0, 7.5, 8.6, 9.4, 10.7, 11.1, 11.6, 12.1, 13.0, 14.7, 15.0, 15.5, 17.9, 18.6, 19.3, 20.0, 20.7, 21.1, 21.6, 22.3, 23.1, 23.4, 24.3, 25.0, 26.2, 26.9, 28.6, 29.8, 30.3, 31.1, 32.4, 33.3, 33.9, 36.6 and 37.4 (± 0.2 2θ). In some embodiments of the present disclosure, the pamoate salt of ketamine is in a crystal form represented by an XRPD pattern substantially in accordance with the pattern shown in FIG. 1, FIG. 2, FIG. 3 or FIG. 7.

In accordance with embodiments of the present disclosure, the sustained-release pharmaceutical composition comprises a ketamine pamoate salt, and a pharmaceutically acceptable carrier thereof. The pharmaceutically acceptable carrier may be selected from the group consisting of palmitic acid, oleic acid, stearic acid, decanoic acid, linoleic acid, *N*-methyl-2-pyrrolidone, ethyl acetate, ethanol, butanol, 2-butanol, isobutanol, isopropanol, glycerin, benzyl benzoate, dimethyl sulfoxide, *N,N*-dimethylacetamide, propylene glycol, dimethyl glycol, benzyl alcohol, polyethylene glycol 4000 (PEG4000), polysorbate 80 (Tween 80), sodium carboxymethyl cellulose, sodium chloride, poly lactic acid, poly(lactic-co-glycolic) acid, and any combination thereof.

In accordance with embodiments of the present disclosure, the sustained-release pharmaceutical composition may be an injectable aqueous suspension, an injectable solution, or an injectable matrix delivery system.

In some embodiments of the present disclosure, the sustained-release pharmaceutical composition is an injectable aqueous suspension, comprising the ketamine pamoate salt, and a pharmaceutically acceptable carrier thereof selected from the group consisting of polyethylene glycol 4000 (PEG4000), polysorbate 80 (Tween 80), sodium carboxymethyl cellulose, sodium chloride, or any combination thereof. In some embodiments, the injectable aqueous suspension of the present disclosure has an average particle size (d50) of less than 20 μm and a specific surface area of more than 300 $m^2/g$.

In some embodiments of the present disclosure, the sustained-release pharmaceutical composition is an injectable solution, comprising the ketamine pamoate salt, and a pharmaceutically acceptable carrier thereof selected from the group consisting of *N*-methyl-2-pyrrolidone, ethyl acetate, ethanol, butanol, 2-butanol, isobutanol, isopropanol, glycerin, benzyl benzoate, dimethyl sulfoxide, *N,N*-dimethylacetamide, propylene glycol, dimethyl glycol, benzyl alcohol, or any combination thereof. In some embodiments, the injectable solution of the present disclosure may further comprise at least one of palmitic acid, oleic acid, stearic acid, decanoic acid, and linoleic acid.

In some embodiments of the present disclosure, the sustained-release pharmaceutical composition is an injectable matrix delivery system comprising the ketamine pamoate salt, and a pharmaceutically acceptable carrier

thereof selected from the group consisting of poly lactic acid, poly(lactic-co-glycolic) acid, or a combination thereof. In some embodiments, the injectable matrix delivery system of the present disclosure may further comprise at least one of *N*-methyl-2-pyrrolidone, ethyl acetate, ethanol, butanol, 2-butanol, isobutanol, isopropanol, glycerin, benzyl benzoate, dimethyl sulfoxide, *N*,*N*-dimethylacetamide, propylene glycol, dimethyl glycol, benzyl alcohol, or any combination thereof.

In accordance with embodiments of the present disclosure, the ketamine pamoate salt is present at a concentration of 1% to 99%, 5% to 90%, 5% to 60%, 10% to 60%, or 15% to 40% (w/w) in the sustained-release pharmaceutical composition.

In accordance with embodiments of the present disclosure, the sustained-release pharmaceutical composition is an injectable formulation. In some embodiments, the sustained-release pharmaceutical composition is formulated for subcutaneous, intramuscular or intradermal injection.

In accordance with embodiments of the present disclosure, the sustained-release pharmaceutical composition has resistance to heat.

In accordance with embodiments of the present disclosure, the sustained-release pharmaceutical composition may further comprise one or more additional agents. In some embodiments, the additional agent is selected from the group consisting of a wetting agent, a suspending agent, a tonicity adjusting agent, a pH adjusting agent, a buffering agent, an antioxidant, a preservative, and any combination thereof.

The present disclosure also provides a method for treating a disease or a condition by using the above sustained-release pharmaceutical composition. In accordance with embodiments of the present disclosure, the method comprises administering to a subject in need thereof the sustained-release pharmaceutical composition.

In accordance with embodiments of the present disclosure, the disease or the condition is selected from the group consisting of a central nervous system disease, depression, anti-inflammatory, pain, and any combination thereof. In some embodiments, the disease or the condition is selected from the group consisting of major depressive disorder (MDD), treatment-resistant depression (TRD), suicidal ideation, bipolar disorder, obsessive-compulsive disorder, posttraumatic stress disorder (PTSD), autism spectrum disorder, tinnitus, refractory chronic migraine, asthma, anxiety, substance use disorder, alcohol use disorder, eating disorder, refractory status epilepticus, brain ischemia, Alzheimer’s disease, Parkinson’s disease, stroke, traumatic brain injury, multiple sclerosis, and any combination thereof.

The present disclosure also provides a method for anesthetizing a subject in need thereof by using the above sustained-release pharmaceutical composition. In accordance with embodiments of the present disclosure, the method comprises administering to the subject the sustained-release pharmaceutical composition.

In accordance with embodiments of the present disclosure, after the administration, the sustained-release pharmaceutical composition exhibits a steady release profile lasting 72 hours, such as one week, two weeks, three weeks, or one month.

BRIEF DESCRIPTION OF THE DRAWINGS

FIG. 1 illustrates the X-ray powder diffraction pattern of *R, S*-ketamine pamoate salt (crystal form, ratio 2:1).
FIG. 2 illustrates the X-ray powder diffraction pattern of *S*-ketamine pamoate salt (crystal form, ratio 2:1).
FIG. 3 illustrates the X-ray powder diffraction pattern of *R*-ketamine pamoate salt (crystal form, ratio 2:1).
FIG. 4 illustrates the X-ray powder diffraction pattern of *R, S*-ketamine pamoate salt (amorphous form, ratio 2:1).
FIG. 5 illustrates the X-ray powder diffraction pattern of *S*-ketamine pamoate salt (amorphous form, ratio 2:1).
FIG. 6 illustrates the X-ray powder diffraction pattern of *R*-etamine pamoate salt (amorphous form, ratio 2:1).
FIG. 7 illustrates the X-ray powder diffraction pattern of *R*- or *S*-ketamine pamoate salt (crystal form, ratio 1:1).
FIG. 8 illustrates the $^1$H nuclear magnetic resonance (NMR) spectrum of *R, S*-ketamine pamoate salt (crystal form, ratio 2:1).
FIG. 9 illustrates the $^1$H nuclear magnetic resonance (NMR) spectrum of *S*-ketamine pamoate salt (crystal form, ratio 2:1).
FIG. 10 illustrates the $^1$H nuclear magnetic resonance (NMR) spectrum of *R*-ketamine pamoate salt (crystal form, ratio 2:1).
FIG. 11 illustrates the $^1$H nuclear magnetic resonance (NMR) spectrum of *R*- or *S*-ketamine pamoate salt (crystal form, ratio 1:1).
FIG. 12 illustrates the $^{13}$C nuclear magnetic resonance (NMR) spectrum of *S*-ketamine pamoate salt (crystal form, ratio 2:1).
FIG. 13 illustrates the $^{13}$C nuclear magnetic resonance (NMR) spectrum of *R*-ketamine pamoate salt (crystal form, ratio 2:1).
FIG. 14 illustrates the Fourier-transform infrared (FTIR) spectrum of *S*-ketamine pamoate salt (crystal form, ratio 2:1).
FIG. 15 illustrates the FTIR spectrum of *R*-ketamine pamoate salt (crystal form, ratio 2:1).

FIG. 16 illustrates the FTIR spectrum of *S*-ketamine pamoate salt (amorphous form, ratio 2:1).
FIG. 17 illustrates the FTIR spectrum of *R*-ketamine pamoate salt (amorphous form, ratio 2:1).
FIG. 18 illustrates the FTIR spectrum of *R*, *S*-ketamine pamoate salt (amorphous form, ratio 2:1).
FIG. 19 illustrates the FTIR spectrum of *R*- or *S*-ketamine pamoate salt (amorphous form, ratio 1:1).
FIG. 20 illustrates the differential scanning calorimetry (DSC) pattern of *R, S*-ketamine pamoate salt (crystal form, ratio 2:1).
FIG. 21 illustrates the DSC pattern of *S*-ketamine pamoate salt (crystal form, ratio 2:1).
FIG. 22 illustrates the DSC pattern of *R*-ketamine pamoate salt (crystal form, ratio 2:1).
FIG. 23 illustrates the DSC pattern of *R*- or *S*-ketamine pamoate salt (crystal form, ratio 1:1).
FIG. 24 illustrates the DSC pattern of *S*-ketamine pamoate salt (amorphous form, ratio 2:1).
FIG. 25 illustrates the DSC pattern of *R*-ketamine pamoate salt (amorphous form, ratio 2:1).
FIG. 26 illustrates the DSC pattern of *R, S*-ketamine pamoate salt (amorphous form, ratio 2:1).
FIG. 27 illustrates the intrinsic dissolution rate of *R*, *S*-ketamine pamoate salt (crystal form) in medium with different pH values.
FIG. 28 illustrates the intrinsic dissolution rate of *R, S*-ketamine hydrochloride in medium with different pH values.
FIG. 29 illustrates the intrinsic dissolution rate of crystal and amorphous forms of *S*-ketamine pamoate salt, *R*-ketamine pamoate salt and *R, S*-ketamine pamoate salt in pH 7.4 medium.
FIG. 30 illustrates the X-ray powder diffraction pattern of ketamine pamoate salt after heating (121°C) for 4 hours.
FIG. 31 illustrates the DSC pattern of ketamine pamoate salt before and after heating (121°C) for 4 hours.
FIG. 32 illustrates the mean plasma levels of ketamine after subcutaneous injection of formulation SL01 at the dose of 60 mg ketamine/kg in rats.
FIG. 33 illustrates the mean plasma levels of ketamine after subcutaneous injection of formulation SL02 at the dose of 60 mg ketamine/kg in rats.
FIG. 34 illustrates the mean plasma levels of ketamine after subcutaneous injection of formulation SL03 at the dose of 60 mg ketamine/kg in rats.
FIG. 35 illustrates the mean plasma levels of ketamine after subcutaneous injection of formulation AS01 at the dose of 60 mg ketamine/kg in rats.
FIG. 36 illustrates the mean plasma levels of ketamine after subcutaneous injection of formulation SL02 at the dose of 3 mg ketamine/kg in minipigs.
FIG. 37 illustrates the mean plasma levels of ketamine after subcutaneous injection of formulation SL04 at the dose of 6 mg ketamine/kg in minipigs.
FIG. 38 illustrates the mean plasma levels of ketamine after subcutaneous injection of formulation AS02 at the dose of 6 mg ketamine/kg in minipigs.
FIG. 39 illustrates the mean the plasma levels of ketamine after intramuscular injection of formulation AS03 at the dose of 10.3 mg ketamine/kg in minipigs.
FIG. 40 illustrates the mean plasma levels of ketamine after subcutaneous injection of formulation AS05 at the dose of 3 mg ketamine/kg in minipigs.
FIG. 41 illustrates the mean ketamine releasing profiles after subcutaneous injection of formulations SL01, SL02, SL03 and AS01 at the dose of 60 mg ketamine/kg in rats.
FIG. 42 illustrates the mean ketamine releasing profiles after subcutaneous injection of formulations SL02 and AS05 at the dose of 3 mg ketamine/kg, subcutaneous injection of formulations AS02 and SL04 at the dose of 6 mg ketamine/kg, and intramuscular injection of formulation AS03 at the dose of 10.3 mg ketamine/kg in minipigs.
FIG. 43 is a diagram illustrating the protocol of dexamethasone (DEX)-induced depression-like animal model to evaluate the antidepressant effects of ketamine HCl (KET), *R, S*-ketamine pamoate salt (KEP), S-ketamine pamoate salt (S-KEP), and *R*-ketamine pamoate salt (R-KEP). ICR mice were intraperitoneally injected with saline or DEX at postnatal day 1 to 3 (P1 to P3) with dose decrement of 0.5 mg/kg, 0.3 mg/kg, and 0.1 mg/kg, respectively. The drugs for each group or saline group were administered subcutaneously at day 35 (P35, i.e., dosing day 0, D0), and the forced swimming test (FST) were conducted every 10 days from dosing day 1 (D1, P36) to P98. Evaluation of sedation behavior was also conducted by sedation rating scale from the time immediately post injection to dosing day 28 after drug administration.
FIG. 44 is a graph showing antidepressant effects of ketamine HCl (KET), *R, S*-ketamine pamoate salt (KEP), *S*-ketamine pamoate salt (S-KEP), and *R*-ketamine pamoate salt (R-KEP) by forced swimming test (FST) on day 1 to day 63 post drug administration. Results are represented by mean $\pm$ SEM. Student's *t*-test is analyzed with the Saline group versus the other groups at each time point. * $p < 0.05$, ** $p < 0.01$, *** $p < 0.001$, and **** $p < 0.0001$ indicate significant differences.
FIG. 45 is a graph showing sedation rating scores of mice treated with saline (Saline group), ketamine HCl (KET), *R, S*-ketamine pamoate salt (KEP), *S*-ketamine pamoate salt (S-KEP), and *R*-ketamine pamoate salt (R-KEP) from the time immediately post injection to dosing day 28.

DETAILED DESCRIPTION

The following examples are used for illustrating the present disclosure. A person skilled in the art can easily conceive the other advantages and effects of the present disclosure, based on the disclosure of the specification. The present disclosure can also be implemented or applied as described in different examples. It is possible to modify or alter the above examples for carrying out this disclosure without contravening its scope, for different aspects and applications.

It is further noted that, as used in this disclosure, the singular forms "a," "an," and "the" include plural referents, unless expressly and unequivocally limited to one referent. The term "or" is used interchangeably with the term "and/or" unless the context clearly indicates otherwise.

The present disclosure is directed to formulations of ketamine pamoate salts having long-lasting release profiles after single dose administration and displaying minimal initial bursts. In accordance with embodiments of the present disclosure, the formulation of a ketamine pamoate salt is useful for the treatment of central nervous system diseases, depression, pain or anti-inflammatory. The ketamine pamoate salt is a crystal form or an amorphous form of *R, S*-ketamine pamoate salt, *S*-ketamine pamoate salt, or *R*-ketamine pamoate salt. A ketamine pamoate salt having a stoichiometry of 2:1 of ketamine to pamoate, a ketamine pamoate salt having a stoichiometry of 1:1 of ketamine to pamoate, or a combination thereof.

The formulation of a ketamine pamoate salt is a sustained-release pharmaceutical composition comprising the crystal form or the amorphous form of the ketamine pamoate salt and a pharmaceutically acceptable carrier thereof.

In accordance with embodiments of the present disclosure, the sustained-release pharmaceutical composition may contain the ketamine pamoate salt in any suitable concentration, such as 1% to 99%, 1% to 90%, 5% to 90%, 5% to 80%, 5% to 70%, 5% to 60%, 10% to 70%, 10% to 60%, 15% to 50%and 15% to 40% (w/w). It is noted that when a numerical range is disclosed in this disclosure, it is intended to include all numbers within the ranges, as if each of these numbers have been individually disclosed.

In accordance with embodiments of the present disclosure, the sustained-release pharmaceutical composition may be formulated as an aqueous solution, which comprises a biocompatible solvent as the pharmaceutically acceptable carrier. The biocompatible solvent may be an organic solvent including, but not limited to, *N*-methyl-2-pyrrolidone, ethyl acetate, ethanol, butanol, 2-butanol, isobutanol, isopropanol, glycerin, benzyl benzoate, dimethyl sulfoxide, *N,N*-dimethylacetamide, propylene glycol, dimethyl glycol, benzyl alcohol, or any combination thereof.

In accordance with embodiments of the present disclosure, the sustained-release pharmaceutical composition may be formulated as an aqueous suspension, which comprises at least one of PEG4000, Tween 80, sodium carboxymethyl cellulose, sodium chloride, or any combination thereof.

In accordance with embodiments of the present disclosure, the sustained-release pharmaceutical composition may be formulated as a matrix delivery system, which comprises a controlled release matrix as the pharmaceutically acceptable carrier. The controlled release matrix may contain poly lactic acid, poly(lactic-co-glycolic) acid, or a combination thereof.

The sustained-release pharmaceutical composition of the present disclosure may further comprise a wetting agent, a suspending agent, a tonicity adjusting agent, a pH adjusting agent, a buffering agent, an antioxidant, a preservative, and any combination thereof.

The various formulations of the present disclosure do not have undesirable initial bursts and may display a sustained-releasing profile over 1 week, 2 weeks, 3 weeks, 1 month, 2 months, 3 months, 4 months, 5 months, 6 months or longer. The formulations without the significant burst release of ketamine pamoate salts may not only reduce the risks of several systemic adverse effects, e.g., pinpoint pupils, sedation, hypotension, and respiratory depression, but also lessen the burden of physicians to monitor patients frequently. Furthermore, the formulations of ketamine pamoate salts exhibit high bioavailability, pharmaceutically effective plasma concentration for at least one week, and minimal risk of local site reactions.

Different examples have been used to illustrate the present disclosure. The examples below should not be taken as a limit to the scope of the present disclosure.

EXAMPLES

The preparation flow of *S*-ketamine pamoate salt and *R*-ketamine pamoate salt was shown as Scheme 1 below.

Scheme 1

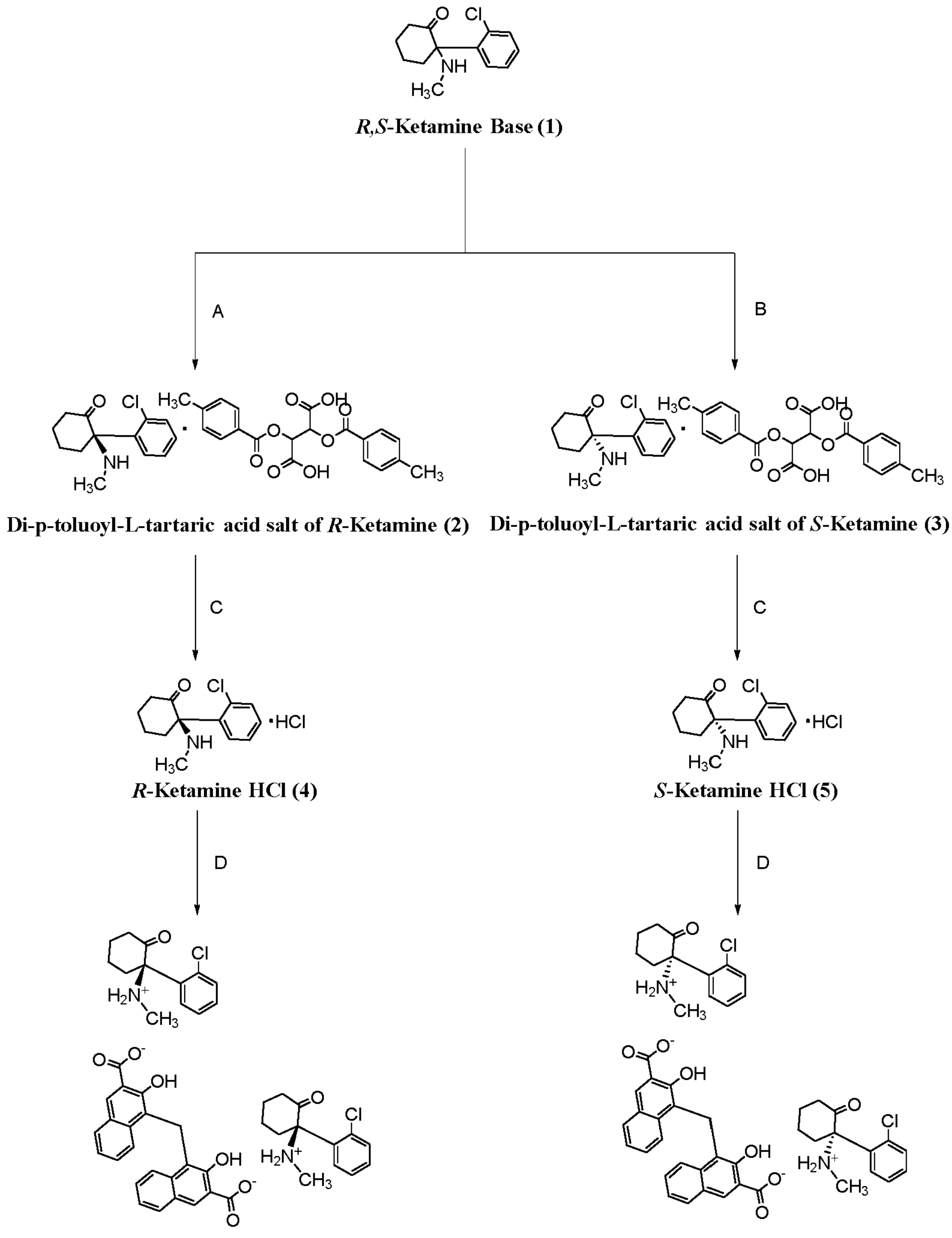


*R,S*-Ketamine Base (1)

Di-p-toluoyl-L-tartaric acid salt of *R*-Ketamine (2)

Di-p-toluoyl-L-tartaric acid salt of *S*-Ketamine (3)

*R*-Ketamine HCl (4)

*S*-Ketamine HCl (5)

*R*-Ketamine pamoate salt (6)

*S*-Ketamine pamoate salt (7)

A: Di-p-toluoyl-L-tartaric acid/EtOH, EtOH/$H_2O$(3:2)
B: Di-p-toluoyl-L-tartaric acid/EtOH, EtOH/$H_2O$(2:3)
C: HCl/THF
D: Disodium pamoate/$H_2O$

Example 1: Preparation of *R, S*-ketamine free base (1)

10 g of *R, S*-ketamine hydrochloride was dissolved in 100 mL of water, and then 150 mL saturated sodium bicarbonate aqueous solution was added with stirring for 10 min. The reaction mixture was extracted with dichloromethane (100 mL x 2). The separated organic layers were combined and distilled under reduced pressure to obtain *R, S*-ketamine free base (1).

Example 2: Preparation of (-)-O, O'-di-p-toluoyl-L-tartaric acid salt of *R*-ketamine (2)

Di-p-toluoyl-L-tartaric acid (13 g, 33.6 mmol) and *R*, *S*-ketamine free base (8 g, 33.6 mmol) were dissolved in ethanol (160 mL) with stirring for 5 min. 10 mL water was added dropwise to the solution at room temperature, followed by stirring for 1 hr to obtain the precipitate. The filtrate solution was collected after suction filtration and dried under vacuum. The residue was dissolved in 100 mL of 60% ethanol solution (i.e., $EtOH:H_2O$ = 3:2) at 60°C, and cooled to room temperature for 1 hr to obtain the solid, followed by drying under vacuum. The resulting powder was analyzed by high-performance liquid chromatography (HPLC), differential scanning calorimetry (DSC), optical rotation, nuclear magnetic resonance (NMR) spectrum, and literature information. The characterization of (-)-O, O'-di-p-toluoyl-L-tartaric acid salt of *R*-ketamine (2) was confirmed by specific rotation, melting point (m.p.), and HPLC chiral purity as shown below:

m.p. = 133.5-141.3°C, $[\alpha]_D^{25^\circ} = -75^\circ$, *c* = 1.0, dimethyl formamide, chiral purity = 98.4 %. $^1$H-NMR (DMSO-$d_6$): 7.87 (d, 4H, *J* = 8.0 Hz), 7.68 (d, 1H, *J* = 6.8 Hz), 7.44 (m, 3H), 7.36 (d, 4H, *J* = 8.0 Hz), 5.74 (s, 2H), 2.66-2.32 (m, 2H), 2.39 (s, 6H), 2.04 (s, 3H), 1.90-1.58 (m, 6H).

Example 3: Preparation of (-)-O, O'-di-p-toluoyl-L-tartaric acid salt of *S*-ketamine (3)

The precipitate from Example 2 was dried under reduced pressure. The solid was dissolved in 100 mL of 40% ethanol solution (i.e., $EtOH:H_2O$ = 2:3) at 60°C, and cooled to room temperature for 1 hr to obtain the solid, followed by drying under vacuum. The resulting powder was analyzed by HPLC, DSC, optical rotation, NMR spectrum, and literature information. The characterization of (-)-O, O'-di-p-toluoyl-L-tartaric acid salt of S-ketamine (3) was confirmed by specific rotation, melting point, and HPLC chiral purity as shown below:

m.p. = 157.1-163.3°C, $[\alpha]_D^{25^\circ} = -108^\circ$, *c* = 1.0, dimethyl formamide, chiral purity = 100%. $^1$H-NMR (DMSO-$d_6$): 7.87 (d, 4H, *J* = 7.6 Hz), 7.67 (d, 1H, *J* = 7.6 Hz), 7.44 (m, 3H), 7.36 (d, 4H, *J* = 8.0 Hz), 5.74 (s, 2H), 2.64-2.31 (m, 2H), 2.39 (s, 6H), 2.03 (s, 3H), 1.91-1.59 (m, 6H).

Example 4: Preparation of *R*-ketamine pamoate salt (6) (Crystal, ratio 2:1)

Di-p-toluoyl-L-tartaric acid salt of *R*-ketamine (2) was dissolved in ten-fold tetrahydrofuran (THF) by stirring at 2-10°C. Hydrochloride (37%) was added to the solution to obtain the precipitate, and then the precipitate was collected by suction filtration to obtain *R*-ketamine hydrochloride (4). *R*-ketamine hydrochloride (4) and disodium pamoate were dissolved separately in ten-fold water. Afterwards, water was distilled from the reaction mixture by decompression. The residue was dissolved in ethanol stirring at 60°C and recrystallized by decreasing temperature. The resulting powder was analyzed by HPLC, DSC, infrared (IR), X-ray diffraction pattern (XRD), and NMR spectrums. The characterization of the crystal form of *R*-ketamine pamoate salt (6) was confirmed by analysis results and specific rotation of *R*-ketamine pamoate salt (6) of $[\alpha]_D^{25^\circ} = +67^\circ$.

Example 5: Preparation of *S*-ketamine pamoate salt (7) (Crystal, ratio 2:1)

Di-p-toluoyl-L-tartaric acid salt of *S*-ketamine (3) was dissolved in ten-fold tetrahydrofuran (THF) by stirring at 2°C to 10°C. Hydrochloride (37%) was added to the solution to obtain the precipitate, and then the precipitate was collected by suction filtration to obtain *S*-ketamine hydrochloride (5). *S*-ketamine hydrochloride (5) and disodium pamoate were dissolved separately in ten-fold water. Afterwards, water was distilled from the reaction mixture by decompression. The residue was recrystallized with ethanol stirring at 60°C and isolated by vacuum filtration. The resulting powder was analyzed by HPLC, DSC, optical rotation, IR, XRD, and NMR spectrums. The characterization of the crystal form of *S*-ketamine pamoate salt (7) was confirmed by analysis results and specific rotation of *S*-ketamine pamoate salt (7) of $[\alpha]_D^{25^\circ} = -67^\circ$.

Example 6: Preparation of *R*-ketamine pamoate salt (Amorphous, ratio 2:1)

*R*-ketamine pamoate salt (6) was dissolved in methanol, and the solvent was removed under reduced pressure to obtain the amorphous form of *R*-ketamine pamoate salt. The resulting powder was analyzed by HPLC, DSC, optical rotation, IR, XRD, and NMR spectrums. The characterization of the amorphous form of *R*-ketamine pamoate salt was confirmed by analysis results and specific rotation of *R*-ketamine pamoate salt of $[\alpha]_D^{25^\circ} = +67^\circ$.

Example 7: Preparation of *S*-ketamine pamoate salt (Amorphous, ratio 2:1)

*S*-ketamine pamoate salt (7) was dissolved in methanol, and the solvent was removed under reduced pressure to obtain the amorphous form of *S*-ketamine pamoate salt (amorphous). The resulting powder was analyzed by HPLC, DSC, optical rotation, IR, XRD, and NMR spectrums. The characterization of the amorphous form of *S*-ketamine pamoate salt was confirmed by analysis results and specific rotation of *S*-ketamine pamoate salt of $[\alpha]_D^{25^\circ} = -67^\circ$.

Example 8: Preparation of *R, S*-ketamine pamoate salt (Crystal, ratio 2:1)

Ketamine HCl (20 g, 72.9 mmol) and disodium pamoate monohydrate (15 g, 33.3 mmol) were dissolved in 65% ethanol aqueous solution (350 mL) in a round-bottom flask. The mixture was stirred constantly at 70°C for 30 minutes. Then, the mixture were gradually cooled to ambient temperature with ice bath. After that, the mixture was stirred constantly at ambient temperature overnight. This reaction mixture was filtered, and the powder was collected and dried under reduced pressure. The resulting powder was analyzed by DSC, IR, XRD, and NMR spectrums.

Example 9: Preparation of *R, S*-ketamine pamoate salt (Amorphous, ratio 2:1)

The solid collected from Example 8 was dissolved in methanol. The solvent was removed under reduced pressure and dried to obtain the amorphous form of *R, S*-ketamine pamoate salt. The resulting powder was analyzed by DSC, IR, XRD, and NMR spectrums.

Example 10: Preparation of *R*- or *S*-ketamine pamoate salt (Crystal, ratio 1:1)

Ketamine free base (10 g, 42.1 mmol) and pamoic acid (16 g, 41.2 mmol) were dissolved in acetonitrile (2300 mL) and dimethyl sulfoxide (100 mL) in a round-bottom flask. The mixture was stirred constantly at ambient temperature overnight. This reaction mixture was filtered, and the powder was collected and dried under reduced pressure. The resulting powder was analyzed by DSC, IR, XRD, and NMR spectrums.

Example 11: XRPD analysis

The X-ray powder diffraction (XRPD) pattern was obtained on a Bruker D8 Discover X-ray powder diffractometer, equipped with a CuK$\alpha$ radiation source of wavelength ($\lambda$ = 1.54056 Å), operating at 40 kV and 40 mA.

Each sample was scanned between 2° and 80° in 2$\theta$, with a step size of 0.02° in 2$\theta$ and a scan rate of 0.6 second/step. The angular peak positions in 2$\theta$ and corresponding $I/I_o$ data for all crystal forms of ketamine pamoate salt peaks with intensities equal to or greater than 10% of the largest peak were tabulated in Table 1.

The crystal forms of *R*- or *S*-ketamine pamoate salt (ratio 1:1), *R, S*-ketamine pamoate salt (ratio 2:1), *S*-ketamine pamoate salt (ratio 2:1), and *R*-ketamine pamoate salt (ratio 2:1) were characterized by the X-ray diffraction pattern (XRD), and the results were provided in FIGs. 1 to 7.

Table 1. X-ray diffraction peaks of crystal forms of ketamine pamoate salts

| *R- or S*-ketamine pamoate salt (Crystal, ratio 1:1) | | | *R, S*-ketamine pamoate salt (Crystal, ratio 2:1) | | |
|---|---|---|---|---|---|
| 2$\theta$ [deg.] | d-spacing | I/I*o* | 20 [deg.] | d-spacing | I/I*o* |
| 6.0 | 14.66 | 11.0 | 6.0 | 14.70 | 10.1 |
| 7.5 | 11.84 | 10.7 | 7.5 | 11.85 | 11.1 |
| 8.6 | 10.31 | 17.3 | 8.6 | 10.32 | 14.7 |
| 9.4 | 9.41 | 44.3 | - | - | - |
| 10.7 | 8.25 | 25.1 | 10.7 | 8.26 | 22.7 |
| 11.1 | 7.99 | 35.5 | - | - | - |
| 11.6 | 7.64 | 25.3 | 11.6 | 7.64 | 32.1 |
| 12.1 | 7.33 | 100.0 | 12.0 | 7.34 | 100.0 |
| 13.0 | 6.80 | 58.6 | 13.0 | 6.79 | 80.1 |
| 14.7 | 6.01 | 37.3 | 14.7 | 6.01 | 50.0 |
| 15.0 | 5.91 | 48.2 | 15.0 | 5.91 | 59.0 |
| 15.5 | 5.69 | 56.5 | 15.4 | 5.75 | 11.5 |
| 17.9 | 4.96 | 44.1 | 17.9 | 4.96 | 63.0 |

(continued)

| *R- or S*-ketamine pamoate salt (Crystal, ratio 1:1) | | | *R, S*-ketamine pamoate salt (Crystal, ratio 2:1) | | |
|---|---|---|---|---|---|
| 2θ [deg.] | d-spacing | I/I*o* | 20 [deg.] | d-spacing | I/I*o* |
| 18.6 | 4.76 | 42.1 | 18.6 | 4.77 | 40.0 |
| 19.3 | 4.58 | 46.7 | - | - | - |
| - | - | - | 19.6 | 4.53 | 40.4 |
| 20.0 | 4.43 | 22.6 | 20.0 | 4.43 | 28.0 |
| 20.7 | 4.29 | 12.4 | - | - | - |
| 21.1 | 4.21 | 12.7 | 21.1 | 4.22 | 14.1 |
| 21.6 | 4.11 | 23.1 | 21.6 | 4.11 | 28.6 |
| 22.3 | 3.99 | 20.4 | 22.3 | 3.99 | 15.1 |
| 23.1 | 3.84 | 35.2 | - | - | - |
| 23.4 | 3.80 | 39.6 | 23.3 | 3.81 | 46.5 |
| 24.3 | 3.65 | 31.9 | 24.4 | 3.65 | 37.3 |
| 25.0 | 3.56 | 29.5 | 25.1 | 3.54 | 26.8 |
| - | - | - | 25.5 | 3.49 | 12.3 |
| - | - | - | 26.0 | 3.42 | 14.7 |
| 26.2 | 3.40 | 62.4 | 26.3 | 3.39 | 13.2 |
| 26.9 | 3.31 | 18.3 | 26.9 | 3.31 | 20.0 |
| 28.6 | 3.12 | 14.3 | 28.7 | 3.11 | 16.2 |
| 29.8 | 2.99 | 46.3 | 29.7 | 3.00 | 16.1 |
| 30.3 | 2.95 | 17.2 | 30.3 | 2.95 | 20.7 |
| 31.1 | 2.87 | 10.9 | 31.1 | 2.87 | 10.1 |
| 32.4 | 2.76 | 12.8 | 32.4 | 2.76 | 11.7 |
| 33.3 | 2.69 | 10.6 | - | - | - |
| 33.9 | 2.64 | 20.2 | 33.9 | 2.64 | 18.6 |
| 36.6 | 2.45 | 11.4 | 36.7 | 2.45 | 10.8 |
| 37.4 | 2.40 | 10.9 | - | - | - |

| 2θ [deg.] | *S*-ketamine pamoate salt (Crystal, ratio 2:1) d-spacing | I/I*o* | 20 [deg.] | *R*-ketamine pamoate salt (Crystal, ratio 2:1) d-spacing | I/I*o* |
|---|---|---|---|---|---|
| 6.0 | 14.72 | 13.7 | 6.0 | 14.75 | 14.3 |
| 10.8 | 8.19 | 19.3 | 10.8 | 8.19 | 25.0 |
| 11.7 | 7.56 | 36.1 | 11.7 | 7.56 | 46.4 |
| 12.0 | 7.35 | 34.0 | 12.0 | 7.36 | 39.8 |
| 12.6 | 7.01 | 12.1 | 12.6 | 7.01 | 15.9 |
| 13.1 | 6.76 | 38.8 | 13.1 | 6.77 | 46.9 |
| 14.6 | 6.08 | 16.5 | 14.6 | 6.07 | 19.3 |
| 15.1 | 5.87 | 26.7 | 15.0 | 5.89 | 33.4 |
| 18.2 | 4.87 | 48.9 | 18.2 | 4.86 | 57.0 |
| 19.2 | 4.61 | 32.7 | 19.3 | 4.60 | 38.4 |
| 19.7 | 4.50 | 22.4 | 19.7 | 4.50 | 28.2 |
| 20.1 | 4.41 | 12.4 | 20.6 | 4.31 | 10.8 |
| 22.0 | 4.04 | 15.6 | 22.0 | 4.04 | 19.7 |
| 22.8 | 3.89 | 21.0 | 22.9 | 3.89 | 25.4 |
| 23.3 | 3.81 | 16.4 | 23.6 | 3.76 | 22.0 |
| 23.7 | 3.76 | 18.1 | 24.1 | 3.69 | 20.4 |
| 24.1 | 3.69 | 15.8 | - | - | - |
| 24.7 | 3.60 | 12.3 | 24.7 | 3.60 | 17.1 |
| 25.2 | 3.53 | 13.6 | 25.2 | 3.53 | 16.9 |
| - | - | - | 25.9 | 3.43 | 13.6 |
| 27.3 | 3.27 | 13.2 | 27.3 | 3.26 | 19.3 |

(continued)

| 2θ [deg.] | *S*-ketamine pamoate salt (Crystal, ratio 2:1) d-spacing | I/I*o* | 20 [deg.] | *R*-ketamine pamoate salt (Crystal, ratio 2:1) d-spacing | I/I*o* |
|---|---|---|---|---|---|
| 30.1 | 2.97 | 11.9 | 30.1 | 2.97 | 14.9 |
| 31.6 | 2.83 | 100.0 | 31.6 | 2.83 | 100.0 |
| 45.4 | 2.00 | 49.4 | 45.4 | 2.00 | 54.2 |
| 56.4 | 1.63 | 10.6 | 56.4 | 1.63 | 16.6 |
| 75.2 | 1.26 | 12.3 | 75.2 | 1.26 | 12.2 |

Example 12: NMR analysis

Crystal forms of ketamine pamoate salts were dissolved in deuterium solvent (DMSO) and the nuclear magnetic resonance (NMR) spectra were obtained using a Bruker Ascend TM 400 MHz NMR spectrometer.

The characterizations of crystal forms of *R*- or *S*-ketamine pamoate salt (ratio 1:1), *R, S*-ketamine pamoate salt (ratio 2:1), *S*-ketamine pamoate salt (ratio 2:1), and *R*-ketamine pamoate salt (ratio 2:1) were confirmed by $^{1}$H-NMR spectroscopy (as shown in Table 2 and FIGs. 8 to 11). Also, crystal forms of *S*-ketamine pamoate salt (ratio 2:1) and *R*-ketamine pamoate salt (ratio 2:1) were subjected to $^{13}$C-NMR spectroscopy, and the chemical shifts were reported in ppm (as shown in Table 3 and FIGs. 12 and 13).

Table 2. $^{1}$H-NMR (400 MHz, DMSO) data of ketamine pamoate salts

| Compound | *R*- or *S*-ketamine pamoate salt (Crystal, ratio 1:1) | *R, S*-ketamine pamoate salt (Crystal, ratio 2:1) | *S*-ketamine pamoate salt (Crystal, ratio 2:1) | *R*-ketamine pamoate salt (Crystal, ratio 2:1) |
|---|---|---|---|---|
| Chemical shift (δ ppm) | 2.02-1.46 (m, 5H) | 1.96-1.57 (m, 12H) | 1.95-1.51 (m, 12H) | 1.97-1.53 (m, 12H) |
| | 2.20 (s, 3H) | 2.12 (s, 6H) | 2.19 (s, 6H) | 2.15 (s, 6H) |
| | 2.50-2.38 (m, 2H), 3.17 (br. d, 1H,*J* = 13.6 Hz) | 2.51-2.34 (m, 4H) | 2.51-2.34 (m, 4H) | 2.50-2.37 (m, 4H) |
| | 4.76 (s, 2H) | 4.72 (s, 2H) | 4.74 (s, 2H) | 4.72 (s, 2H) |
| | 7.14 (dd, 2H,*J* = 14.8 Hz, 7.6Hz) | 7.09 (dd, 2H,*J* = 14.8 Hz, 7.2 Hz) | 7.08 (dd, 2H,*J* = 14.8 Hz, 7.2 Hz) | 7.08 (dd, 2H,*J* = 14.8 Hz, 7.2 Hz) |
| | 7.29 (dd, 2H,*J* = 15.6 Hz, 8.4 Hz) | 7.21 (dd, 2H, *J*= 8.4 Hz, 1.2 Hz) | 7.21 (dd, 2H, *J* = 15.2 Hz, 7.2 Hz) | 7.21 (dd, 2H, *J* = 15.2 Hz, 7.2 Hz) |
| | 7.57 (m, 3H) | 7.51 (m, 6H) | 7.52 (m, 6H) | 7.52 (m, 6H) |
| | 7.86 (d, 1H, *J* = 8.0 Hz), 7.80 (d, 2H,*J* = 8.0 Hz) | 7.75 (dd, 4H,*J* = 16.4 Hz, 8 Hz) | 7.82 (d, 2H, *J* = 7.6 Hz), 7.73 (d, 2H, *J* = 8 Hz) | 7.79 (d, 2H, *J* = 7.2 Hz), 7.73 (d, 2H, *J* = 8.0 Hz) |
| | 8.16 (d, 2H,*J* = 8.4Hz) | 8.17 (d, 2H, *J* = 8.8 Hz) | 8.19 (d, 2H, *J* = 8.8 Hz) | 8.17 (d, 2H, *J* = 8.4 Hz) |
| | 8.38 (s, 2H) | 8.29 (s, 2H) | 8.30 (s, 2H) | 8.28 (s, 2H) |

Table 3. $^{13}C$-NMR (100 MHz, DMSO) data of ketamine pamoate salts

| Compound | *S*-ketamine pamoate salt (Crystal, ratio 2:1) | *R*-ketamine pamoate salt (Crystal, ratio 2:1) | Number of Carbon | Assignment |
|---|---|---|---|---|
| Chemical shift (δ ppm) | 20.3 | 20.3 | 1C | Ph-$CH_2$-Ph |
| | 21.4 | 21.3 | 2C | -$CH_2$- |
| | 28.6 | 28.7 | 6C | -$CH_2$- |
| | 37.2 | 37.3 | 2C | N-$CH_3$ |
| | 71.1 | 70.9 | 2C | >C-N |
| | 119.8 | 119.6 | 2C | Aromatic, =C< |
| | 120.3 | 120.3 | 2C | Aromatic, =CH- |
| | 122.4 | 122.4 | 2C | Aromatic, =CH- |
| | 124.0 | 124.0 | 2C | Aromatic, =CH- |
| | 126.9 | 126.8 | 2C | Aromatic, =C< |
| | 127.4 | 127.4 | 2C | Aromatic, =CH- |
| | 128.3 | 128.2 | 2C | Aromatic, =C< |
| | 129.9 | 129.9 | 2C | Aromatic, =C< |
| | 130.3 | 130.3 | 2C | Aromatic, =CH- |
| | 131.7 | 131.4 | 4C | Aromatic, =CH- |
| | 131.9 | 131.8 | 4C | Aromatic, =CH- |
| | 134.0 | 133.8 | 2C | Aromatic, =C< |
| | 136.0 | 136.1 | 2C | Aromatic, =C-OH |
| | 155.9 | 155.9 | 2C | Aromatic, =C-Cl |
| | 172.4 | 172.2 | 2C | O-C=O |
| | 206.9 | 206.7 | 2C | >C=O |

Example 13: Fourier-transform infrared (FT-IR) spectroscopy analysis

The polymorphs of ketamine pamoate salt were further characterized by infrared (IR) spectroscopy obtained in a disk using a Bruker FPA-FTIR Vertex 70V, Hyperion 3000 system, and the results were shown in FIGs. 14 to 19. The IR absorbances (in wavenumbers, $cm^{-1}$) sufficient to identify crystal and amorphous forms of *S*-ketamine pamoate salt, *R*-ketamine pamoate salt, and *R, S*-ketamine pamoate salt were summarized in Table 4 below.

Table 4. FT-IR peaks of ketamine pamoate salts (in wavenumbers, $cm^{-1}$)

| *S*-ketamine pamoate salt (Crystal, ratio 2:1) | *R*-ketamine pamoate salt (Crystal, ratio 2:1) | *S*-ketamine pamoate salt (Amorphous, ratio 2:1) | *R*-ketamine pamoate salt (Amorphous, ratio 2:1) | *R, S*-ketamine pamoate salt (Amorphous, ratio 2:1) | *R*- or *S*-ketamine pamoate salt (Crystal, ratio 1:1) |
|---|---|---|---|---|---|
| 716 | 716 | 715 | 715 | 715 | 739 |
| 731 | 730 | - | - | - | 759 |
| 760 | 759 | 755 | 756 | 755 | 787 |
| 812 | 811 | 811 | 812 | 812 | 797 |
| 832 | 832 | 831 | 831 | 831 | 812 |
| 853 | 854 | - | - | - | 832 |
| 895 | 901 | - | - | - | 896 |
| - | - | - | - | - | 941 |
| - | - | - | - | - | 1013 |

(continued)

| *S*-ketamine pamoate salt (Crystal, ratio 2:1) | *R*-ketamine pamoate salt (Crystal, ratio 2:1) | *S*-ketamine pamoate salt (Amorphous, ratio 2:1) | *R*-ketamine pamoate salt (Amorphous, ratio 2:1) | *R, S*-ketamine pamoate salt (Amorphous, ratio 2:1) | *R*- or *S*-ketamine pamoate salt (Crystal, ratio 1:1) |
|---|---|---|---|---|---|
| 1049 | 1048 | 1048 | 1048 | 1048 | 1050 |
| 1087 | 1087 | 1084 | 1083 | 1083 | 1094 |
| - | - | - | - | - | 1142 |
| 1143 | 1143 | 1149 | 1148 | 1148 | 1155 |
| - | - | - | - | - | 1172 |
| - | - | - | - | - | 1208 |
| - | - | - | - | - | 1231 |
| 1233 | 1233 | 1232 | 1232 | 1232 | 1294 |
| - | - | - | - | - | 1308 |
| - | - | - | - | - | 1330 |
| 1333 | 1332 | 1334 | 1335 | 1334 | 1350 |
| 1352 | 1351 | 1351 | 1351 | 1351 | 1388 |
| 1393 | 1392 | 1392 | 1392 | 1392 | 1433 |
| 1448 | 1448 | 1449 | 1450 | 1449 | 1453 |
| 1509 | 1510 | 1509 | 1509 | 1509 | 1507 |
| 1556 | 1554 | 1561 | 1561 | 1561 | 1602 |
| 1643 | 1643 | 1641 | 1641 | 1641 | 1651 |
| 1728 | 1727 | 1724 | 1725 | 1725 | 1726 |

Example 14: DSC analysis

Differential scanning calorimetry (DSC) analysis of the samples of *R, S*-ketamine pamoate salt (ratio 2:1), *S*-ketamine pamoate salt (ratio 2:1), *R*-ketamine pamoate salt (ratio 2:1) and *R*- or *S*-ketamine pamoate salt (ratio 1:1) exhibited a glass transition at approximately 234°C, 212°C, 214°C and 230°C, respectively, indicating that these samples were crystal form (FIGs. 20 to 23). DSC analysis of the samples of *S*-ketamine pamoate salt (ratio 2:1) and *R*-ketamine pamoate salt (ratio 2:1) exhibited a temperature at approximately 210°C and 193°C, indicating that these samples were amorphous (FIGs. 24 and 25). DSC analysis of the sample of *R, S*-ketamine pamoate salt (ratio 2:1) exhibited a temperature at approximately 213°C, indicating that the sample was amorphous (FIG. 26).

The DSC analysis was performed by using a Mettler Toledo DSC3 under standard conditions. The DSC analysis for crystal forms of ketamine pamoate salts were summarized in Table 5 below.

Table 5. DSC analysis peaks of ketamine pamoate salts

| No. | Compound | Onset Temperature (°C) |
|---|---|---|
| 1 | *R, S*-ketamine pamoate salt (Crystal, ratio 2:1) | 233.5 |
| 2 | *S*-ketamine pamoate salt (Crystal, ratio 2:1) | 212.1 |
| 3 | *R*-ketamine pamoate salt (Crystal, ratio 2:1) | 214.0 |
| **4** | *R*- or *S*-ketamine pamoate salt (Crystal, ratio 1:1) | 229.5 |

Example 15: Solubility test

The solubility of ketamine pamoate salts was measured based on United States Pharmacopeia (USP) guidance and summarized in Table 6 below. The results showed that ketamine pamoate salts exhibited poor solubility in different pH media. The highest solubility was observed in *R, S*-ketamine pamoate salt in pH 1.2 (1.68 mg/mL), and the lowest solubility was observed in *R, S*-ketamine pamoate salt in pH 6.8 (0.16 mg/mL). *R*- or *S*-ketamine pamoate salt (ratio 1:1) had the similar properties to *R, S*-ketamine pamoate salt (ratio 2:1) in solubility, wherein the highest solubility was observed in *R*- or *S*-ketamine pamoate salt (ratio 1:1) in pH 1.2 (0.72 mg/mL), and the lowest solubility was observed in *R*- or *S*-ketamine pamoate salt (ratio 1:1) in pH 6.8 (0.07 mg/mL).

In addition, it was found that ketamine pamoate salts were poorly soluble in aqueous solution in comparison with

ketamine hydrochloride (solubility: >200 mg/mL in water).

Table 6. Solubilities of ketamine pamoate salts

| Medium | Solubility (mg/mL) | | | | | | |
|---|---|---|---|---|---|---|---|
| | *R, S*-ketamine pamoate salt (Ratio 2:1) | | *S*-ketamine pamoate salt (Ratio 2:1) | | *R*-ketamine pamoate salt (Ratio 2:1) | | *R*- or *S*-ketamine pamoate salt (Ratio 1:1) |
| | Crystal | Amorphous | Crystal | Amorphous | Crystal | Amorphous | Crystal |
| pH 1.2 | 1.68 | 1.31 | 1.24 | 1.00 | 1.09 | 1.15 | 0.72 |
| pH 4.5 | 1.08 | 0.87 | 1.61 | 0.84 | 1.18 | 0.99 | 0.45 |
| pH 6.8 | 0.16 | 0.16 | 0.39 | 0.48 | 0.40 | 0.64 | 0.07 |
| pH 7.4 | 0.22 | 0.18 | 0.50 | 0.58 | 0.51 | 0.95 | 0.08 |

Example 16: Dissolution rate study

The intrinsic dissolution rate of the crystal and amorphous forms of ketamine pamoate salts was determined by using the intrinsic dissolution rotating disk method described in Chapter 1087 of the U.S. Pharmacopeia under the conditions of medium pH as 1.2, 4.5, 6.8 or 7.4, medium volume as 900 mL, rotating speed as 50 rpm, medium temperature as 37°C, and detection wavelength as 210 nm. The results were shown in Tables 7 and 8 and FIGs. 27 to 29.

As shown in Table 7 and FIGs. 29 and 30, it was observed that intrinsic dissolution rate of the crystal form of *R, S*-ketamine pamoate salt (ratio 2:1) decreased as the pH increased, and in comparison with *R, S*-ketamine hydrochloride, *R, S*-ketamine pamoate salt (ratio 2:1) exhibited substantially low dissolution rate in different pH media.

In addition, the dissolution rate of the crystal and amorphous forms of *S*-ketamine pamoate salt (ratio 2:1), *R*-ketamine pamoate salt (ratio 2:1) and *R, S*-ketamine pamoate salt (ratio 2:1) in pH 7.4 medium was shown in Table 8 and FIG. 29, and the solubility classification of such ketamine pamoate salts based on biopharmaceutics classification system (BCS) was also shown in Table 8. It was observed that the intrinsic dissolution rate of ketamine pamoate salts was over 80 times lower than that of ketamine hydrochloride.

Table 7. Dissolution rates of *R, S*-ketamine pamoate salt (crystal, ratio 2:1) and *R, S*-ketamine HCl in different media

| | Intrinsic dissolution rate* ($mg/min/cm^2$) | |
|---|---|---|
| Medium | *R, S*-ketamine pamoate salt (Crystal, ratio 2:1) | *R, S*-ketamine HCl |
| pH 1.2 | 0.095 | 5.329 |
| pH 4.5** | 0.005 | 3.729 |
| pH 6.8** | 0.011 | 2.918 |
| pH 7.4** | 0.012 | 1.740 |
| * Average of three samples.<br>** Containing 1% polysorbate 20 in medium. | | |

Table 8. Dissolution rates and BCS solubility classifications of ketamine pamoate salts in pH 7.4 medium**

| No. | Compound | Intrinsic dissolution rate* ($mg/min/cm^2$) in pH 7.4** | BCS solubility classification |
|---|---|---|---|
| 1 | *S*-ketamine pamoate salt (Crystal, ratio 2:1) | 0.027 | Low |
| 2 | *R*-ketamine pamoate salt (Crystal, ratio 2:1) | 0.025 | Low |
| 3 | *R, S*-ketamine pamoate salt (Amorphous, ratio 2:1) | 0.024 | Low |
| 4 | *S*-ketamine pamoate salt (Amorphous, ratio 2:1) | 0.026 | Low |
| 5 | *R*-ketamine pamoate salt (Amorphous, ratio 2:1) | 0.021 | Low |
| 6 | *S*-ketamine HCl | 2.248 | High |

(continued)

| No. | Compound | Intrinsic dissolution rate* ($mg/min/cm^2$) in pH 7.4** | BCS solubility classification |
|---|---|---|---|
| 7 | *R*-ketamine HCl | 2.356 | High |
| *Average of three samples.<br>** Containing 1% polysorbate 20 in medium. | | | |

Example 17: Preparation of ketamine pamoate salt solution

*R, S*-ketamine pamoate salt (Crystal, ratio 2:1) and an excipient were added into a glass vial and dissolved in a biocompatible organic solvent, such as *N*-methyl-2-pyrrolidone (NMP) and *N,N*-dimethylacetamide (DMAc). The mixture was stirred constantly at ambient temperature or heated slightly until all of the ingredients were dissolved. The compositions of the obtained pharmaceutical solutions were listed in Table 9 below.

Table 9. Compositions of ketamine pamoate salt solutions

| Formulation | Active Pharmaceutical Ingredient (API), wt% | Solvent, wt% | Excipient, wt% |
|---|---|---|---|
| SL01 | *R, S*-ketamine pamoate salt, 20% | DMAc, 80% | - |
| SL02 | *R, S*-ketamine pamoate salt, 25% | NMP, 75% | - |
| SL03 | *R, S*-ketamine pamoate salt, 40% | NMP, 55% | Palmitic acid, 5% |
| SL04 | *R, S*-ketamine pamoate salt, 40% | NMP, 50% | Palmitic acid, 10% |
| SL05 | *R, S*-ketamine pamoate salt, 40% | NMP, 50% | Oleic acid, 10% |
| SL06 | *R, S*-ketamine pamoate salt, 40% | NMP, 55% | Stearic acid, 5% |
| SL07 | *R, S*-ketamine pamoate salt, 40% | NMP, 50% | Decanoic acid, 10% |
| SL08 | *R, S*-ketamine pamoate salt, 35% | NMP, 55% | Linoleic acid, 10% |
| SL09 | *R, S*-ketamine pamoate salt, 35% | NMP, 55% | Tetradecanoic acid, 10% |

Example 18: Preparation of aqueous suspension of ketamine pamoate salts

*R, S*-ketamine pamoate salt (Crystal, ratio 2:1), *S*-ketamine pamoate salt (Crystal, ratio 2:1) and *R*-ketamine pamoate salt (Crystal, ratio 2:1) were individually added into a flask and suspended in an excipient, which was composed of polyethylene glycol 4000 (PEG4000), polysorbate 80 (Tween80), sodium carboxymethyl cellulose (NaCMC), and/or sodium chloride (NaCl) dissolved in dd-$H_2O$.

The aqueous suspension was mixed uniformly by sonicating and further subjected to milling. The compositions of the aqueous suspensions and the used milling process were listed in Table 10. The aqueous suspensions were added into glass vials for the analysis of particle size distributions, which was performed by Bettersizer S2-E laser particle size analyzer. The particle size distribution results were shown in Table 11.

Table 10. Compositions and milling processes of aqueous suspensions of ketamine pamoate salts

| Formulation | API, wt% | Excipient | Milling process |
|---|---|---|---|
| AS01 | *R, S*-ketamine pamoate salt, 33% | PEG4000 (30 mg/mL), Tween 80 (3 mg/mL) | Bead milling for 15 minutes, 0.50 mm Zirconia Grinding Media |
| AS02 | *R, S*-ketamine pamoate salt, 33% | PEG4000 (30 mg/mL), Tween 80 (3 mg/mL) | High pressure homogenizers |
| AS03 | *R, S*-ketamine pamoate salt, 33% | PEG4000 (30 mg/mL), Tween 80 (3 mg/mL) | Bead milling for 30 minutes, 0.50 mm Zirconia Grinding Media |
| AS04 | *R, S*-ketamine pamoate salt, 33% | PEG4000 (30 mg/mL), Tween 80 (3 mg/mL) | Bead milling for 15 minutes, 0.50 mm Zirconia Grinding Media |
| AS05 | *S*-ketamine pamoate salt, 33% | PEG4000 (30 mg/mL), Tween 80 (3 mg/mL) | Bead milling for 15 minutes, 0.50 mm Zirconia Grinding Media |

(continued)

| Formulation | API, wt% | Excipient | Milling process |
|---|---|---|---|
| AS06 | *S*-ketamine pamoate salt, 25% | PEG4000 (30 mg/mL), Tween 80 (3 mg/mL) | Bead milling for 15 minutes, 0.50 mm Zirconia Grinding Media |
| AS07 | *R*-ketamine pamoate salt, 15% | PEG4000 (30 mg/mL), Tween 80 (3 mg/mL) | Bead milling for 15 minutes, 0.50 mm Zirconia Grinding Media |
| AS08 | *R, S*-ketamine pamoate salt, 33% | NaCMC (5 mg/mL), NaCl (8 mg/mL), Tween 80 (1 mg/mL) | High pressure homogenizer |
| AS09 | *R, S*-ketamine pamoate salt, 15% | NaCMC (5 mg/mL), NaCl (8 mg/mL), Tween 80 (1 mg/mL) | High pressure homogenizer |
| AS10 | *R, S*-ketamine pamoate salt, 15% | NaCMC (5 mg/mL), NaCl (8 mg/mL) | High pressure homogenizer |
| AS11 | *R, S*-ketamine pamoate salt, 15% | NaCMC (5 mg/mL), NaCl (10 mg/mL) | High pressure homogenizer |

Table 11. Particle size distributions of aqueous suspensions of ketamine pamoate salts

| Formulation | Volume Statistics | | | |
|---|---|---|---|---|
| | d10 (μm) | d50 (μm) | d90 (μm) | Specific Surface Area ($m^2/g$) |
| AS01 | 1.3 | 7.8 | 20.4 | 641 |
| AS02 | 0.7 | 2.2 | 6.3 | 1459 |
| AS03 | 0.9 | 2.4 | 4.8 | 1285 |
| AS04 | 0.9 | 2.6 | 6.1 | 1170 |
| AS05 | 0.8 | 3.2 | 11.1 | 1093 |
| AS06 | 1.7 | 11.4 | 42.3 | 554 |
| AS07 | 2.7 | 13.7 | 31.4 | 432 |
| AS08 | 3.3 | 16.4 | 41.4 | 379 |
| AS09 | 0.6 | 1.8 | 4.4 | 1668 |
| AS10 | 0.6 | 2.1 | 5.5 | 1551 |
| AS11 | 0.6 | 2.1 | 5.9 | 1552 |

Example 19: Preparation of matrix delivery system of ketamine pamoate salts

*R, S*-ketamine pamoate salt (Crystal, ratio 2:1), poly lactic acid (PLA) or poly(lactic-co-glycolic) acid (PLGA), and a biocompatible solvent were added into a glass vial, and then was placed into a 50°C water bath with constant stirring until all the ingredients were dissolved. The mixture was removed from water bath and stirred at room temperature to form a solution (i.e., matrix delivery system). The compositions of the matrix delivery systems were listed in Table 12 below.

Table 12. Compositions of matrix delivery systems of ketamine pamoate salts

| Formulation | API, wt% | PLA or PLGA type*, wt% | Solvent, wt% |
|---|---|---|---|
| PS01 | *R, S*-ketamine pamoate salt, 20% | PLGA 50/50 (44 kD, ester capped), 5% | DMA, 75% |
| PS02 | *R, S*-ketamine pamoate salt, 20% | PLGA 75/25 (17 kD, ester capped), 5% | DMA, 75% |
| PS03 | *R, S*-ketamine pamoate salt, 25% | PLGA 50/50 (44 kD, ester capped), 5% | DMSO, 70% |
| PS04 | *R, S*-ketamine pamoate salt, 25% | PLGA 75/25 (17 kD, ester capped), 5% | DMSO, 70% |
| PS05 | *R, S*-ketamine pamoate salt, 25% | PLA (17 kD, ester capped), 5% | DMSO, 70% |

(continued)

| Formulation | API, wt% | PLA or PLGA type*, wt% | Solvent, wt% |
|---|---|---|---|
| PS06 | *R, S*-ketamine pamoate salt, 30% | PLGA 75/25 (17 kD, ester capped), 5% | NMP, 65% |
| * PLGA type: lactidyl/glycolidyl (LA/GA) ratio | | | |

Example 20: Stability study of pharmaceutical formulation of ketamine pamoate salts

To assess the stability of the pharmaceutical formulation of a ketamine pamoate salt at high temperature (121°C), the pharmaceutical type I glass vials (3 mL), the rubber closures, and the flip-off seals made of aluminum and polypropylene were used. Further, the formulation AS11 prepared in Example 18 were heated to 121°C in an oven for 1 to 4 hours.

Subsequently, HPLC was used to analyze the assay of ketamine and the total related substances, and the results were shown in Table 13. In addition, the particle size distributions of the formulation were further measured, and the results were shown in Table 14.

The formulation after heating for 4 hours was dried by vacuum pump, and the powder was characterized by XRD and DSC. The XRD of the heated ketamine pamoate salt was shown in Table 15 and FIG. 30, and the DSC of the heated ketamine pamoate salt was shown in FIG. 31. By comparing with the results obtained in Example 14, it was found that R, S-ketamine pamoate salt before and after heating for 4 hours exhibited onset temperature at 231.3°C and 231.6°C, respectively.

The HPLC result showed that assay of ketamine was between 99.7% to 100.8%, and there was no related substances generated. Further, the results determined by the particle size distributions, XRD and DSC showed that there was no significant difference between the formulation before and after heating. These results suggested that the suspension of ketamine pamoate salt had superior heat resistance.

Table 13. Assay and total related substances of ketamine pamoate salt formulation in heating condition (121°C)

| Formulation | Time (hours) | Assay of ketamine (%) | Total related substances (%) |
|---|---|---|---|
| AS11 | 0 | 100.8 | ND |
| | 1 | 100.4 | ND |
| | 2 | 99.7 | ND |
| | 3 | 100.1 | ND |
| | 4 | 100.3 | ND |
| * ND: Not Detected | | | |

Table 14. Particle size distributions of ketamine pamoate salt formulation in heating condition (121°C)

| Formulation | Time (hours) | d10 (μm) | d50 (μm) | d90 (μm) | Specific Surface Area ($m^2/g$) |
|---|---|---|---|---|---|
| AS11 | 0 | 0.6 | 2.1 | 5.9 | 1552 |
| | 1 | 0.7 | 2.6 | 6.5 | 1411 |
| | 2 | 0.6 | 2.3 | 6.1 | 1569 |
| | 3 | 0.6 | 2.5 | 6.5 | 1530 |
| | 4 | 0.6 | 2.5 | 6.4 | 1544 |

Table 15. X-ray diffraction peaks of ketamine pamoate salt before and after heating for 4 hours

| 2θ [deg.] | Before heating d-spacing | I/Io | 2θ [deg.] | After heating d-spacing | I/Io |
|---|---|---|---|---|---|
| 6.0 | 14.70 | 10.1 | 6.0 | 14.72 | 9.3 |
| 7.5 | 11.85 | 11.1 | 7.5 | 11.83 | 12.1 |
| 8.6 | 10.32 | 14.7 | 8.6 | 10.31 | 18.4 |
| 10.7 | 8.26 | 22.7 | 10.7 | 8.26 | 30.5 |
| 11.6 | 7.64 | 32.1 | 11.5 | 7.66 | 32.2 |

(continued)

| 2θ [deg.] | Before heating d-spacing | I/Io | 2θ [deg.] | After heating d-spacing | I/Io |
|---|---|---|---|---|---|
| 12.0 | 7.34 | 100.0 | 12.1 | 7.34 | 100 |
| - | - | - | 12.8 | 6.91 | 56 |
| 13.0 | 6.79 | 80.1 | 13.0 | 6.79 | 89.8 |
| 14.7 | 6.01 | 50.0 | 14.7 | 6.01 | 58.6 |
| 15.0 | 5.91 | 59.0 | 15.0 | 5.91 | 62.1 |
| 15.4 | 5.75 | 11.5 | 15.4 | 5.74 | 14.6 |
| 17.9 | 4.96 | 63.0 | 17.8 | 4.97 | 78.6 |
| 18.6 | 4.77 | 40.0 | 18.6 | 4.76 | 53.1 |
| - | - | - | 19.3 | 4.6 | 26.7 |
| 19.6 | 4.53 | 40.4 | 19.6 | 4.53 | 51.4 |
| 20.0 | 4.43 | 28.0 | 20.1 | 4.42 | 29.9 |
| 21.1 | 4.22 | 14.1 | 21.1 | 4.21 | 17.3 |
| 21.6 | 4.11 | 28.6 | 21.6 | 4.11 | 31.5 |
| 22.3 | 3.99 | 15.1 | 22.3 | 3.99 | 15.2 |
| 23.3 | 3.81 | 46.5 | 23.4 | 3.8 | 58.2 |
| 24.4 | 3.65 | 37.3 | 24.3 | 3.66 | 47 |
| 25.1 | 3.54 | 26.8 | 25.1 | 3.54 | 33.2 |
| 25.5 | 3.49 | 12.3 | 25.5 | 3.49 | 15.7 |
| 26.0 | 3.42 | 14.7 | 26.0 | 3.42 | 16 |
| 26.3 | 3.39 | 13.2 | 26.3 | 3.39 | 14.4 |
| 26.9 | 3.31 | 20.0 | 26.9 | 3.31 | 24.6 |
| 28.7 | 3.11 | 16.2 | 28.6 | 3.12 | 21.3 |
| 29.7 | 3.00 | 16.1 | 29.7 | 3.00 | 17 |
| 30.3 | 2.95 | 20.7 | 30.3 | 2.95 | 22.3 |
| 31.1 | 2.87 | 10.1 | 31.2 | 2.87 | 13.2 |
| 32.4 | 2.76 | 11.7 | 32.2 | 2.78 | 14.1 |
| - | - | - | 33.3 | 2.69 | 11 |
| 33.9 | 2.64 | 18.6 | 33.9 | 2.64 | 20 |
| - | - | - | 35.0 | 2.56 | 10.9 |
| - | - | - | 35.6 | 2.52 | 11.9 |
| - | - | - | 36.1 | 2.49 | 10.1 |
| 36.7 | 2.45 | 10.8 | 36.7 | 2.45 | 13.7 |

Example 21: Pharmacokinetic profiles of pharmaceutical formulations of ketamine pamoate salts in rats

The formulations SL01, SL02, SL03 and AS01 prepared in Examples 17 and 18 were injected subcutaneously into male CD (SD) IGS rats at a dose of 60 mg ketamine/kg. Blood samples were collected from tail veins at indicated time points. Plasma samples were separated by centrifuge and then stored in frozen condition for later analysis. LC-MS/MS was used to analyze the concentrations of ketamine in each plasma samples.

The pharmacokinetic profiles of the formulations SL01, SL02, SL03 and AS01 were shown in Tables 16 to 19 and FIGs. 32 to 35. It was found that the formulation SL01 maintained a release profile of ketamine over 10 days; the formulation SL02 maintained a release profile of ketamine over 14 days; the formulation SL03 maintained a release profile of ketamine over 10 days; and the formulation AS01 maintained a release profile of ketamine over 14 days.

Table 16. Pharmacokinetic profile of the formulation SL01 in rats

| Time (days) | SL01 (60 mg/kg) | |
|---|---|---|
| | Mean (ng/mL) | S.D. (n = 3) |
| 0 | 0 | 0 |
| 0.083 | 474.89 | 62.88 |
| 0.167 | 264.33 | 36.72 |

(continued)

| Time (days) | SL01 (60 mg/kg) | |
|---|---|---|
| | Mean (ng/mL) | S.D. (n = 3) |
| 0.25 | 227.31 | 46.09 |
| 1 | 100.52 | 14.55 |
| 2 | 17.20 | 11.03 |
| 3 | 1.82 | 2.01 |
| 7 | 0.68 | 0.40 |
| 10 | 0.42 | 0.28 |
| S.D.: standard deviation | | |

Table 17. Pharmacokinetic profile of the formulation SL02 in rats

| Time (days) | SL02 (60 mg/kg) | |
|---|---|---|
| | Mean (ng/mL) | S.D. (n = 3) |
| 0 | 0 | 0 |
| 0.083 | 327.66 | 67.57 |
| 0.167 | 166.47 | 32.49 |
| 0.25 | 119.27 | 21.93 |
| 1 | 77.83 | 32.23 |
| 2 | 35.29 | 9.83 |
| 3 | 14.32 | 16.65 |
| 7 | 1.70 | 0.46 |
| 10 | 0.40 | 0.70 |
| 14 | 0.05 | 0.08 |
| 21 | 0 | 0 |
| S.D.: standard deviation | | |

Table 18. Pharmacokinetic profile of the formulation SL03 in rats

| Time (days) | SL03 (60 mg/kg) | |
|---|---|---|
| | Mean (ng/mL) | S.D. (n = 3) |
| 0 | 0 | 0 |
| 0.083 | 190.47 | 66.38 |
| 0.167 | 108.36 | 29.34 |
| 0.25 | 98.13 | 32.19 |
| 1 | 76.07 | 30.35 |
| 2 | 49.81 | 11.48 |
| 3 | 20.28 | 17.47 |
| 7 | 1.78 | 3.05 |
| 10 | 0.39 | 0.65 |

Table 19. Pharmacokinetic profile of the formulation AS01 in rats

| Time (days) | AS01 (60 mg/kg) | |
|---|---|---|
| | Mean (ng/mL) | S.D.(n = 3) |
| 0 | 0 | 0 |
| 0.083 | 43.75 | 8.16 |
| 0.167 | 56.70 | 14.39 |
| 1 | 52.77 | 6.30 |
| 2 | 39.28 | 1.02 |

(continued)

| Time (days) | AS01 (60 mg/kg) | |
|---|---|---|
| | Mean (ng/mL) | S.D.(n = 3) |
| 3 | 23.07 | 2.03 |
| 7 | 6.31 | 3.12 |
| 10 | 2.63 | 1.04 |
| 14 | 0.07 | 0.13 |

S.D.: standard deviation

Example 22: Pharmacokinetic profiles of pharmaceutical formulations of ketamine pamoate salts in minipigs

The formulations SL02, SL04, AS02, AS03 and AS05 prepared in Examples 17 and 18 were injected subcutaneously (SC) or intramuscularly (IM) into Lanyu minipigs (provided by PigModel Animal Technology Co., Ltd.). The detailed dosing record of the minipigs animal study was listed in Table 20 below.

Blood samples were collected from external jugular veins at indicated time points. Plasma samples were separated by centrifuge and then stored in frozen conditions for later analysis. LC-MS/MS was used to analyze the concentrations of ketamine in the plasma samples. The pharmacokinetic profiles of the formulations SL02, SL04, AS02, AS03 and AS05 were shown in Tables 21 to 25 and FIGs. 36 to 40. It was found that the formulation AS03 maintained a release profile of ketamine over 21 days. The ketamine plasma concentration after injection of formulation AS02 was lower than 10 ng/mL for 2 hours to 28 days.

Table 20. Dosing record of the minipigs animal study

| Formulation | Route of administration | Dose (mg/kg) | Gender of minipigs |
|---|---|---|---|
| SL02 | SC | 3 | female |
| SL04 | SC | 6 | male |
| AS02 | SC | 6 | male |
| AS03 | IM | 10.3 | male |
| AS05 | SC | 3 | male |

Table 21. Pharmacokinetic profile of the formulation SL02 in minipigs

| Time (days) | SL02 (3 mg/kg) | |
|---|---|---|
| | Mean (ng/mL) | S.D. (n = 3) |
| 0 | 0 | 0 |
| 0.083 | 17.55 | 4.34 |
| 0.167 | 12.77 | 3.28 |
| 0.25 | 9.79 | 2.70 |
| 1 | 3.80 | 0.34 |
| 2 | 3.52 | 0.75 |
| 3 | 3.00 | 0.49 |
| 4 | 2.95 | 1.00 |
| 5 | 3.47 | 0.49 |
| 6 | 1.62 | 0.57 |
| 7 | 1.98 | 1.64 |
| 8 | 0.77 | 0.69 |
| 9 | 0.33 | 0.38 |
| 10 | 0.02 | 0.04 |
| 12 | 0 | 0 |

S.D.: standard deviation

Table 22. Pharmacokinetic profile of the formulation SL04 in minipigs

| Time (days) | SL04 (6 mg/kg) | |
|---|---|---|
| | Mean (ng/mL) | S.D. (n = 3) |
| 0 | 0 | 0 |
| 0.083 | 32.61 | 7.46 |
| 0.167 | 27.09 | 2.32 |
| 0.25 | 15.75 | 1.99 |
| 1 | 7.05 | 1.42 |
| 2 | 6.15 | 1.33 |
| 3 | 6.35 | 2.32 |
| 4 | 3.72 | 1.33 |
| 5 | 5.15 | 1.33 |
| 6 | 3.80 | 0.66 |
| 7 | 4.73 | 3.02 |
| 8 | 3.33 | 1.40 |
| 9 | 2.86 | 0.85 |
| 10 | 2.90 | 1.01 |
| 12 | 2.35 | 0.56 |
| 14 | 3.04 | 1.57 |
| 21 | 1.68 | 0.18 |
| 28 | 1.10 | 0.40 |

S.D.: standard deviation

Table 23. Pharmacokinetic profile of the formulation AS02 in minipigs

| Time (days) | AS02 (6 mg/kg) | |
|---|---|---|
| | Mean (ng/mL) | S.D. (n = 3) |
| 0 | 0.11 | 0.18 |
| 0.083 | 7.27 | 6.36 |
| 0.25 | 4.75 | 2.10 |
| 1 | 4.58 | 1.58 |
| 3 | 7.92 | 2.66 |
| 5 | 9.17 | 2.96 |
| 7 | 7.11 | 0.99 |
| 10 | 4.84 | 0.87 |
| 14 | 3.44 | 1.14 |
| 21 | 0.81 | 0.42 |
| 28 | 0.27 | 0.34 |

S.D.: standard deviation

Table 24. Pharmacokinetic profile of the formulation AS03 in minipigs

| Time (days) | AS03 (10.3 mg/kg) | |
|---|---|---|
| | Mean (ng/mL) | S.D. (n = 3) |
| 0 | 0 | 0 |
| 0.083 | 34.74 | 2.15 |
| 0.167 | 27.01 | 4.07 |
| 0.25 | 20.71 | 3.54 |
| 1 | 16.09 | 2.52 |
| 3 | 13.07 | 2.41 |
| 5 | 16.50 | 3.96 |

(continued)

| Time (days) | AS03 (10.3 mg/kg) | |
|---|---|---|
| | Mean (ng/mL) | S.D. (n = 3) |
| 7 | 11.74 | 0.52 |
| 10 | 12.26 | 3.26 |
| 14 | 6.15 | 2.90 |
| 21 | 0.05 | 0.04 |
| 28 | 0 | 0 |
| S.D.: standard deviation | | |

Table 25. Pharmacokinetic profile of the formulation AS05 in minipigs

| Time (days) | AS05 (3 mg/kg) | |
|---|---|---|
| | Mean (ng/mL) | S.D. (n = 3) |
| 0 | 0 | 0 |
| 0.083 | 5.25 | 2.74 |
| 0.25 | 5.05 | 2.23 |
| 1 | 7.73 | 1.19 |
| 3 | 5.04 | 1.34 |
| 7 | 0.51 | 0.48 |
| 10 | 0.01 | 0.01 |
| 14 | 0 | 0 |
| S.D.: standard deviation | | |

Example 23: *In vivo* releasing profiles of the injectable pharmaceutical formulations in rats and minipigs

According to the pharmacokinetic profile of the ketamine pamoate salt formulations in rats and minipigs, the area under the curve during specific time point ($AUC_{0-t}$) and the area under the plasma concentration-time curve from time 0 extrapolated to infinity ($AUC_{0-\infty}$) were measured. The releasing percentage of ketamine was further estimated through the following formula:

$$\%\ \text{Release} = \frac{AUC_{0-t}}{AUC_{0-\infty}} \times 100\%.$$

The *in vivo* releasing profiles of the formulation SL01, SL02, SL03 and AS01 in rats were shown in Table 26 and FIG. 41. The *in vivo* releasing profiles of the formulation SL02, SL04, AS02, AS03 and AS05 in minipigs were shown in Table 27 and FIG. 42. The releasing profiles in two species showed that the formulations of ketamine pamoate salts could constantly release ketamine for various periods from 10 to 28 days.

Table 26. Mean ketamine releasing percentages after subcutaneous injection of SL01, SL02, SL03 or AS01 at the dose of 60 mg ketamine/kg in rats

| Time (days) | SL01 (60 mg/kg) | | SL02 (60 mg/kg) | | AS01 (60 mg/kg) | | SL03 (60 mg/kg) | |
|---|---|---|---|---|---|---|---|---|
| | Mean ketamine release (%) | S.D. (n = 3) | Mean ketamine release (%) | S.D. (n = 3) | Mean ketamine release (%) | S.D. (n = 3) | Mean ketamine release (%) | S.D. (n = 3) |
| 0 | 0 | 0 | 0 | 0 | 0 | 0 | 0 | 0 |
| 0.083 | 7.37 | 1.00 | 5.73 | 0.71 | 0.87 | 0.12 | 3.26 | 0.94 |
| 0.167 | 18.85 | 2.69 | 14.49 | 1.87 | 2.90 | 0.46 | 8.38 | 2.26 |
| 0.25 | 26.46 | 3.67 | 19.50 | 2.43 | - | - | 11.96 | 3.06 |
| 1 | 71.86 | 6.40 | 50.62 | 7.60 | 24.91 | 4.39 | 39.14 | 12.34 |
| 2 | 93.57 | 4.05 | 74.35 | 12.96 | 47.02 | 5.84 | 65.20 | 20.29 |

(continued)

| Time (days) | SL01 (60 mg/kg) | | SL02 (60 mg/kg) | | AS01 (60 mg/kg) | | SL03 (60 mg/kg) | |
|---|---|---|---|---|---|---|---|---|
| | Mean ketamine release (%) | S.D. (n = 3) | Mean ketamine release (%) | S.D. (n = 3) | Mean ketamine release (%) | S.D. (n = 3) | Mean ketamine release (%) | S.D. (n = 3) |
| 3 | 97.03 | 1.69 | 84.47 | 9.74 | 62.02 | 6.55 | 79.71 | 18.55 |
| 7 | 98.89 | 0.20 | 97.18 | 2.24 | 90.14 | 4.39 | 98.16 | 3.16 |
| 10 | 99.52 | 0 | 97.69 | 0 | 96.51 | 1.77 | 99.44 | 0.78 |
| 14 | - | - | 99.89 | 0 | 99.70 | 0 | - | - |

Table 27. Mean ketamine releasing percentages after injection of SL02, SL04, AS02, AS03 or AS05 in minipigs

| Time (days) | SL02 (3 mg/kg) | | SL04 (6 mg/kg) | | AS02 (6 mg/kg) | | AS03 (10.3 mg/kg) | | AS05 (3 mg/kg) | |
|---|---|---|---|---|---|---|---|---|---|---|
| | Mean ketamin e release (%) | S.D. (n = 3) | Mean ketamine release (%) | S.D. (n = 3) | Mean ketamine release (%) | S.D. (n = 3) | Mean ketamine release (%) | S.D. (n = 3) | Mean ketamine release (%) | S.D. (n = 3) |
| 0 | 0 | 0 | 0 | 0 | 0 | 0 | 0 | 0 | 0 | 0 |
| 0.083 | 2.67 | 0.76 | 1.28 | 0.19 | 0.28 | 0.21 | 0.71 | 0.12 | 0.70 | 0.30 |
| 0.167 | 7.28 | 2.07 | 3.64 | 0.28 | - | - | 1.97 | 0.24 | - | - |
| 0.25 | 10.72 | 3.06 | 5.34 | 0.08 | 1.18 | 0.76 | 2.93 | 0.24 | 3.35 | 1.35 |
| 1 | 29.34 | 7.88 | 13.49 | 0.68 | 4.40 | 1.67 | 9.62 | 0.23 | 17.72 | 1.95 |
| 2 | 42.62 | 8.93 | 19.74 | 0.89 | - | - | - | - | - | - |
| 3 | 54.39 | 8.83 | 25.59 | 0.75 | 15.86 | 4.14 | 23.93 | 2.55 | 57.45 | 1.11 |
| 4 | 65.12 | 9.07 | 30.28 | 1.45 | - | - | - | - | - | - |
| 5 | 76.72 | 10.52 | 34.49 | 1.59 | 31.53 | 7.38 | 38.37 | 3.70 | - | - |
| 6 | 85.91 | 10.17 | 38.76 | 1.57 | - | - | - | - | - | - |
| 7 | 92.35 | 6.37 | 42.72 | 2.13 | 46.58 | 9.15 | 52.03 | 3.48 | 95.28 | 1.07 |
| 8 | 97.24 | 2.47 | 46.48 | 2.89 | - | - | - | - | - | - |
| 9 | 98.80 | 0.80 | 49.43 | 3.08 | - | - | - | - | - | - |
| 10 | 99.76 | 0 | 52.15 | 3.68 | 63.32 | 9.75 | 69.45 | 3.13 | 99.92 | 0.00 |
| 12 | - | - | 57.10 | 4.73 | - | - | - | - | - | - |
| 14 | - | - | 62.14 | 6.25 | 78.77 | 9.15 | 87.02 | 0.77 | - | - |
| 21 | - | - | 77.57 | 10.39 | 92.81 | 6.01 | 99.93 | 0.03 | - | - |
| 28 | - | - | 86.90 | 8.85 | 96.28 | 4.28 | - | - | - | - |

Example 24: *In vivo* antidepressant effects

Depression-like animal model induced by dexamethasone (hereinafter abbreviated as DEX) was used to evaluate the antidepressant effects of ketamine HCl (KET), *R, S*-ketamine pamoate salt (KEP), *S*-ketamine pamoate salt (S-KEP), and *R*-ketamine pamoate salt (R-KEP) at equivalent dose (120 mg/kg ketamine free-base). According to literature, depressive-like behaviors were observed on juvenile mice and adult mice which neonatally exposed to DEX. Protocol of this example was shown in FIG. 43 and described in detail below.

Neonatal ICR mice were intraperitoneally injected with saline or DEX on postnatal day 1, 2, and 3 (P1 to P3) at doses of 0.5 mg/kg, 0.3 mg/kg, and 0.1 mg/kg, respectively. The mice received saline were called as Control group, and the mice received DEX were divided into the groups of KET, KEP, S-KEP, R-KEP and Saline (n = 10 to 14 mice in each group). The mice of KET, KEP, S-KEP, and R-KEP groups were subcutaneously injected with KET formulation, the formulation AS04 comprising KEP, the formulation AS06 comprising S-KEP, and the formulation AS07 comprising R-KEP at postnatal day 35, respectively. The KET formulation was 20% (w/w) ketamine HCl dissolved in 0.9% saline. The formulations AS04, AS06 and AS07 were prepared in Example 18. Saline group mice were subcutaneously injected with equal volumes of 0.9% saline.

Antidepressant effects were evaluated by forced swimming test (FST), which performed about every 10 days from the first day (P36) to the 63rd day (P98) after drug administration. All groups of mice were trained for swimming before

drug administration. During the FST, mice were individually placed into 5 L glass cylinders (height 27 cm, diameter 18 cm) filled with 4 L of water (23±1°C). The total duration of immobility time during 5 minutes of FST was observed. Results were presented as mean±SEM. Student's t-test was analyzed with the Saline group (DEX-treated mouse group injected with saline) versus the other groups at each time point. * $p < 0.05$, ** $p < 0.01$, *** $p < 0.001$, and **** $p < 0.0001$ indicate significant differences compared to the Saline group.

Mice neonatally exposed to DEX showed significant increase of immobility time on FST compared to the Control group. The results of FST were showed in FIG. 44, and it was observed that both of KET and KEP reduced the immobility time that was increased in DEX-treated mice 1 day after administration. Moreover, KEP sustainably reduced the immobility time to at least 52 days after administration, and this effect was getting to be more significant. By contrast, KET only reduced the immobility time from 1 to 10 days after injection, and this effect could not be sustained during 11 to 21 days post injection.

Furthermore, sedation behavior was also evaluated by rodent sedation rating scale (Table 28) immediately post drug administration to 28 days in this study. As shown in FIG. 45, mice in the KET group immediately showed mild sedation-related behavior after injection, and this effect was fully recovered 2 hours post administration. Mice treated with KEP showed normal behavior from 0 to 28 days post-injection.

Table 28. Rodent sedation rating scale contents

| Score | Rating Content |
|---|---|
| 5-awake, active | Engaged in locomotion, rearing, head movements or grooming |
| 4-awake, inactive | Eyes fully open, head up, little to no locomotion, rearing or grooming, normal posture |
| 3-mild sedation | Eyes partly closed, head somewhat down, impaired locomotion including abnormal posture, use of only some limbs, dragging and stumbling |
| 2-moderate sedation | Head mostly or completely down, eyes partly closed, flattened posture, no spontaneous movement |
| 1-heavy sedation | Eyes mostly closed, loss of righting reflex |
| 0-asleep | Eyes fully closed, body relaxed, asleep |

In consequence, KET, KEP, S-KEP, and R-KEP all revealed rapid-onset antidepressant effects on FST after single injection at equivalent dose (120 mg/kg ketamine free-base), and this effect sustained for at least 10 days on DEX-treated mice. Surprisingly, in the KEP, S-KEP, and R-KEP groups, sedation or other ketamine-related psychotomimetic effects and nervous system disorders would not occur post administration, implying that KEP, S-KEP, and R-KEP had additional beneficial properties for being used as an antidepressant in comparison with KET.

The present disclosure has been described using exemplary embodiments in detail in the above. However, it is to be understood that the scope of the disclosure is not limited to the disclosed embodiments.

Reference List

1. Han, Felicity Y., et al. "Sustained-release ketamine-loaded nanoparticles fabricated by sequential nanoprecipitation." International Journal of Pharmaceutics (2020): 119291.
2. Way, Walter L. "Ketamine - its pharmacology and therapeutic uses." Anesthesiology: The Journal of the American Society of Anesthesiologists 56.2 (1982): 119-136.

## Claims

1. A sustained-release pharmaceutical composition comprising a crystal or an amorphous form of a ketamine pamoate salt, and a pharmaceutically acceptable carrier thereof, wherein the ketamine pamoate salt is selected from the group consisting of *R, S*-ketamine pamoate salt, *S*-ketamine pamoate salt, *R*-ketamine pamoate salt, and any combination thereof, and the ketamine pamoate salt is a ketamine pamoate salt having a stoichiometry of 2:1 of ketamine to pamoate, a ketamine pamoate salt having a stoichiometry of 1:1 of ketamine to pamoate, or a combination thereof, wherein the pharmaceutically acceptable carrier is selected from the group consisting of *N*-methyl-2-pyrrolidone, ethyl acetate, ethanol, butanol, 2-butanol, isobutanol, isopropanol, glycerin, benzyl benzoate, dimethyl sulfoxide,

*N,N*-dimethylacetamide, propylene glycol, dimethyl glycol, benzyl alcohol, polyethylene glycol 4000 (PEG4000), polysorbate 80 (Tween 80), sodium carboxymethyl cellulose, poly lactic acid, poly(lactic-co-glycolic) acid, and any combination thereof.

**2.** The sustained-release pharmaceutical composition according to claim 1, which is an injectable aqueous suspension, wherein the pharmaceutically acceptable carrier is polyethylene glycol 4000 (PEG4000), polysorbate 80 (Tween 80), sodium carboxymethyl cellulose, or any combination thereof.

**3.** The sustained-release pharmaceutical composition according to claim 2, wherein the injectable aqueous suspension has an average particle size (d50) of less than 20 $\mu$m and a specific surface area of more than 300 $m^2/g$, measured by Bettersizer S2-E laser particle size analyzer.

**4.** The sustained-release pharmaceutical composition according to claim 1, which is an injectable solution, wherein the pharmaceutically acceptable carrier is *N*-methyl-2-pyrrolidone, ethyl acetate, ethanol, butanol, 2-butanol, isobutanol, isopropanol, glycerin, benzyl benzoate, dimethyl sulfoxide, *N,N*-dimethylacetamide, propylene glycol, dimethyl glycol, benzyl alcohol, or any combination thereof.

**5.** The sustained-release pharmaceutical composition according to claim 4, further comprising at least one of palmitic acid, oleic acid, stearic acid, decanoic acid, and linoleic acid.

**6.** The sustained-release pharmaceutical composition according to claim 1, which is an injectable matrix delivery system, wherein the pharmaceutically acceptable carrier is poly lactic acid, poly(lactic-co-glycolic) acid, or a combination thereof.

**7.** The sustained-release pharmaceutical composition according to claim 6, further comprising at least one of *N*-methyl-2-pyrrolidone, ethyl acetate, ethanol, butanol, 2-butanol, isobutanol, isopropanol, glycerin, benzyl benzoate, dimethyl sulfoxide, *N,N*-dimethylacetamide, propylene glycol, dimethyl glycol, benzyl alcohol, or any combination thereof.

**8.** The sustained-release pharmaceutical composition according to claim 1, further comprising one or more additional agents selected from the group consisting of a wetting agent, a suspending agent, a tonicity adjusting agent, a pH adjusting agent, a buffering agent, an antioxidant, and a preservative.

**9.** The sustained-release pharmaceutical composition according to claim 1, wherein the ketamine pamoate salt is present at a concentration of 1% to 99% w/w, preferably of 5% to 60% w/w.

**10.** The sustained-release pharmaceutical composition according to claim 1, which is formulated for subcutaneous, intramuscular or intradermal injection.

**11.** A sustained-release pharmaceutical composition according to claim 1 for use in treating a disease or a condition, comprising administering to a subject in need thereof the sustained-release pharmaceutical composition, wherein the disease or the condition is selected from the group consisting of a central nervous system disease, depression, anti-inflammatory, pain, and any combination thereof, preferably the group consisting of major depressive disorder (MDD), treatment-resistant depression (TRD), suicidal ideation, bipolar disorder, obsessive-compulsive disorder, posttraumatic stress disorder (PTSD), autism spectrum disorder, tinnitus, refractory chronic migraine, asthma, anxiety, substance use disorder, alcohol use disorder, eating disorder, refractory status epilepticus, brain ischemia, Alzheimer's disease, Parkinson's disease, stroke, traumatic brain injury, multiple sclerosis, and any combination thereof.

**12.** A sustained-release pharmaceutical composition according to claim 1 for use in anesthetizing a subject in need thereof, comprising administering to the subject the sustained-release pharmaceutical composition.

### Patentansprüche

**1.** Pharmazeutische Zusammensetzung mit verzögerter Freisetzung, umfassend eine Kristall- oder eine amorphe Form eines Ketaminpamoatsalzes, und einen pharmazeutisch akzeptablen Träger davon, wobei das Ketaminpamoatsalz aus einer Gruppe bestehend aus R, S-Ketaminpamoatsalz, S-Ketaminpamoatsalz, R-Ketaminpamoatsalz, und einer beliebigen Kombination davon ausgewählt ist, und wobei das Ketaminpamoatsalz ein Ketaminpamoatsalz mit einem

stöchiometrischen Verhältnis von Ketamin zu Pamoat von 2:1, ein Ketaminpamoatsalz mit einem stöchiometrischen Verhältnis von Ketamin zu Pamoat von 1:1, oder eine Kombination davon ist,
wobei der pharmazeutisch akzeptable Träger aus einer Gruppe bestehend aus N-Methyl-2-pyrrolidon, Ethylacetat, Ethanol, Butanol, 2-Butanol, Isobutanol, Isopropanol, Glycerin, Benzylbenzoat, Dimethylsulfoxid, N,N-Dimethylacetamid, Propylenglykol, Dimethylglykol, Benzylalkohol, Polyethylenglykol 4000 (PEG4000), Polysorbat 80 (Tween 80), Natriumcarboxymethylcellulose, Polymilchsäure, Poly(lactid-co-glycolid)säure und einer beliebigen Kombination davon ausgewählt ist.

2. Pharmazeutische Zusammensetzung mit verzögerter Freisetzung gemäß Anspruch 1, die eine injizierbare wässrige Suspension ist, wobei der pharmazeutische akzeptable Träger Polyethylenglykol 4000 (PEG4000), Polysorbat 80 (Tween 80), Natriumcarboxymethylcellulose oder eine beliebige Kombination davon ist.

3. Pharmazeutische Zusammensetzung mit verzögerter Freisetzung gemäß Anspruch 2, wobei die injizierbare wässrige Suspension eine durchschnittliche Partikelgröße (d50) von weniger als 20 $\mu$m und eine spezifische Oberfläche von mehr als 300 $m^2/g$, gemessen mit dem Bettersizer S2-E Laser-Partikelgrößenanalysator, aufweist.

4. Pharmazeutische Zusammensetzung mit verzögerter Freisetzung gemäß Anspruch 1, die eine injizierbare Suspension ist, wobei der pharmazeutisch akzeptable Träger N-Methyl-2-pyrrolidon, Ethylacetat, Ethanol, Butanol, 2-Butanol, Isobutanol, Isopropanol, Glycerin, Benzylbenzoat, Dimethylsulfoxid, N,N-Dimethylacetamid, Propylenglykol, Dimethylglykol, Benzylalkohol oder eine beliebige Kombination davon ist.

5. Pharmazeutische Zusammensetzung mit verzögerter Freisetzung gemäß Anspruch 4, ferner umfassend mindestens eine von Palmitinsäure, Ölsäure, Stearinsäure, Decansäure und Linolsäure.

6. Pharmazeutische Zusammensetzung mit verzögerter Freisetzung gemäß Anspruch 1, die ein injizierbares Matrix-Abgabesystem ist, wobei der pharmazeutisch akzeptable Träger eine Polymilchsäure, Poly(lactid-co-glycolid)säure, oder eine Kombination davon ist.

7. Pharmazeutische Zusammensetzung mit verzögerter Freisetzung gemäß Anspruch 6, ferner umfassend mindestens eines von N-Methyl-2-pyrrolidon, Ethylacetat, Ethanol, Butanol, 2-Butanol, Isobutanol, Isopropanol, Glycerin, Benzylbenzoat, Dimethylsulfoxid, N,N-Dimethylacetamid, Propylenglykol, Dimethylglykol, Benzylalkohol oder eine beliebige Kombination davon.

8. Pharmazeutische Zusammensetzung mit verzögerter Freisetzung gemäß Anspruch 1, ferner umfassend eines oder mehrere zusätzliche Mittel, ausgewählt aus einer Gruppe bestehend aus einem Benetzungsmittel, einem Suspensionsmittel, einem Tonizitäts-Anpassungsmittel, einem pH-Wert-Anpassungsmittel, einem Puffermittel, einem Antioxidationsmittel und einem Konservierungsmittel.

9. Pharmazeutische Zusammensetzung mit verzögerter Freisetzung gemäß Anspruch 1, wobei das Ketaminpamoatsalz in einer Konzentration von 1 bis 99 Gew.-%, bevorzugt von 5 bis 60 Gew.-%, vorliegt.

10. Pharmazeutische Zusammensetzung mit verzögerter Freisetzung gemäß Anspruch 1, die zur subkutanen, intramuskulären oder intradermalen Injektion formuliert ist.

11. Pharmazeutische Zusammensetzung mit verzögerter Freisetzung gemäß Anspruch 1 zur Verwendung bei der Behandlung einer Krankheit oder eines Zustands, umfassend die Verabreichung der pharmazeutischen Zusammensetzung mit verzögerter Freisetzung an einen Patienten, der diese benötigt, wobei die Krankheit oder der Zustand aus der Gruppe bestehend aus einer Zentralnervensystemerkrankung, Depression, entzündungshemmend, Schmerz und jeder Kombination davon, bevorzugt aus der Gruppe bestehend aus einer schweren depressiven Störung (MDD), einer behandlungsresistenten Depression (TRD), Suizidgedanken, bipolare Störung, Zwangsstörung, posttraumatische Belastungsstörung (PTSD), Autismus-Spektrum-Störung, Tinnitus, refraktäre chronische Migräne, Asthma, Angst, Substanzgebrauchsstörung, Alkoholgebrauchsstörung, Essstörung, refraktärer Status epilepticus, Hirnischämie, Alzheimer-Krankheit, Parkinson-Krankheit, Schlaganfall, traumatische Hirnverletzung, Multiple Sklerose und jede Kombination davon ausgewählt ist.

12. Pharmazeutische Zusammensetzung mit verzögerter Freisetzung gemäß Anspruch 1 zur Verwendung bei der Anästhesie eines Patienten, der diese benötigt, umfassend die Verabreichung der pharmazeutischen Zusammensetzung mit verzögerter Freisetzung an den Patienten.

## Revendications

1. Composition pharmaceutique à libération prolongée comprenant un cristal ou une forme amorphe d'un sel de pamoate de kétamine, et un véhicule pharmaceutiquement acceptable de celle-ci, dans laquelle le sel de pamoate de kétamine est choisi dans le groupe consistant en sel de pamoate de R,S-kétamine, sel de pamoate de S-kétamine, sel de pamoate de R-kétamine, et une quelconque combinaison de ceux-ci, et le sel de pamoate de kétamine est un sel de pamoate de kétamine présentant une stoechiométrie de 2 kétamine pour 1 pamoate, un sel de pamoate de kétamine présentant une stœchiométrie de 1 kétamine pour 1 pamoate, ou une combinaison de ceux-ci,
dans laquelle le véhicule pharmaceutiquement acceptable est choisi dans le groupe consistant en *N*-méthyl-2-pyrrolidone, acétate d'éthyle, éthanol, butanol, 2-butanol, isobutanol, isopropanol, glycérine, benzoate de benzyle, diméthylsulfoxyde, N,N-diméthylacétamide, propylène glycol, diméthylglycol, alcool benzylique, polyéthylène glycol 4000 (PEG 4000), polysorbate 80 (Tween 80), carboxyméthylcellulose de sodium, acide polylactique, acide poly(lactique-co-glycolique), et une quelconque combinaison de ceux-ci.

2. Composition pharmaceutique à libération prolongée selon la revendication 1, qui est une suspension aqueuse injectable, dans laquelle le véhicule pharmaceutiquement acceptable est du polyéthylène glycol 4000 (PEG 4000), du polysorbate 80 (Tween 80), du carboxyméthylcellulose de sodium, ou une quelconque combinaison de ceux-ci.

3. Composition pharmaceutique à libération prolongée selon la revendication 2, dans laquelle la suspension aqueuse injectable présente une taille de particule moyenne (d50) de moins de 20 $\mu$m et une surface spécifique de plus de 300 $m^2$/g, mesurées par un analyseur de taille de particules laser Bettersizer S2-E.

4. Composition pharmaceutique à libération prolongée selon la revendication 1, qui est une solution injectable, dans laquelle le véhicule pharmaceutiquement acceptable est du *N*-méthyl-2-pyrrolidone, de l'acétate d'éthyle, de l'éthanol, du butanol, du 2-butanol, de l'isobutanol, de l'isopropanol, de la glycérine, du benzoate de benzyle, du diméthylsulfoxyde, du N,N-diméthylacétamide, du propylène glycol, du diméthylglycol, de l'alcool benzylique, ou une quelconque combinaison de ceux-ci.

5. Composition pharmaceutique à libération prolongée selon la revendication 4, comprenant en outre au moins un d'acide palmitique, d'acide oléique, d'acide stéarique, d'acide décanoïque et d'acide linoléique.

6. Composition pharmaceutique à libération prolongée selon la revendication 1, qui est un système de distribution de matrice injectable, dans laquelle le véhicule pharmaceutiquement acceptable est de l'acide polylactique, de l'acide poly(lactique-co-glycolique), ou une combinaison de ceux-ci.

7. Composition pharmaceutique à libération prolongée selon la revendication 6, comprenant en outre au moins un de *N*-méthyl-2-pyrrolidone, d'acétate d'éthyle, d'éthanol, de butanol, de 2-butanol, d'isobutanol, d'isopropanol, de glycérine, de benzoate de benzyle, de diméthylsulfoxyde, de N,N-diméthylacétamide, de propylène glycol, de diméthylglycol, d'alcool benzylique, ou une quelconque combinaison de ceux-ci.

8. Composition pharmaceutique à libération prolongée selon la revendication 1, comprenant en outre un ou plusieurs agents supplémentaires choisis dans le groupe consistant en un agent mouillant, un agent de suspension, un agent d'ajustement de la tonicité, un agent d'ajustement du pH, un agent tampon, un antioxydant et un agent de conservation.

9. Composition pharmaceutique à libération prolongée selon la revendication 1, dans laquelle le sel de pamoate de kétamine est présent à une concentration de 1 % à 99 % M/M, de préférence de 5 % à 60 % M/M.

10. Composition pharmaceutique à libération prolongée selon la revendication 1, qui est formulée pour une injection sous-cutanée, intramusculaire ou intradermique.

11. Composition pharmaceutique à libération prolongée selon la revendication 1 destinée à être utilisée dans le traitement d'une maladie ou d'une condition, comprenant l'administration de la composition pharmaceutique à libération prolongée à un sujet en ayant besoin, dans laquelle la maladie ou la condition est choisie dans le groupe consistant en une maladie du système nerveux central, une dépression, une anti-inflammatoire, une douleur, et une quelconque combinaison de celles-ci, de préférence le groupe consistant en un trouble dépressif caractérisé (MDD), une dépression résistante au traitement (TRD), une idée suicidaire, un trouble bipolaire, un trouble obsessionnel compulsif, un trouble de stress post-traumatique (PTSD), un trouble du spectre de l'autisme, un acouphène, une

migraine chronique réfractaire, l'asthme, l'anxiété, la toxicomanie, la dépendance à l'alcool, un trouble alimentaire, un état de mal épileptique, une ischémie cérébrale, la maladie d'Alzheimer, la maladie de Parkinson, un accident vasculaire cérébral, un traumatisme cérébral, une sclérose en plaques, et une quelconque combinaison de ceux-ci.

**12.** Composition pharmaceutique à libération prolongée selon la revendication 1 destinée à être utilisée au cours de l'anesthésie d'un sujet en ayant besoin, comprenant l'administration au sujet de la composition pharmaceutique à libération prolongée.

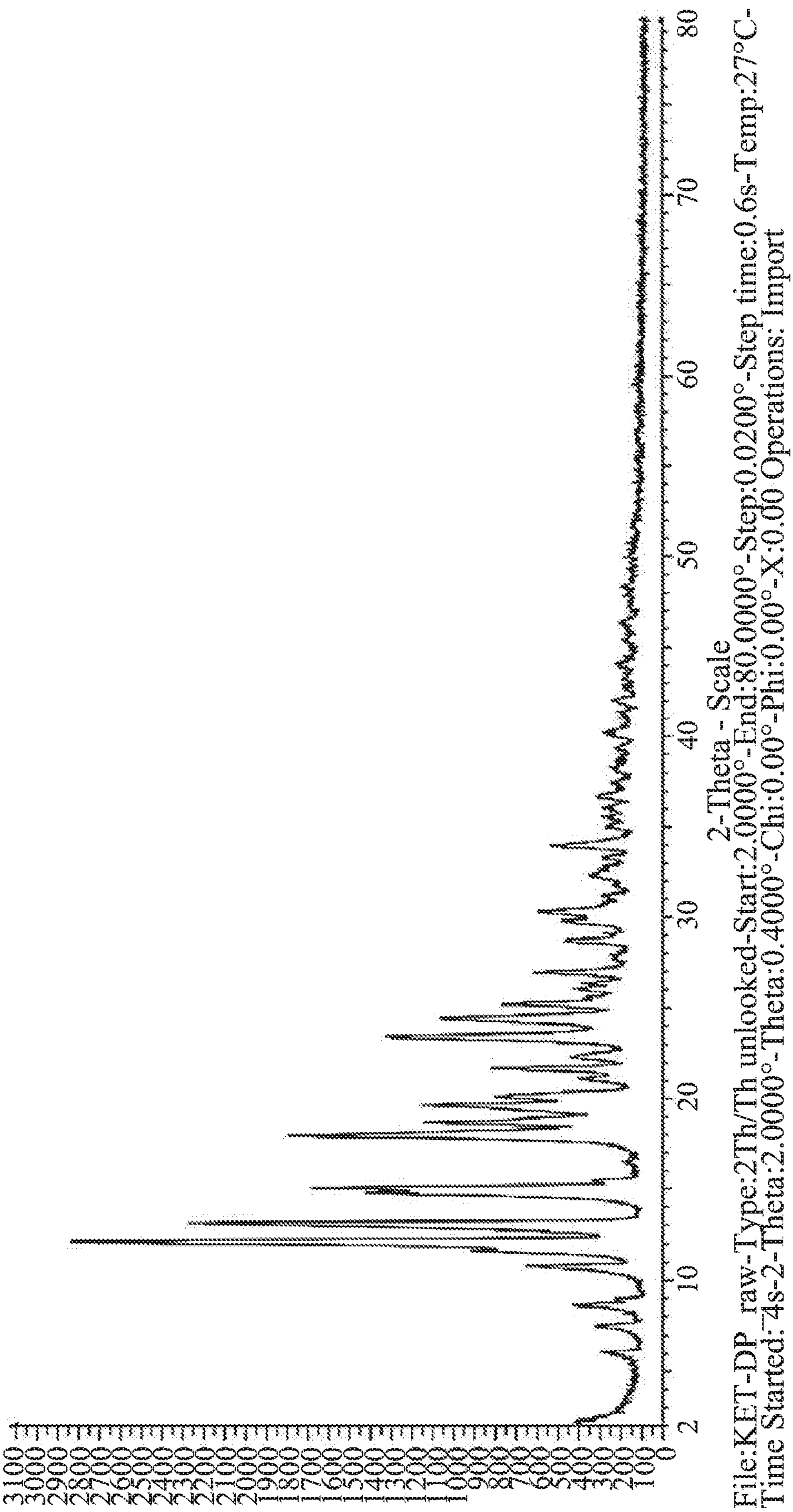
Lin (Counts)
3100
3000
2900
2800
2700
2600
2500
2400
2300
2200
2100
2000
1900
1800
1700
1600
1500
1400
1300
1200
1100
1000
900
800
700
600
500
400
300
200
100
0
2
10
20
30
40
50
60
70
80
2-Theta - Scale
File:KET-DP_raw-Type:2Th/Th unlooked-Start:2.0000°-End:80.0000°-Step:0.0200°-Step time:0.6s-Temp:27°C-
Time Started:-4s-2-Theta:2.0000°-Theta:0.4000°-Chi:0.00°-Phi:0.00°-X:0.00 Operations: Import

FIG. 1

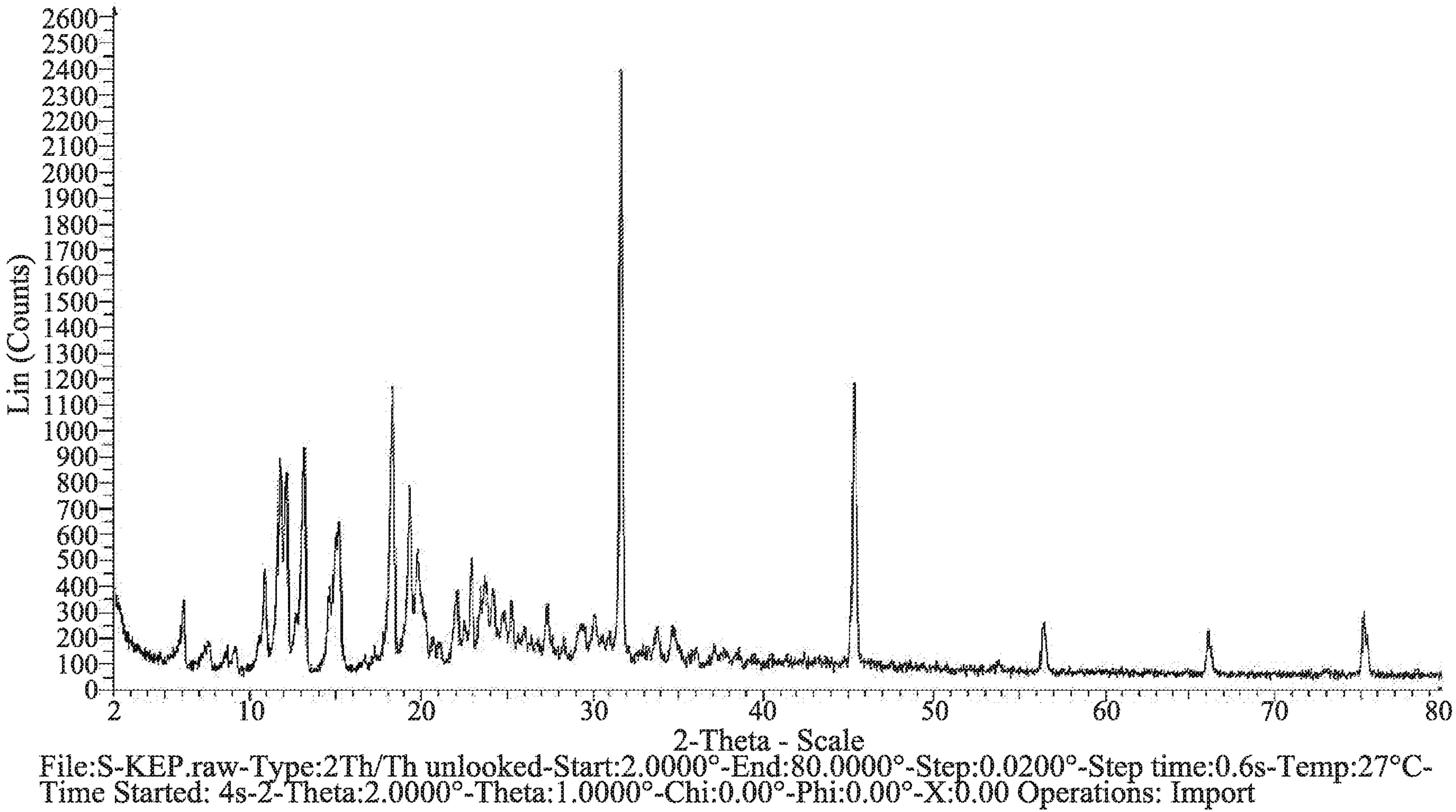
Lin (Counts)
2600
2500
2400
2300
2200
2100
2000
1900
1800
1700
1600
1500
1400
1300
1200
1100
1000
900
800
700
600
500
400
300
200
100
0
2
10
20
30
40
50
60
70
80
2-Theta - Scale
File:S-KEP.raw-Type:2Th/Th unlooked-Start:2.0000°-End:80.0000°-Step:0.0200°-Step time:0.6s-Temp:27°C-
Time Started: 4s-2-Theta:2.0000°-Theta:1.0000°-Chi:0.00°-Phi:0.00°-X:0.00 Operations: Import

FIG. 2

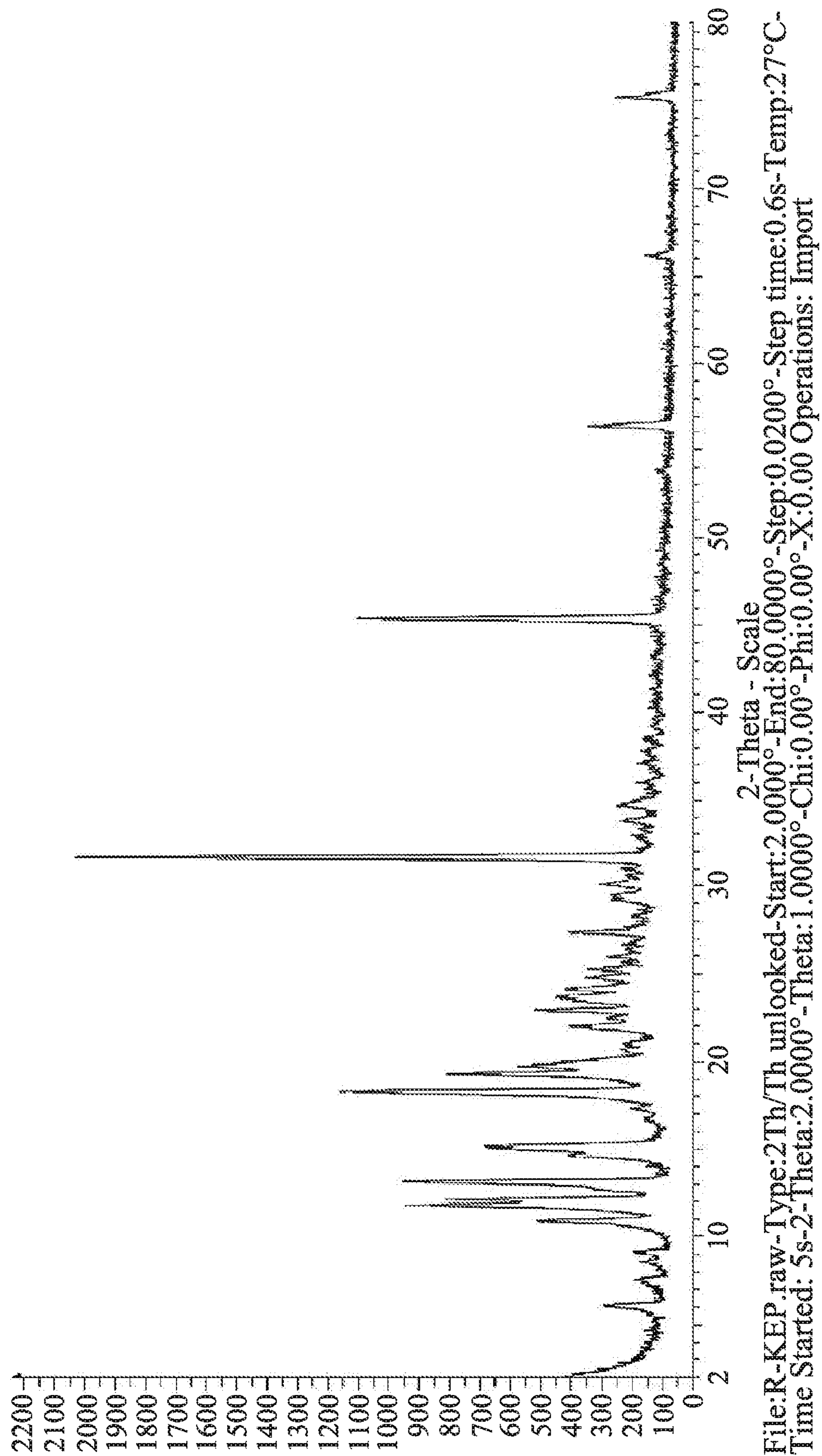
Lin (Counts)
2200
2100
2000
1900
1800
1700
1600
1500
1400
1300
1200
1100
1000
900
800
700
600
500
400
300
200
100
0
2
10
20
30
40
50
60
70
80
2-Theta - Scale
File:R-KEP.raw-Type:2Th/Th unlooked-Start:2.0000°-End:80.0000°-Step:0.0200°-Step time:0.6s-Temp:27°C-
Time Started: 5s-2-Theta:2.0000°-Theta:1.0000°-Chi:0.00°-Phi:0.00°-X:0.00 Operations: Import

FIG. 3

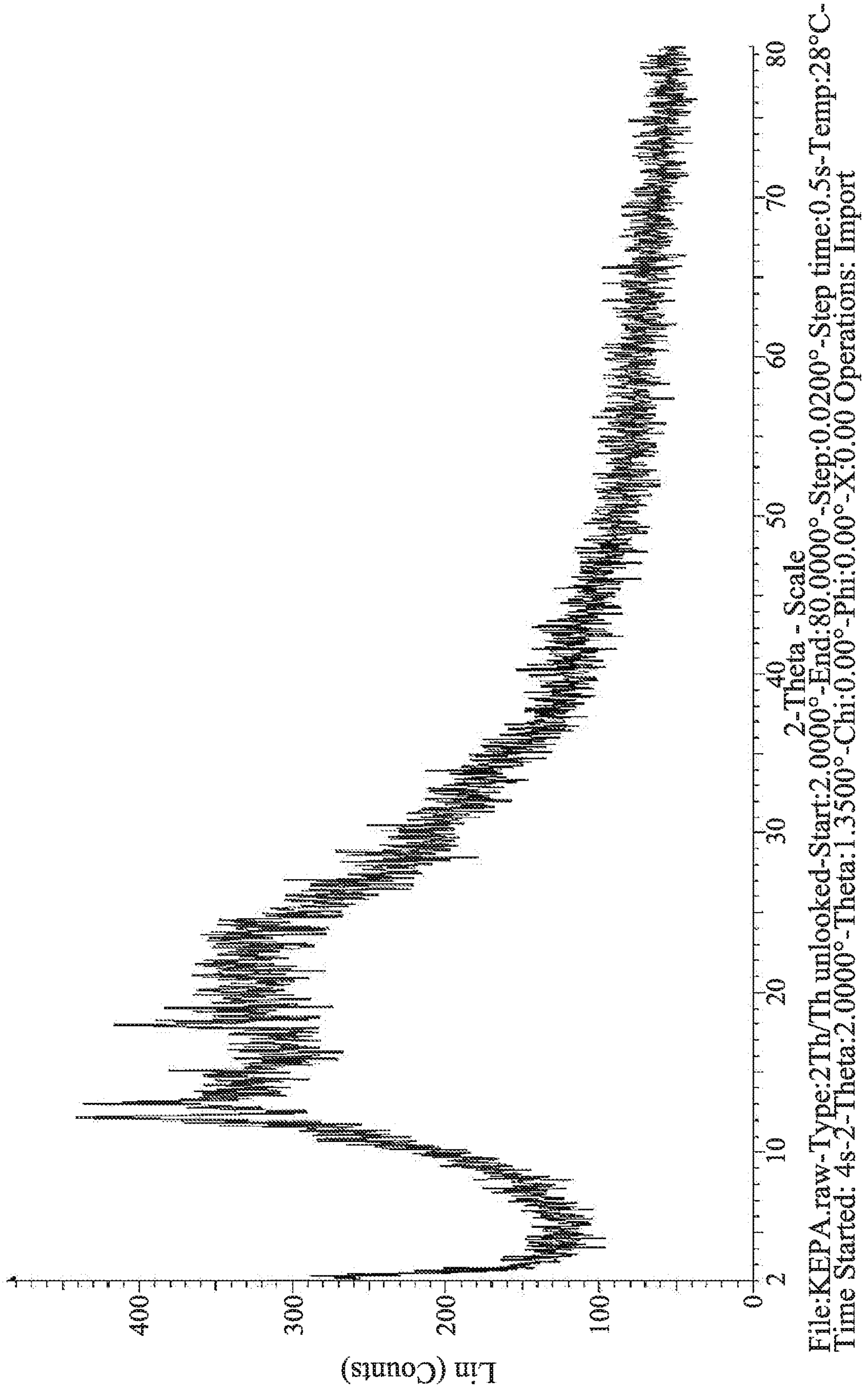
Lin (Counts)
0
100
200
300
400
2
10
20
30
40
50
60
70
80
2-Theta - Scale
File:KEPA.raw-Type:2Th/Th unlooked-Start:2.0000°-End:80.0000°-Step:0.0200°-Step time:0.5s-Temp:28°C-
Time Started: 4s-2-Theta:2.0000°-Theta:1.3500°-Chi:0.00°-Phi:0.00°-X:0.00 Operations: Import

FIG. 4

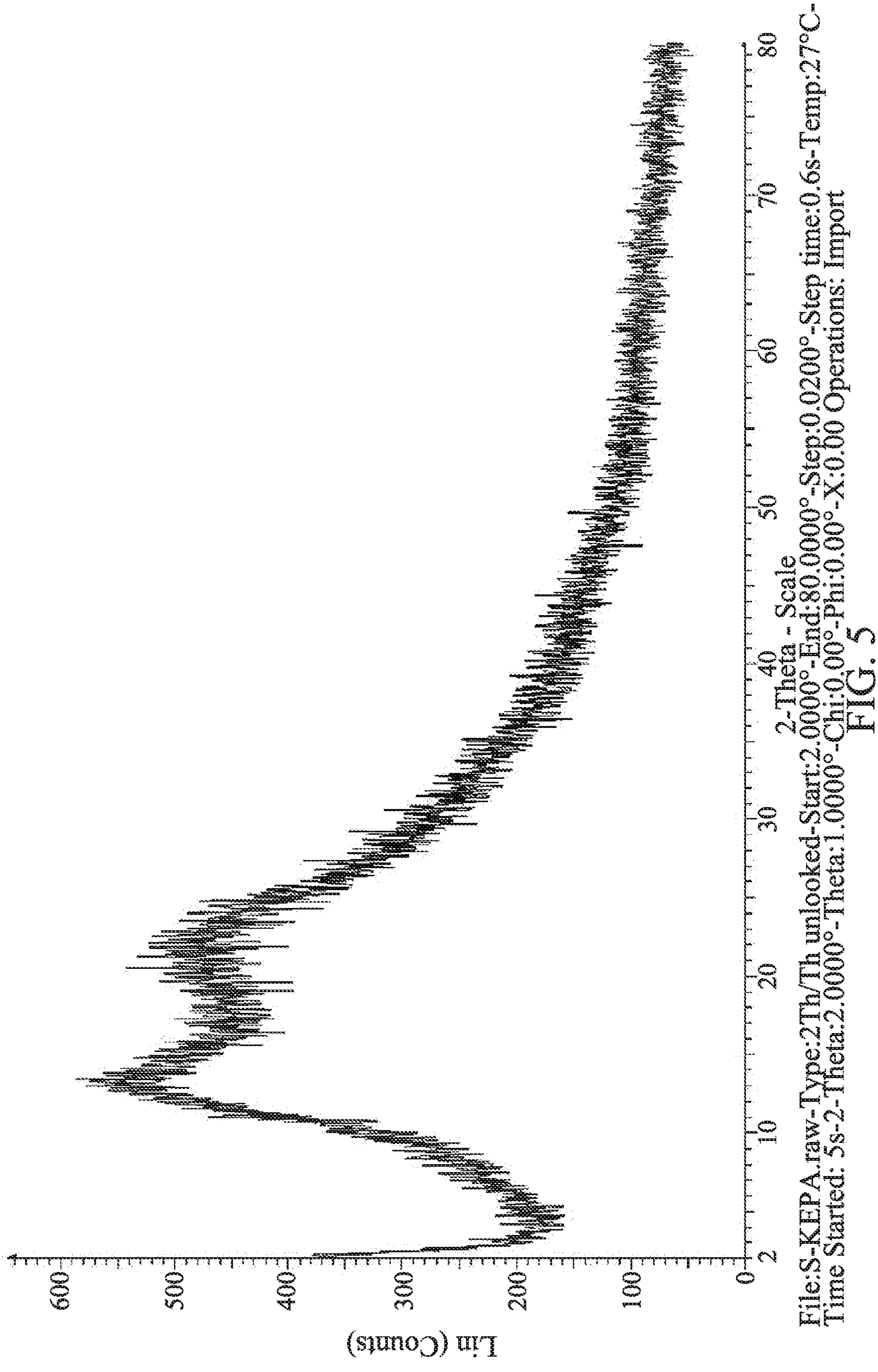
Lin (Counts)
600
500
400
300
200
100
0
2
10
20
30
40
50
60
70
80
2-Theta - Scale
File:S-KEPA.raw-Type:2Th/Th unlooked-Start:2.0000°-End:80.0000°-Step:0.0200°-Step time:0.6s-Temp:27°C-
Time Started: 5s-2-Theta:2.0000°-Theta:1.0000°-Chi:0.00°-Phi:0.00°-X:0.00 Operations: Import

FIG. 5

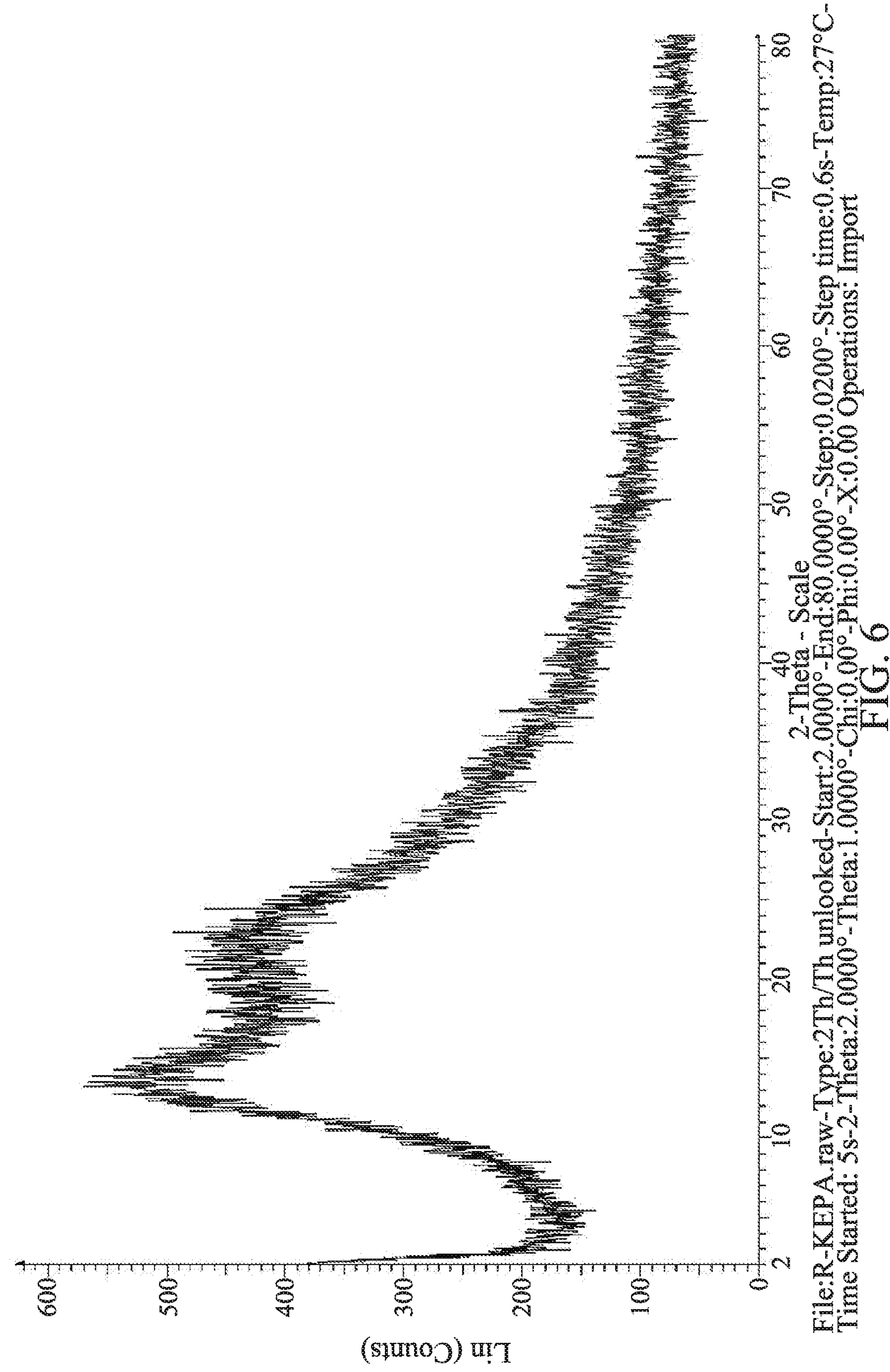
Lin (Counts)
600
500
400
300
200
100
0
2
10
20
30
40
50
60
70
80
2-Theta - Scale
File:R-KEPA.raw-Type:2Th/Th unlooked-Start:2.0000°-End:80.0000°-Step:0.0200°-Step time:0.6s-Temp:27°C-
Time Started: 5s-2-Theta:2.0000°-Theta:1.0000°-Chi:0.00°-Phi:0.00°-X:0.00 Operations: Import

FIG. 6

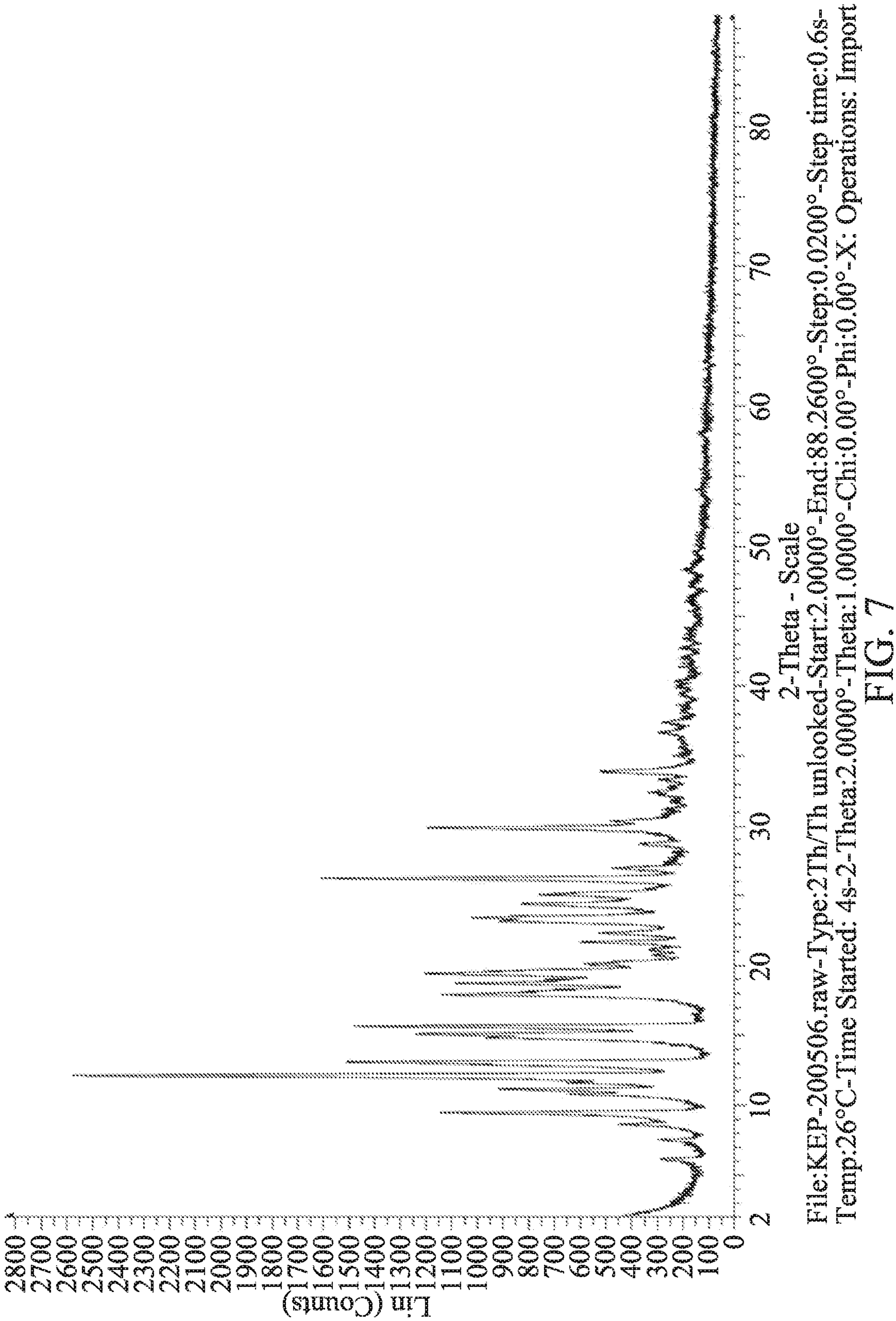
Lin (Counts)
2800
2700
2600
2500
2400
2300
2200
2100
2000
1900
1800
1700
1600
1500
1400
1300
1200
1100
1000
900
800
700
600
500
400
300
200
100
0
2
10
20
30
40
50
60
70
80
2-Theta - Scale
File:KEP-200506.raw-Type:2Th/Th unlooked-Start:2.0000°-End:88.2600°-Step:0.0200°-Step time:0.6s-
Temp:26°C-Time Started: 4s-2-Theta:2.0000°-Theta:1.0000°-Chi:0.00°-Phi:0.00°-X: Operations: Import

FIG. 7

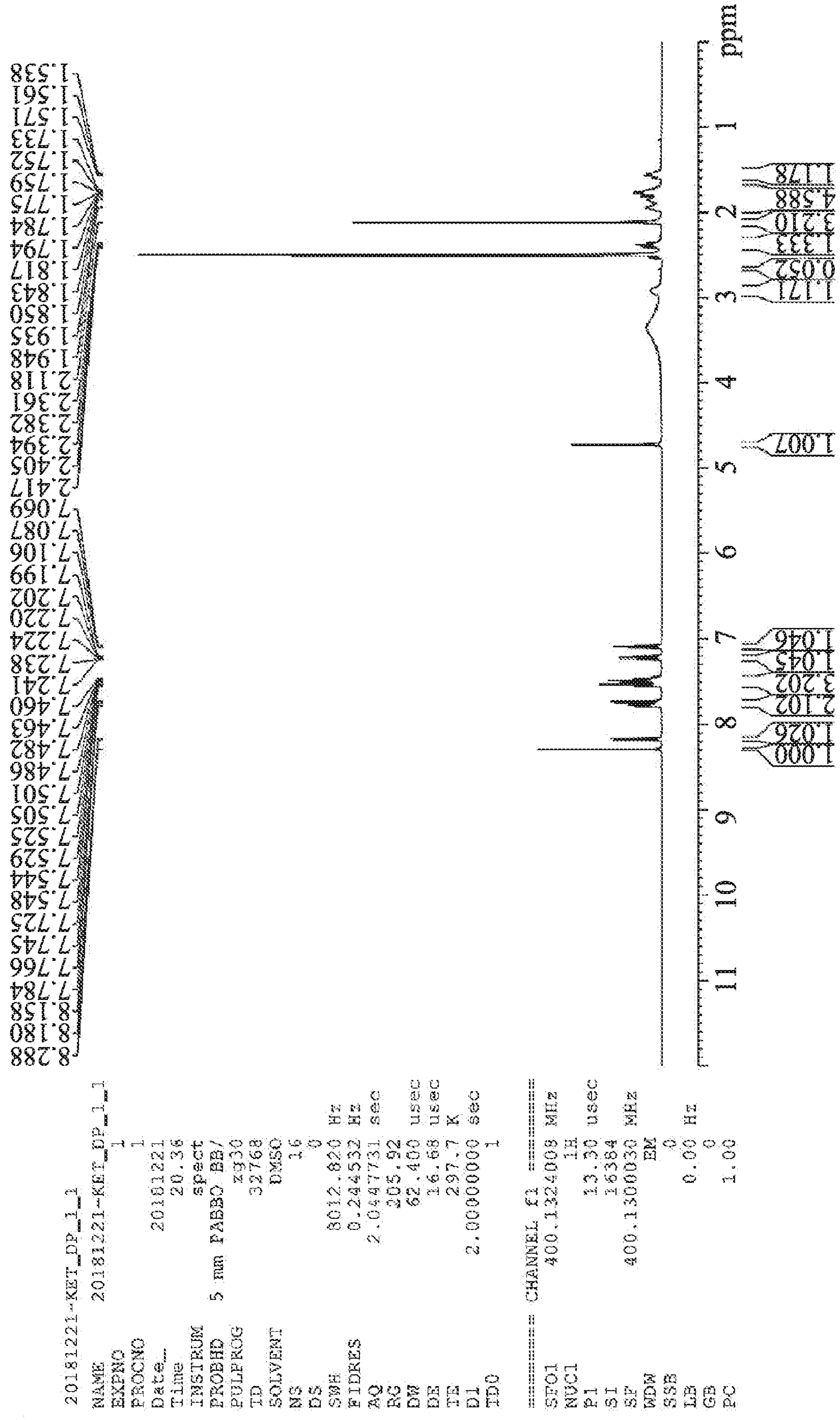

8.288
8.180
8.158
7.784
7.766
7.745
7.725
7.548
7.544
7.529
7.525
7.505
7.501
7.486
7.482
7.463
7.460
7.241
7.238
7.224
7.220
7.202
7.199
7.106
7.087
7.069
2.417
2.405
2.394
2.382
2.361
2.118
1.948
1.935
1.850
1.843
1.817
1.794
1.784
1.775
1.759
1.752
1.733
1.571
1.561
1.538
1.000
1.026
2.102
3.202
1.045
1.046
1.007
1.171
0.052
1.333
3.210
4.588
1.178
11 10 9 8 7 6 5 4 3 2 1 ppm
20181221-KET_DP_1_1
NAME 20181221-KET_DP_1_1
EXPNO 1
PROCNO 1
Date_ 20181221
Time 20.36
INSTRUM spect
PROBHD 5 mm PABBO BB/
PULPROG zg30
TD 32768
SOLVENT DMSO
NS 16
DS 0
SWH 8012.820 Hz
FIDRES 0.244532 Hz
AQ 2.0447731 sec
RG 205.92
DW 62.400 usec
DE 16.68 usec
TE 297.7 K
D1 2.00000000 sec
TD0 1
======== CHANNEL f1 ========
SFO1 400.1324008 MHz
NUC1 1H
P1 13.30 usec
SI 16384
SF 400.1300030 MHz
WDW EM
SSB 0
LB 0.00 Hz
GB 0
PC 1.00


FIG. 8

20190504-S-KEP-H

| | | |
|---|---|---|
| NAME | 20190504-S-KEP-H | |
| EXPNO | 1 | |
| PROCNO | 1 | |
| Date_ | 20190504 | |
| Time | 23.22 | |
| INSTRUM | spect | |
| PROBHD | 5 mm PABBO BB/ | |
| PULPROG | zg30 | |
| TD | 32768 | |
| SOLVENT | DMSO | |
| NS | 16 | |
| DS | 0 | |
| SWH | 8012.820 | Hz |
| FIDRES | 0.244532 | Hz |
| AQ | 2.0447731 | sec |
| RG | 43.08 | |
| DW | 62.400 | usec |
| DE | 16.68 | usec |
| TE | 296.9 | K |
| D1 | 2.00000000 | sec |
| TD0 | 1 | |

======== CHANNEL f1 ========

| | | |
|---|---|---|
| SFO1 | 400.1324008 | MHz |
| NUC1 | 1H | |
| P1 | 13.30 | usec |
| SI | 16384 | |
| SF | 400.1300034 | MHz |
| WDW | EM | |
| SSB | 0 | |
| LB | 0.00 | Hz |
| GB | 0 | |
| PC | 1.00 | |

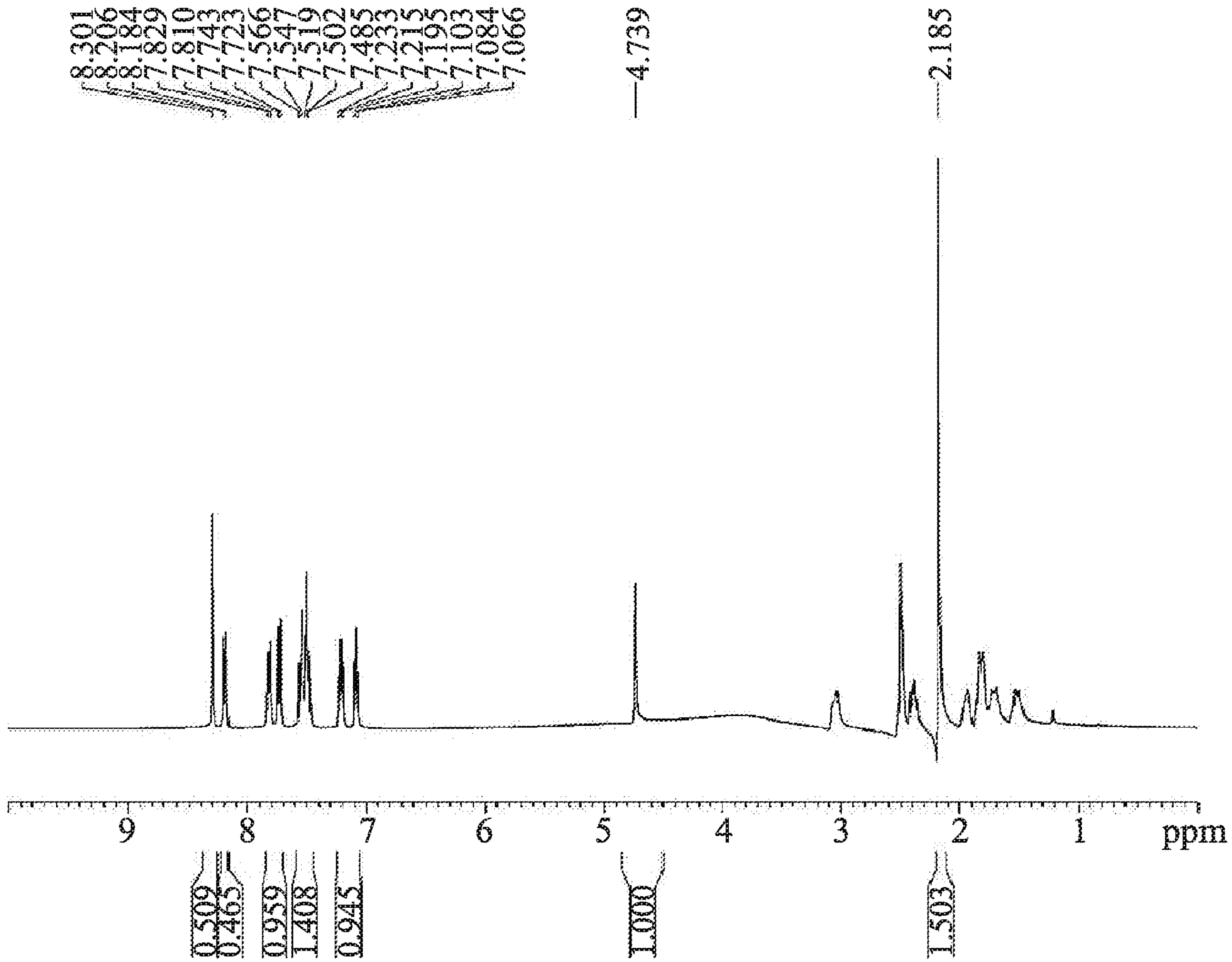


FIG. 9

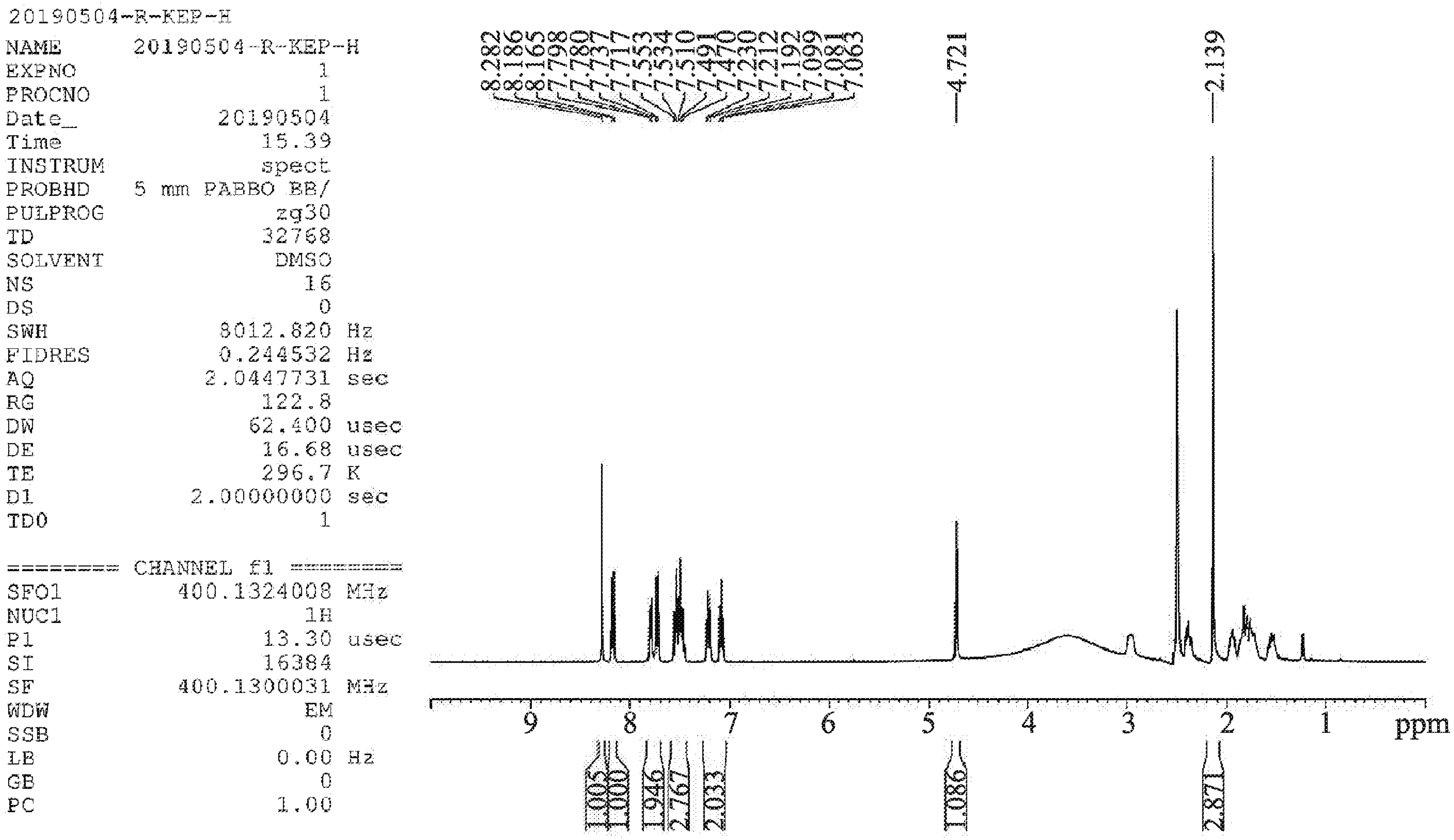
20190504-R-KEP-H
NAME 20190504-R-KEP-H
EXPNO 1
PROCNO 1
Date_ 20190504
Time 15.39
INSTRUM spect
PROBHD 5 mm PABBO BB/
PULPROG zg30
TD 32768
SOLVENT DMSO
NS 16
DS 0
SWH 8012.820 Hz
FIDRES 0.244532 Hz
AQ 2.0447731 sec
RG 122.8
DW 62.400 usec
DE 16.68 usec
TE 296.7 K
D1 2.00000000 sec
TD0 1
======== CHANNEL f1 ========
SFO1 400.1324008 MHz
NUC1 1H
P1 13.30 usec
SI 16384
SF 400.1300031 MHz
WDW EM
SSB 0
LB 0.00 Hz
GB 0
PC 1.00
8.282
8.186
8.165
7.798
7.780
7.737
7.717
7.553
7.534
7.510
7.491
7.470
7.230
7.212
7.192
7.099
7.081
7.063
4.721
2.139
9
8
7
6
5
4
3
2
1
ppm
1.005
1.000
1.946
2.767
2.033
1.086
2.871

FIG. 10

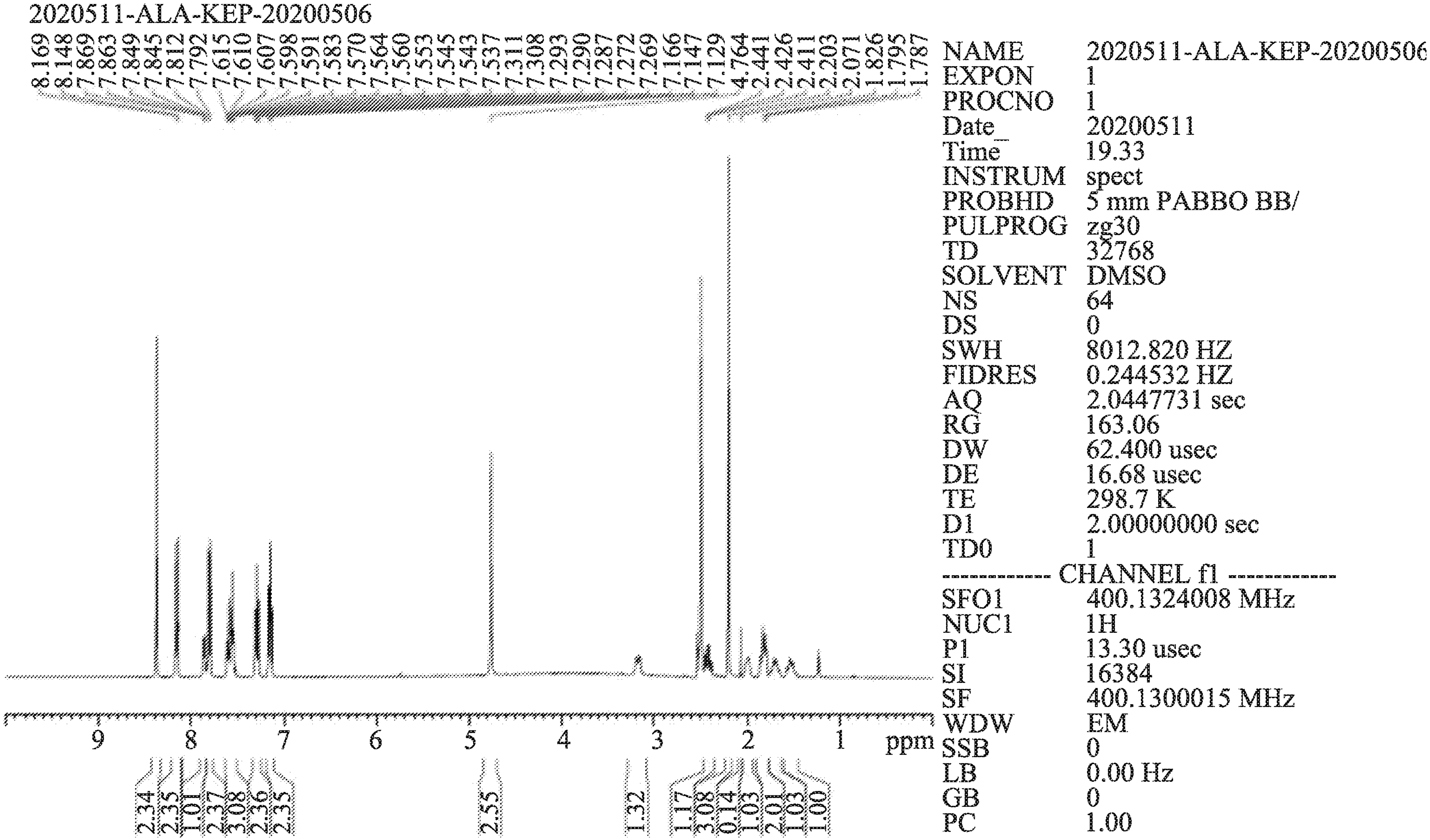
2020511-ALA-KEP-20200506
8.169
8.148
7.869
7.863
7.849
7.845
7.812
7.792
7.615
7.610
7.607
7.598
7.591
7.583
7.570
7.564
7.560
7.553
7.545
7.543
7.537
7.311
7.308
7.293
7.290
7.287
7.272
7.269
7.166
7.147
7.129
4.764
2.441
2.426
2.411
2.203
2.071
1.826
1.795
1.787
9
8
7
6
5
4
3
2
1
ppm
2.34
2.35
1.01
2.37
3.08
2.36
2.35
2.55
1.32
1.17
3.08
0.14
1.03
2.01
1.03
1.00
NAME 2020511-ALA-KEP-20200506
EXPON 1
PROCNO 1
Date_ 20200511
Time_ 19.33
INSTRUM spect
PROBHD 5 mm PABBO BB/
PULPROG zg30
TD 32768
SOLVENT DMSO
NS 64
DS 0
SWH 8012.820 HZ
FIDRES 0.244532 HZ
AQ 2.0447731 sec
RG 163.06
DW 62.400 usec
DE 16.68 usec
TE 298.7 K
D1 2.00000000 sec
TD0 1
------------- CHANNEL f1 -------------
SFO1 400.1324008 MHz
NUC1 1H
P1 13.30 usec
SI 16384
SF 400.1300015 MHz
WDW EM
SSB 0
LB 0.00 Hz
GB 0
PC 1.00

FIG. 11

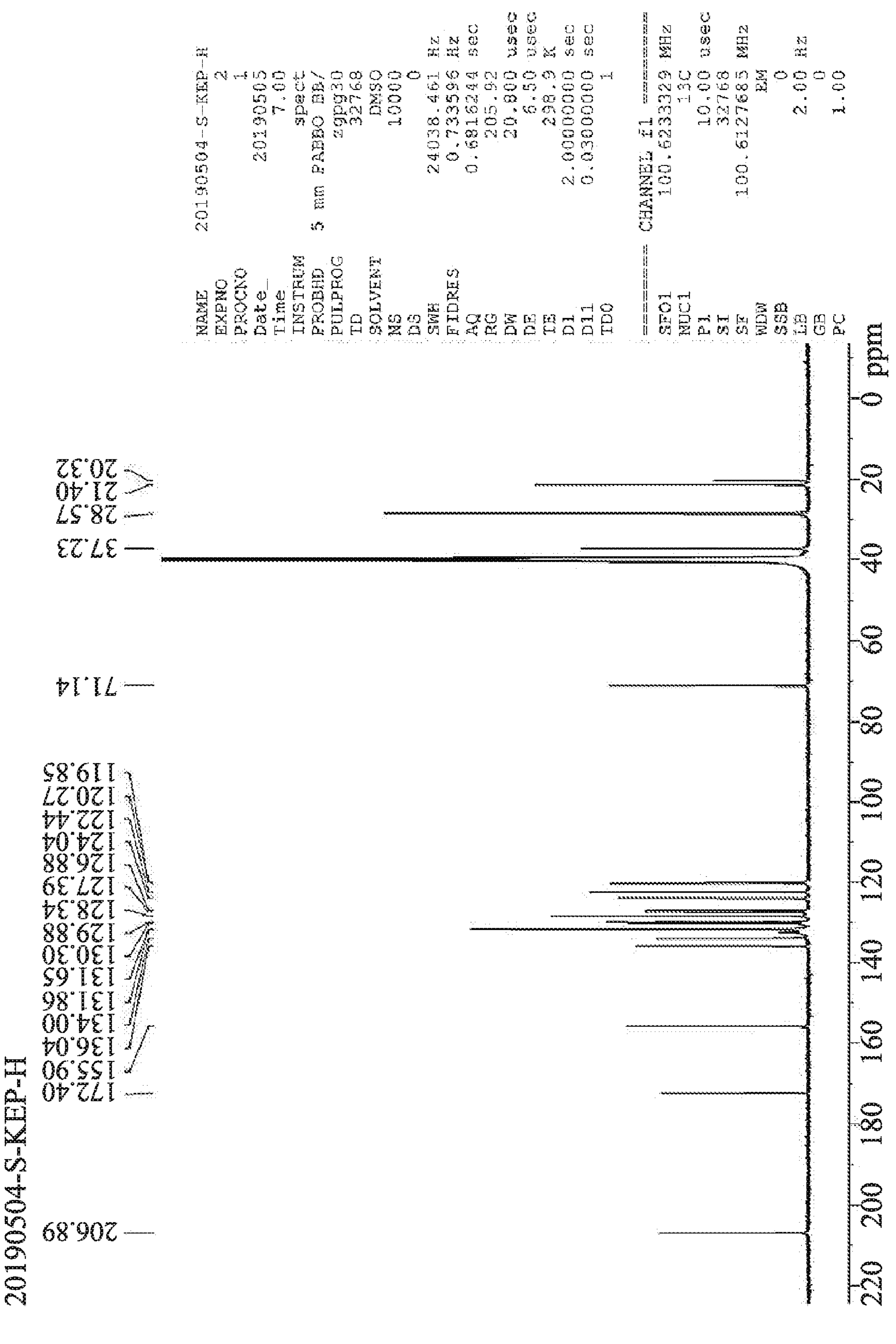
20190504-S-KEP-H
206.89
172.40
155.90
136.04
134.00
131.86
131.65
130.30
129.88
128.34
127.39
126.88
124.04
122.44
120.27
119.85
71.14
37.23
28.57
21.40
20.32
NAME 20190504-S-KEP-H
EXPNO 2
PROCNO 1
Date_ 20190505
Time 7.00
INSTRUM spect
PROBHD 5 mm PABBO BB/
PULPROG zgpg30
TD 32768
SOLVENT DMSO
NS 10000
DS 0
SWH 24038.461 Hz
FIDRES 0.733596 Hz
AQ 0.6816244 sec
RG 205.92
DW 20.800 usec
DE 6.50 usec
TE 298.9 K
D1 2.00000000 sec
D11 0.03000000 sec
TD0 1
======== CHANNEL f1 ========
SFO1 100.6233329 MHz
NUC1 13C
P1 10.00 usec
SI 32768
SF 100.6127685 MHz
WDW EM
SSB 0
LB 2.00 Hz
GB 0
PC 1.00
220
200
180
160
140
120
100
80
60
40
20
0 ppm

FIG. 12

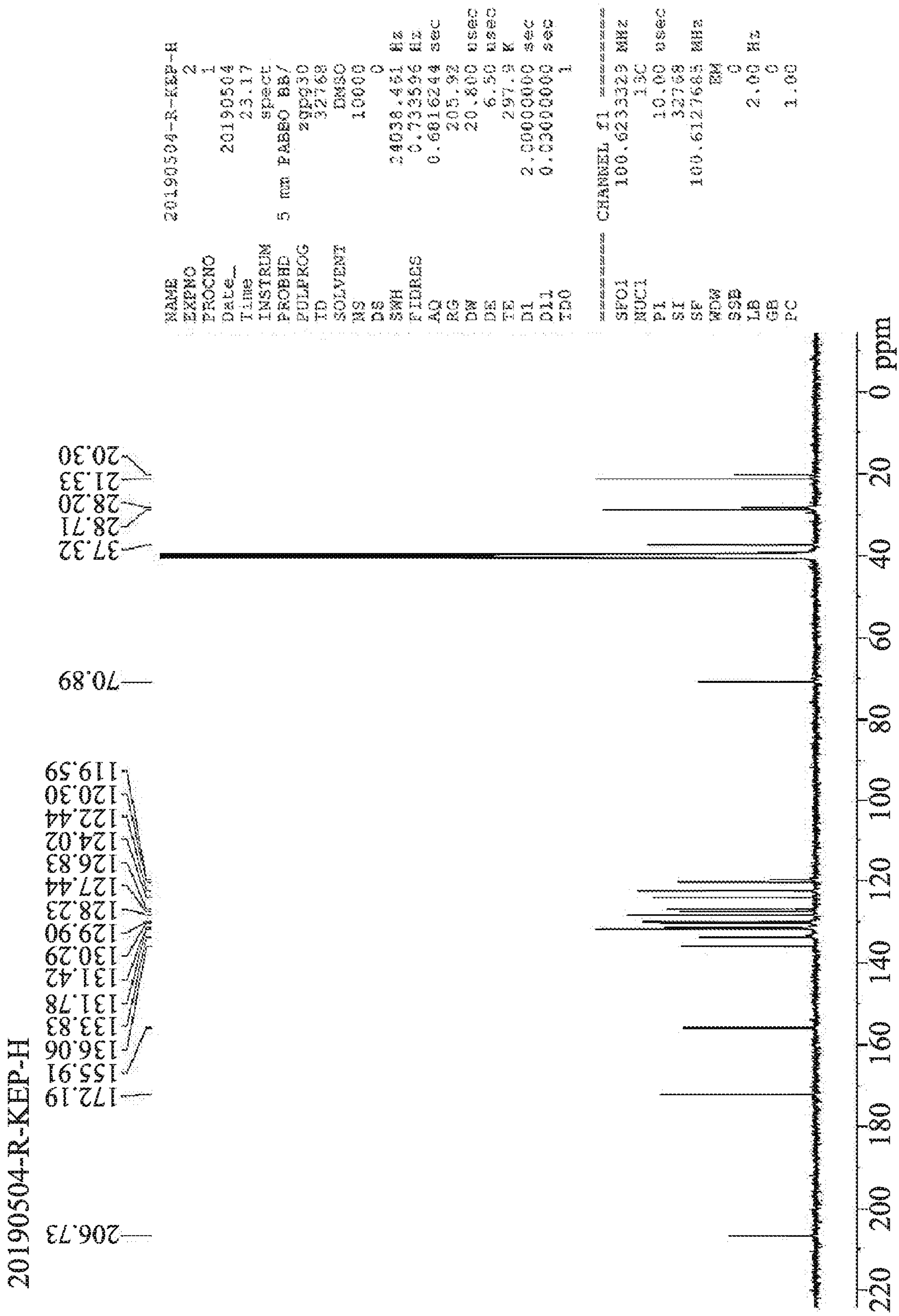
20190504-R-KEP-H
206.73
172.19
155.91
136.06
133.83
131.78
131.42
130.29
129.90
128.23
127.44
126.83
124.02
122.44
120.30
119.59
70.89
37.32
28.71
28.20
21.33
20.30
NAME 20190504-R-KEP-H
EXPNO 2
PROCNO 1
Date_ 20190504
Time 23.17
INSTRUM spect
PROBHD 5 mm PABBO BB/
PULPROG zgpg30
TD 32768
SOLVENT DMSO
NS 10000
DS 0
SWH 24038.461 Hz
FIDRES 0.733596 Hz
AQ 0.6816244 sec
RG 205.92
DW 20.800 usec
DE 6.50 usec
TE 297.9 K
D1 2.00000000 sec
D11 0.03000000 sec
TD0 1
======== CHANNEL f1 ========
SFO1 100.6233329 MHz
NUC1 13C
P1 10.00 usec
SI 32768
SF 100.6127685 MHz
WDW EM
SSB 0
LB 2.00 Hz
GB 0
PC 1.00
220 200 180 160 140 120 100 80 60 40 20 0 ppm

FIG. 13

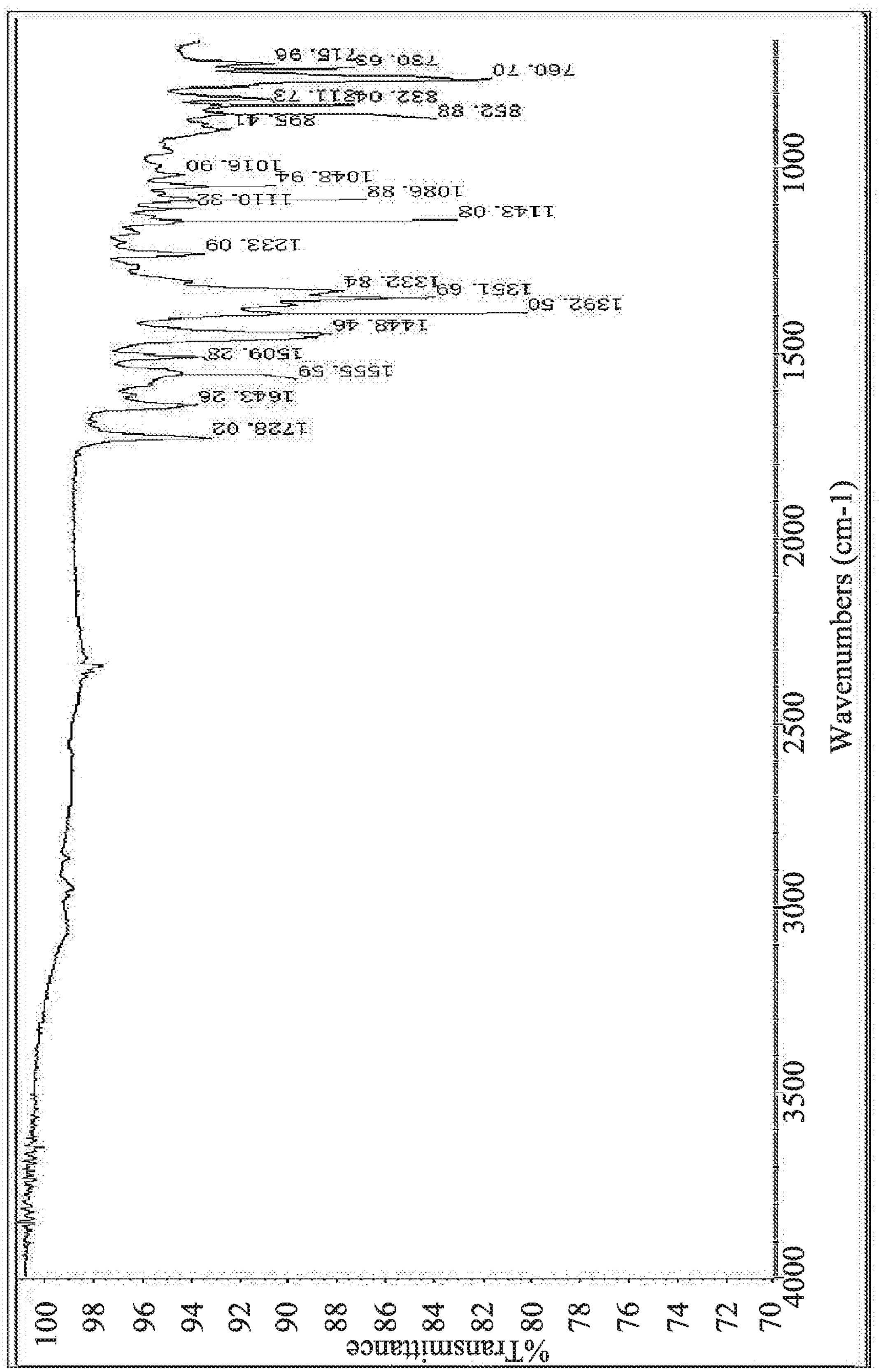
%Transmittance
Wavenumbers (cm-1)
100
98
96
94
92
90
88
86
84
82
80
78
76
74
72
70
4000
3500
3000
2500
2000
1500
1000
1728.02
1643.26
1555.59
1509.28
1448.46
1392.50
1351.69
1332.84
1233.09
1143.08
1110.32
1086.88
1048.94
1016.90
895.41
852.88
832.04
811.73
760.70
730.63
715.96

FIG. 14

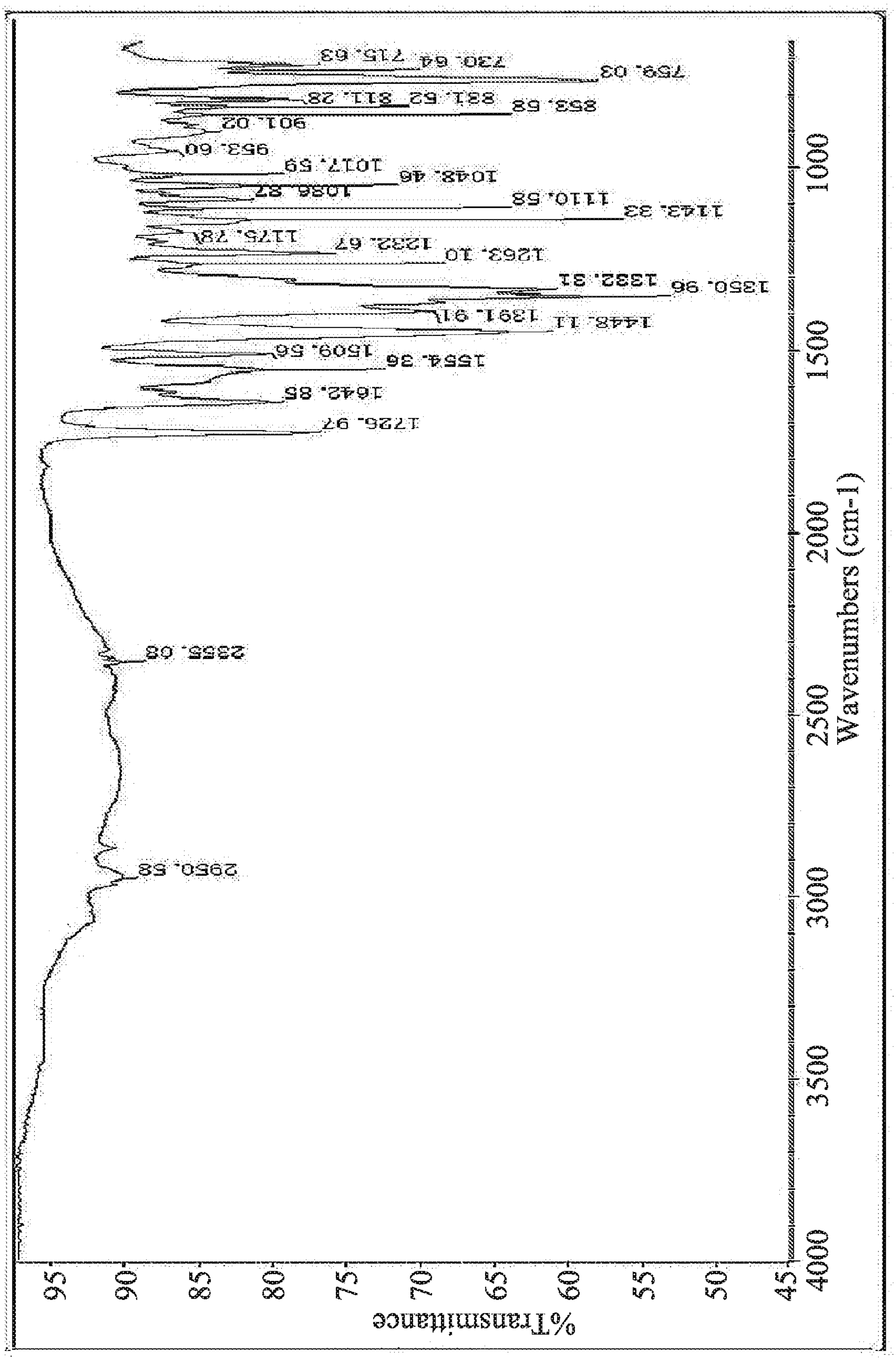
%Transmittance
Wavenumbers (cm-1)
95
90
85
80
75
70
65
60
55
50
45
4000
3500
3000
2500
2000
1500
1000
2950. 58
2355. 08
1726. 97
1642. 85
1554. 36
1509. 56
1448. 11
1391. 91
1350. 96
1332. 81
1263. 10
1232. 67
1175. 78
1143. 33
1110. 58
1086. 87
1048. 46
1017. 59
953. 60
901. 02
853. 68
831. 62
811. 28
759. 03
730. 64
715. 63

FIG. 15

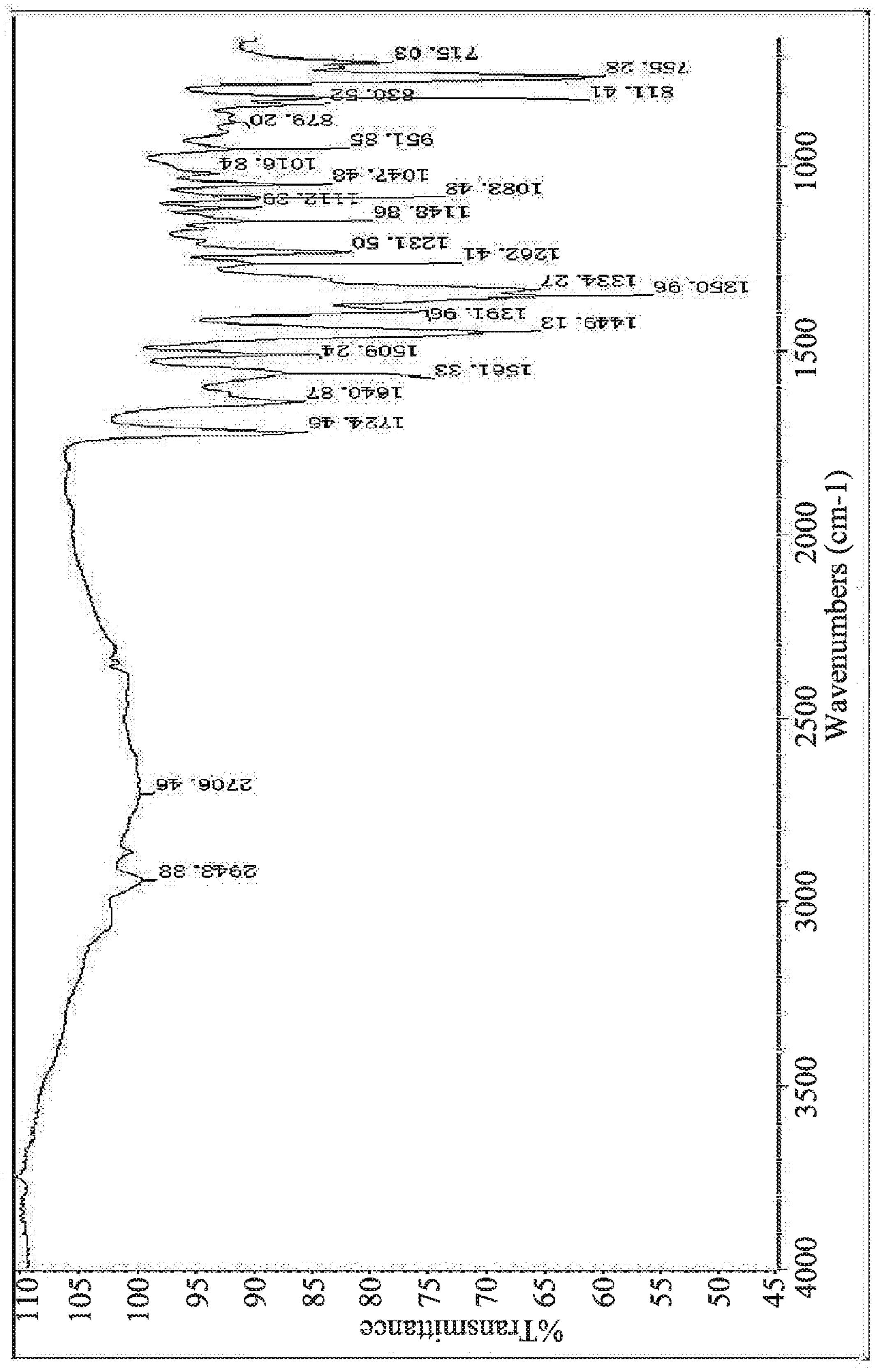
%Transmittance
110
105
100
95
90
85
80
75
70
65
60
55
50
45
4000
3500
3000
2500
2000
1500
1000
Wavenumbers (cm-1)
2943.38
2706.46
1724.46
1640.87
1561.33
1509.24
1449.13
1391.96
1350.96
1334.27
1262.41
1231.50
1148.86
1083.48
1047.48
1016.84
951.85
879.20
830.52
811.41
755.28
715.03

FIG. 16

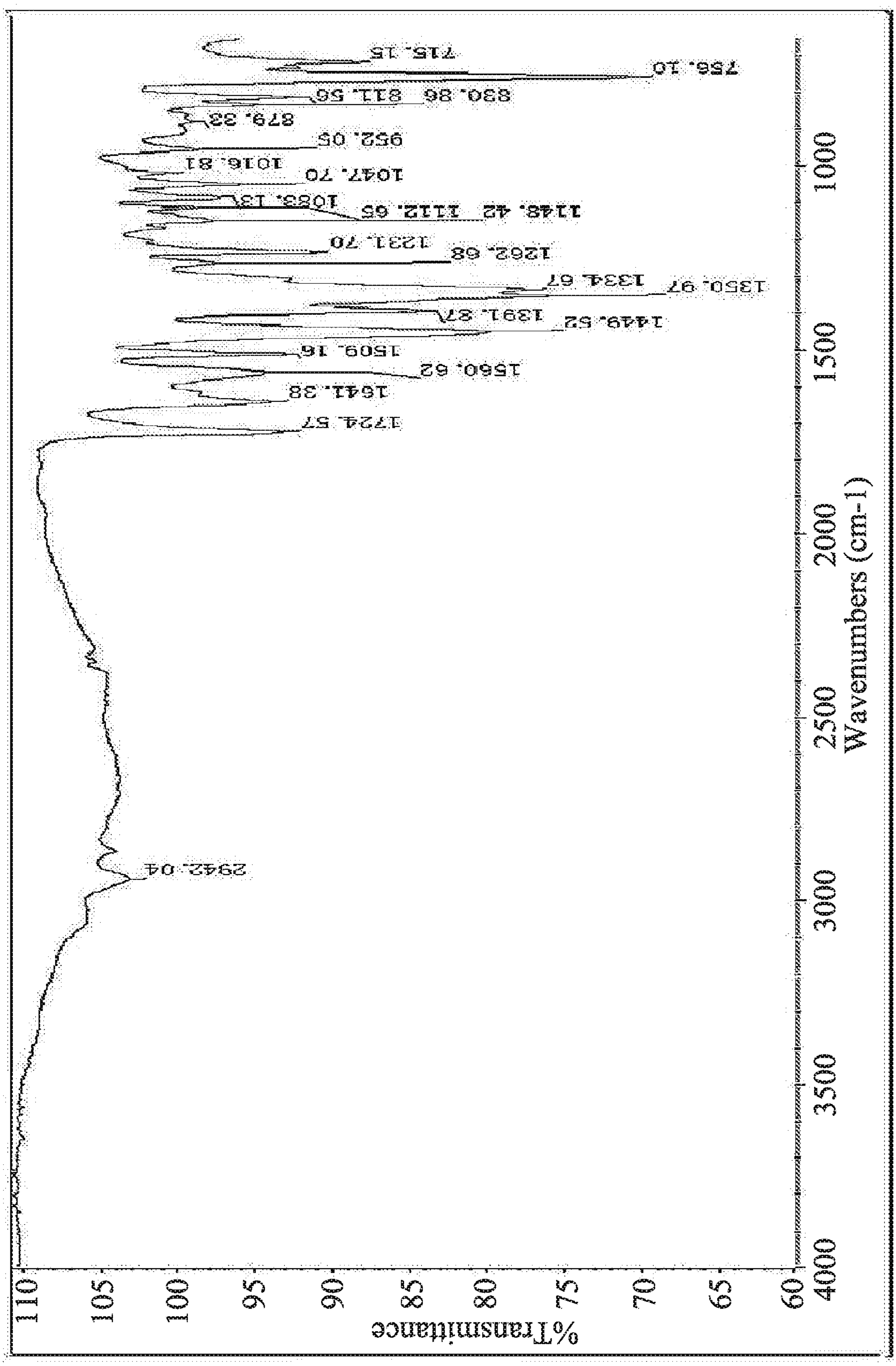
%Transmittance
110
105
100
95
90
85
80
75
70
65
60
4000
3500
3000
2500
2000
1500
1000
Wavenumbers (cm-1)
2942.04
1724.57
1641.38
1560.62
1509.16
1449.52
1391.87
1350.97
1334.67
1262.68
1231.70
1148.42
1112.65
1083.13
1047.70
1016.81
952.05
879.33
830.86
811.56
756.10
715.15

FIG. 17

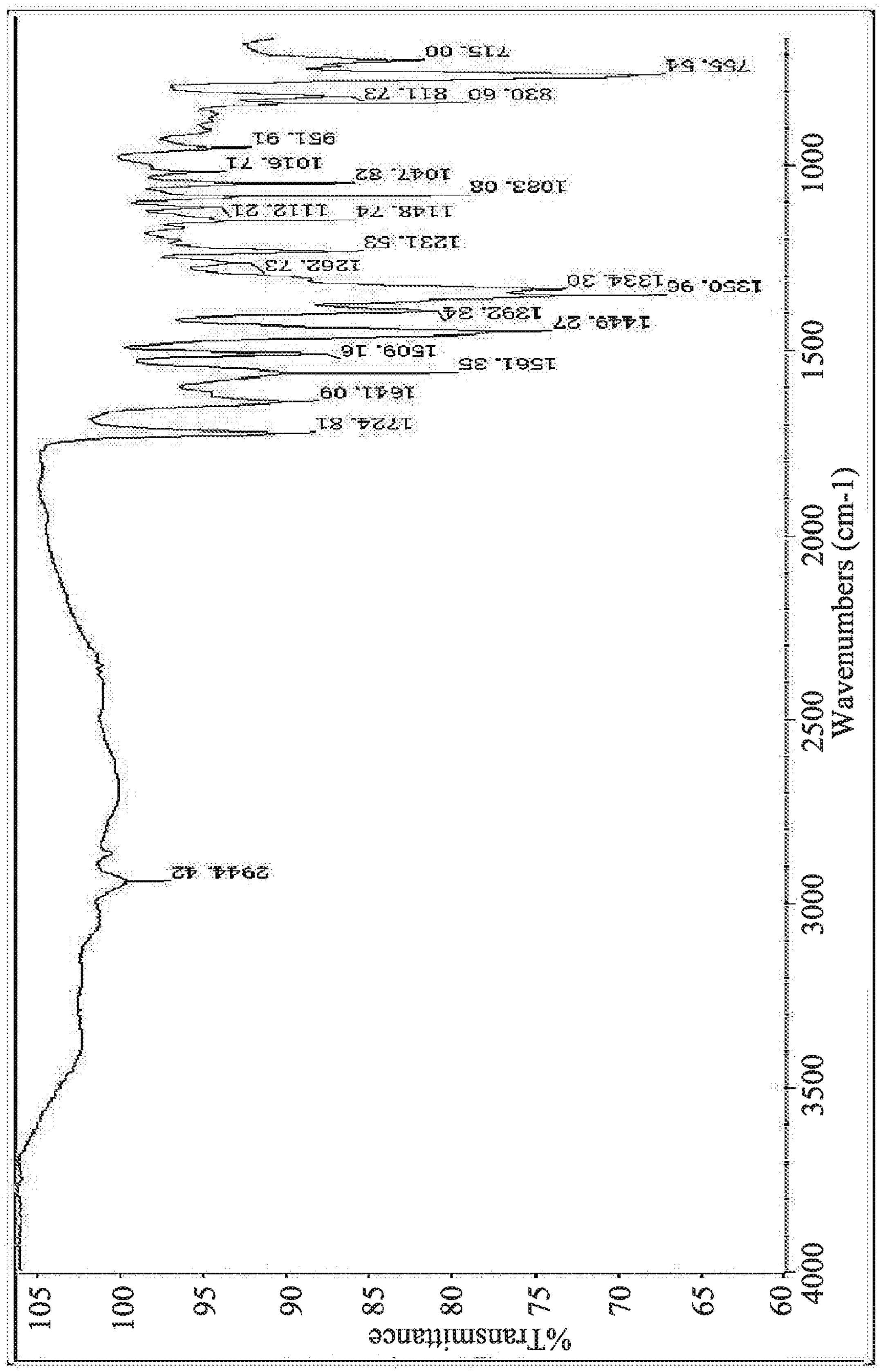
%Transmittance
105
100
95
90
85
80
75
70
65
60
Wavenumbers (cm-1)
4000
3500
3000
2500
2000
1500
1000
2944.42
1724.81
1641.09
1561.35
1509.16
1449.27
1392.34
1350.96
1334.30
1262.73
1231.58
1148.74
1112.21
1083.08
1047.82
1016.71
951.91
830.60
811.73
755.54
715.00

FIG. 18

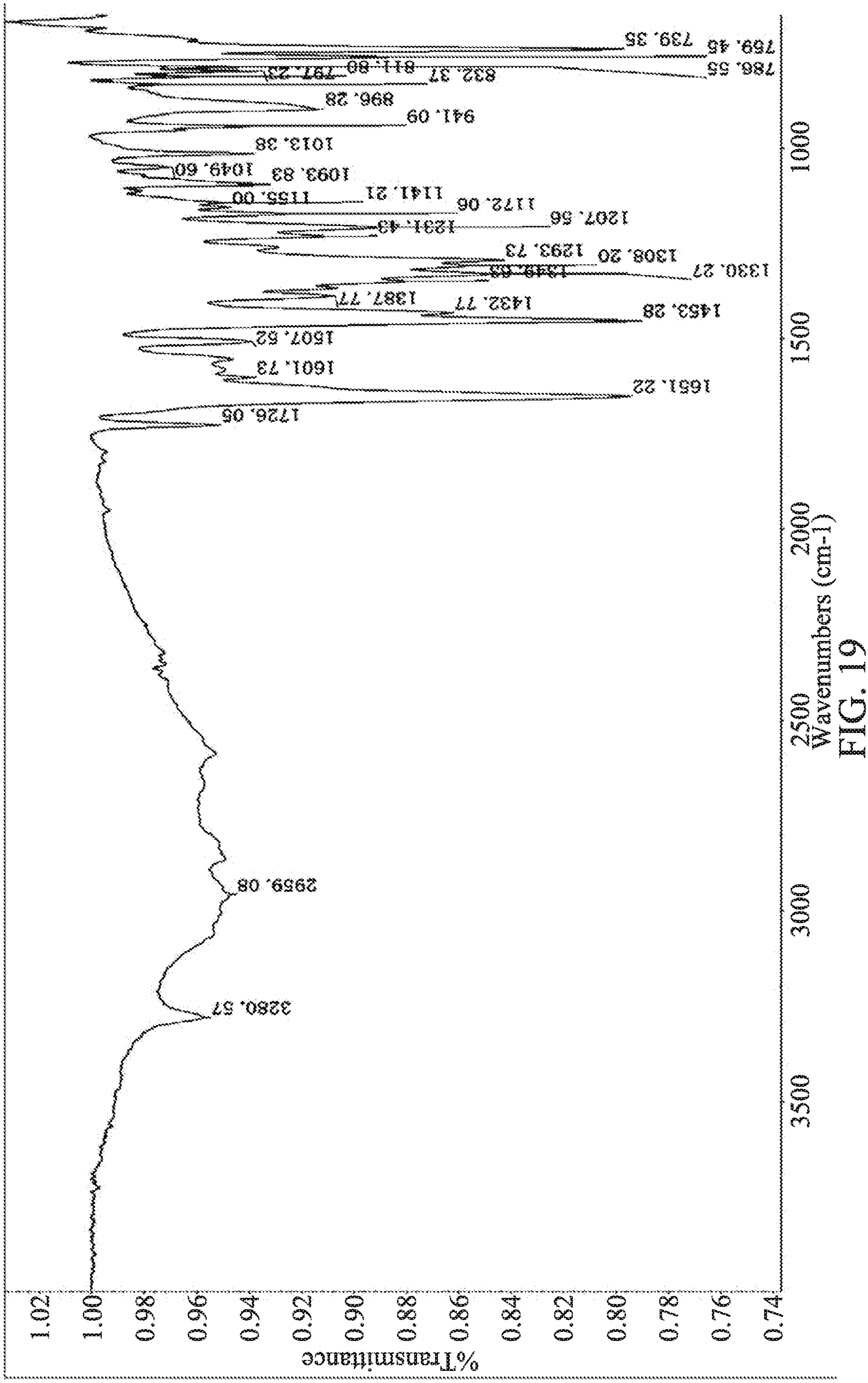
%Transmittance
1.02
1.00
0.98
0.96
0.94
0.92
0.90
0.88
0.86
0.84
0.82
0.80
0.78
0.76
0.74
3500
3000
2500
2000
1500
1000
Wavenumbers (cm-1)
3280. 57
2959. 08
1726. 05
1651. 22
1601. 73
1507. 52
1453. 28
1432. 77
1387. 77
1330. 27
1349. 63
1308. 20
1293. 73
1231. 43
1207. 56
1172. 06
1155. 00
1141. 21
1093. 83
1049. 60
1013. 38
941. 09
896. 28
832. 37
811. 80
797. 23
786. 55
759. 45
739. 35
FIG. 19

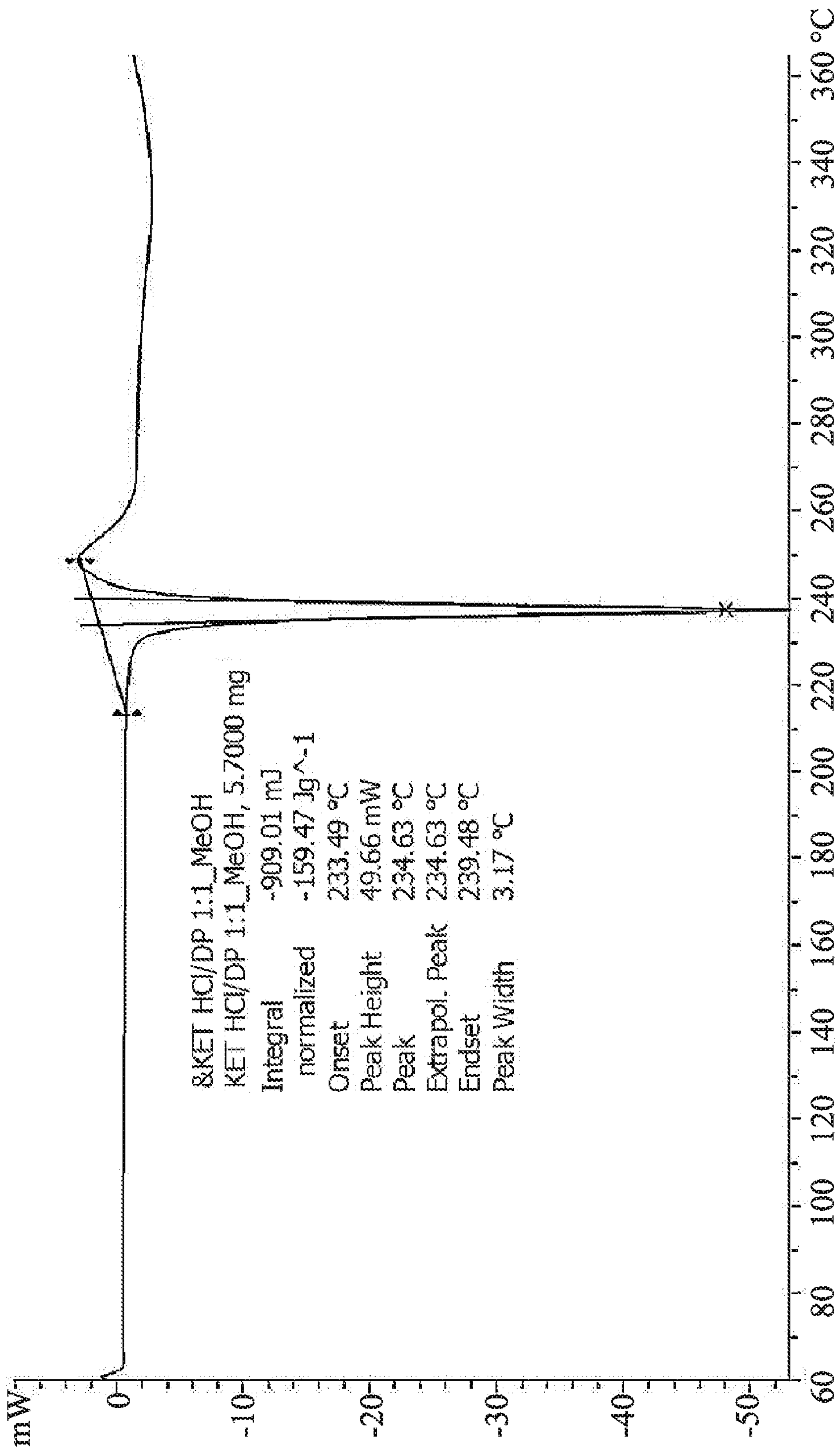
mW
0
-10
-20
-30
-40
-50
60
80
100
120
140
160
180
200
220
240
260
280
300
320
340
360 °C
&KET HCl/DP 1:1_MeOH
KET HCl/DP 1:1_MeOH, 5.7000 mg
Integral -909.01 mJ
normalized -159.47 Jg^-1
Onset 233.49 °C
Peak Height 49.66 mW
Peak 234.63 °C
Extrapol. Peak 234.63 °C
Endset 239.48 °C
Peak Width 3.17 °C

FIG. 20

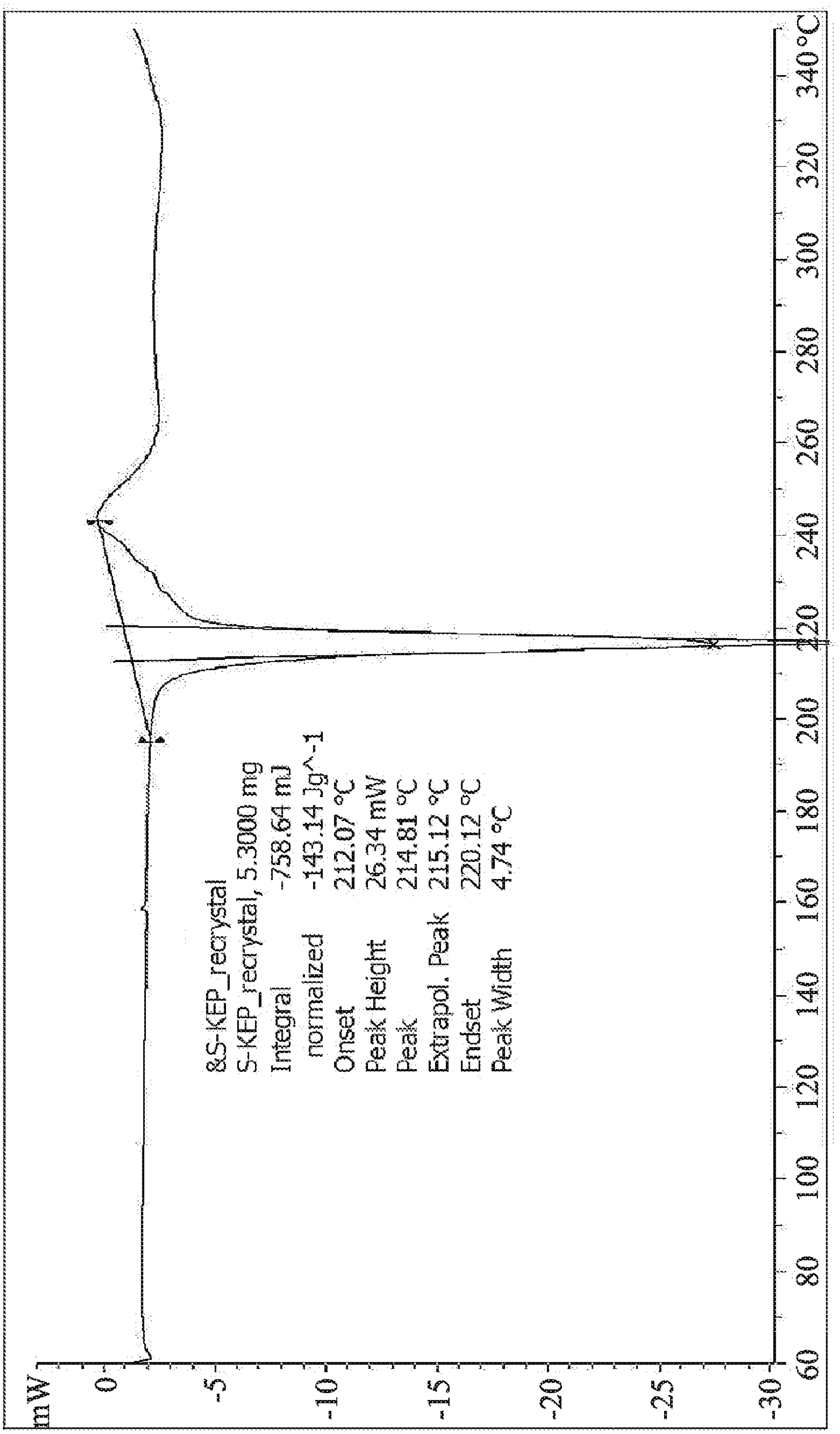
&S-KEP_recrystal
S-KEP_recrystal, 5.3000 mg
Integral -758.64 mJ
normalized -143.14 Jg^-1
Onset 212.07 °C
Peak Height 26.34 mW
Peak 214.81 °C
Extrapol. Peak 215.12 °C
Endset 220.12 °C
Peak Width 4.74 °C
mW
0
-5
-10
-15
-20
-25
-30
60
80
100
120
140
160
180
200
220
240
260
280
300
320
340 °C

FIG. 21

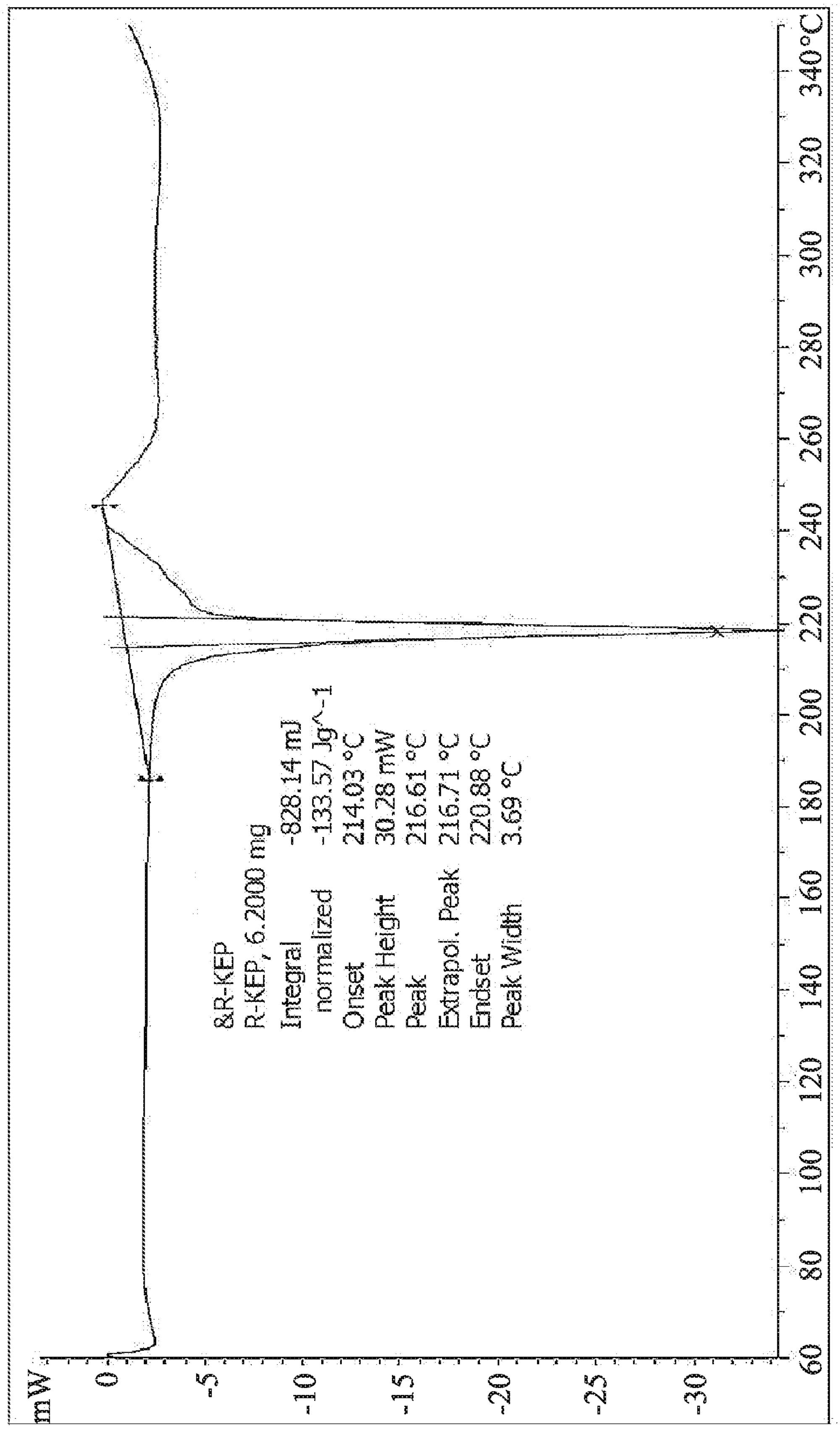
mW
0
-5
-10
-15
-20
-25
-30
60
80
100
120
140
160
180
200
220
240
260
280
300
320
340°C
&R-KEP
R-KEP, 6.2000 mg
Integral -828.14 mJ
normalized -133.57 Jg^-1
Onset 214.03 °C
Peak Height 30.28 mW
Peak 216.61 °C
Extrapol. Peak 216.71 °C
Endset 220.88 °C
Peak Width 3.69 °C

FIG. 22

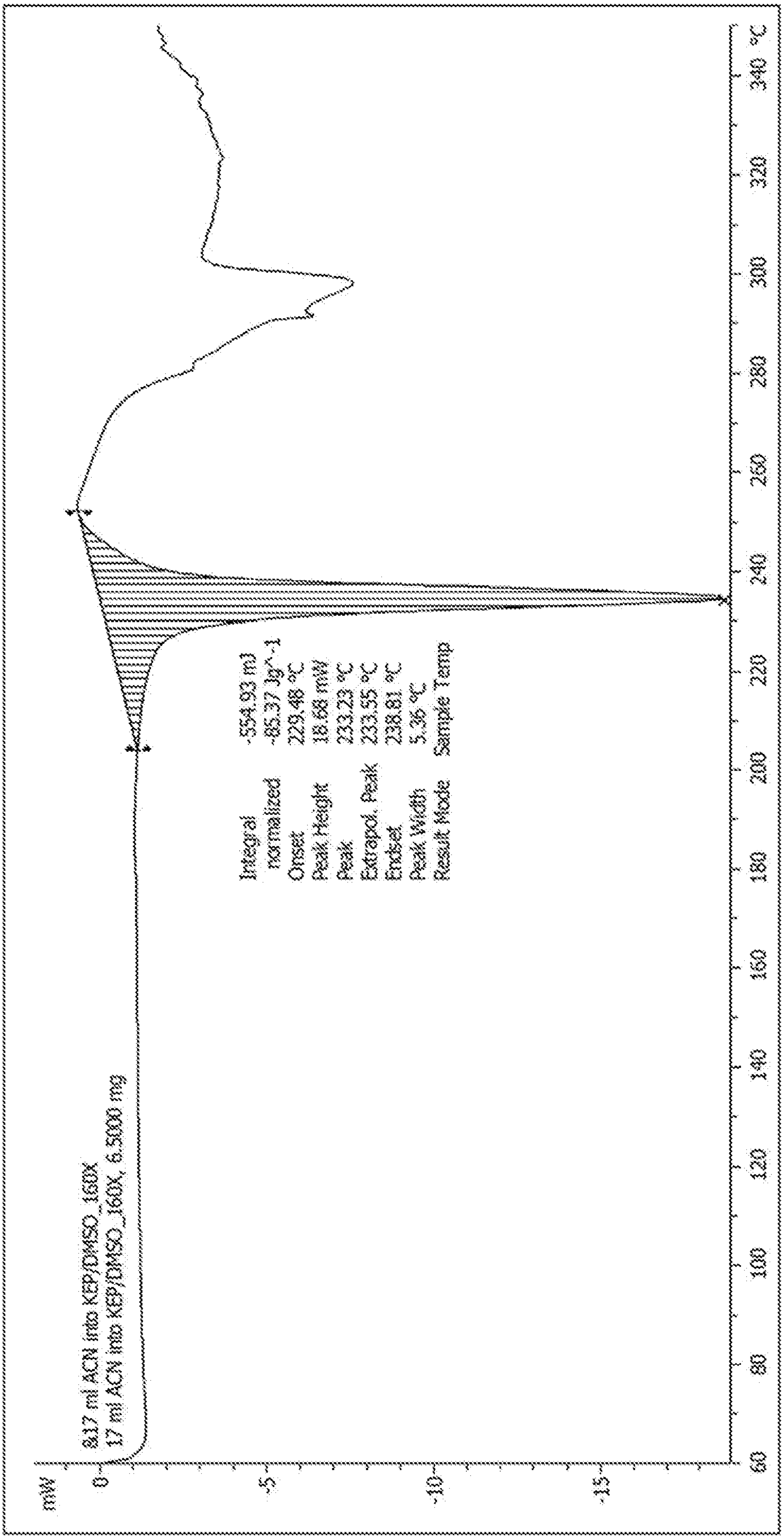
&17 ml ACN into KEP/DMSO_160X
17 ml ACN into KEP/DMSO_160X, 6.5000 mg
Integral -554.93 mJ
normalized -85.37 Jg^-1
Onset 229.48 °C
Peak Height 18.68 mW
Peak 233.23 °C
Extrapol. Peak 233.55 °C
Endset 238.81 °C
Peak Width 5.36 °C
Result Mode Sample Temp
mW
0
-5
-10
-15
60
80
100
120
140
160
180
200
220
240
260
280
300
320
340 °C

FIG. 23

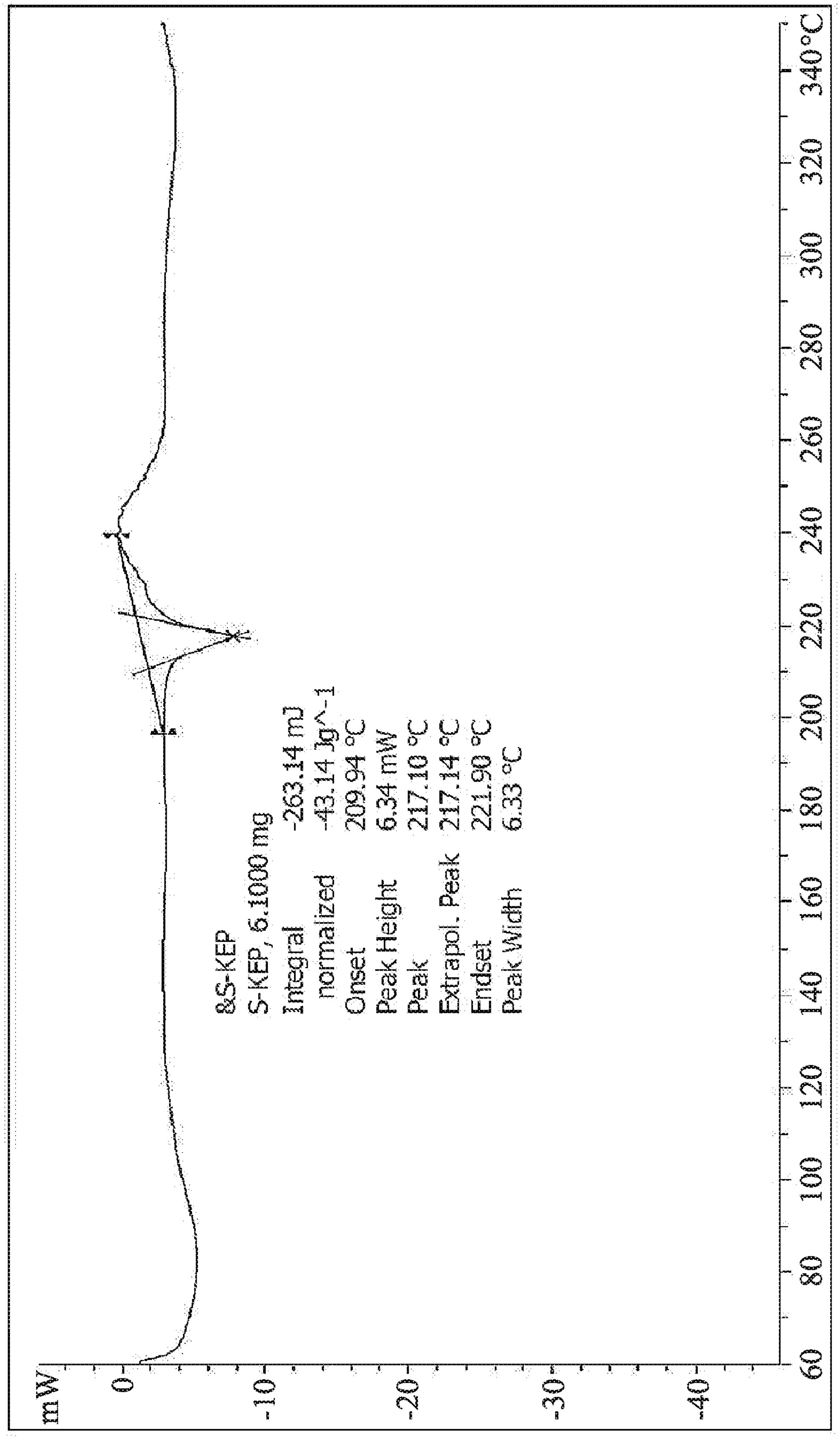
mW
0
-10
-20
-30
-40
60
80
100
120
140
160
180
200
220
240
260
280
300
320
340°C
&S-KEP
S-KEP, 6.1000 mg
Integral -263.14 mJ
normalized -43.14 Jg^-1
Onset 209.94 °C
Peak Height 6.34 mW
Peak 217.10 °C
Extrapol. Peak 217.14 °C
Endset 221.90 °C
Peak Width 6.33 °C

FIG. 24

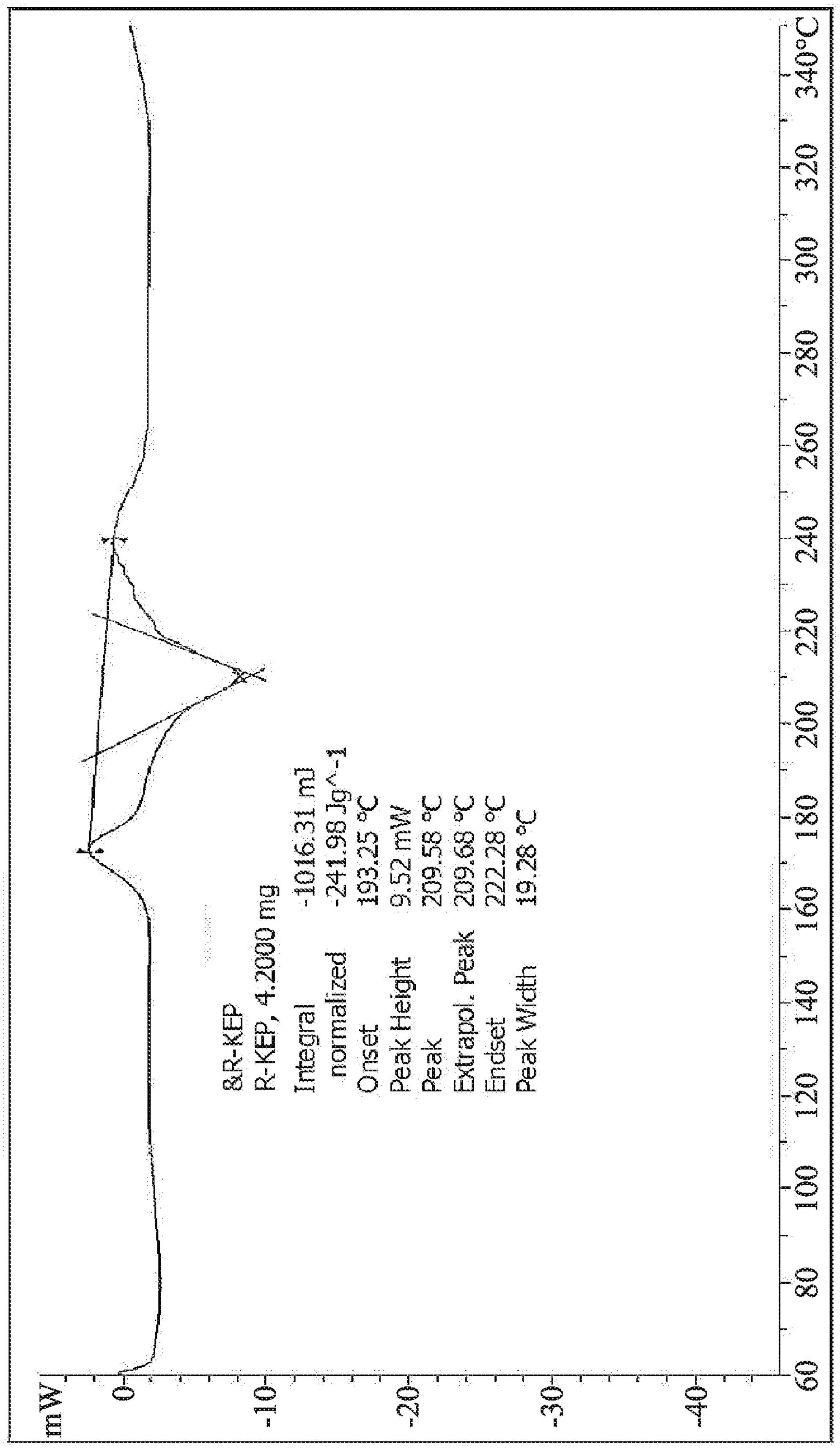
mW
0
-10
-20
-30
-40
60
80
100
120
140
160
180
200
220
240
260
280
300
320
340°C
8R-KEP
R-KEP, 4.2000 mg
Integral -1016.31 mJ
normalized -241.98 Jg^-1
Onset 193.25 °C
Peak Height 9.52 mW
Peak 209.58 °C
Extrapol. Peak 209.68 °C
Endset 222.28 °C
Peak Width 19.28 °C

FIG. 25

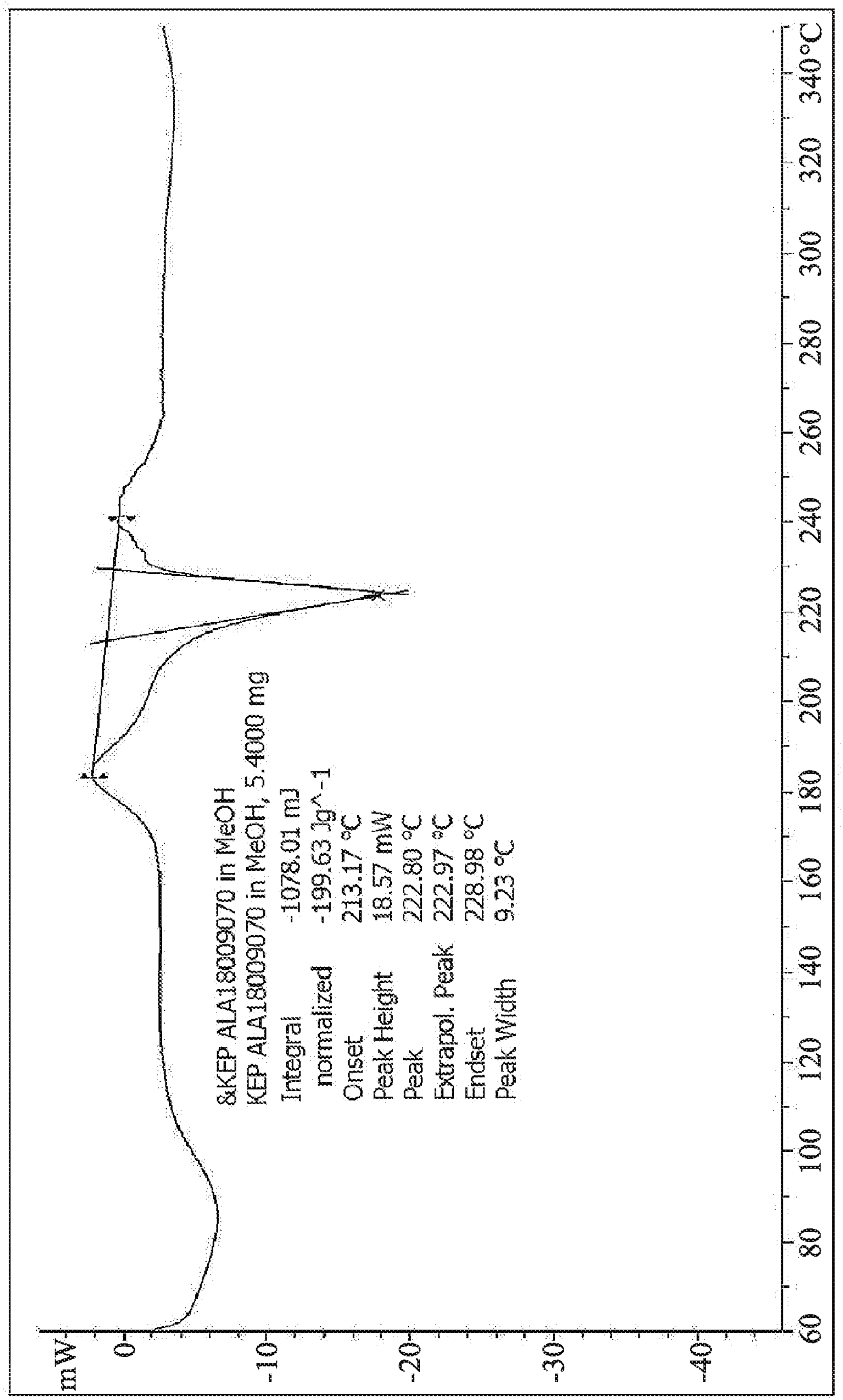
&KEP ALA18009070 in MeOH
KEP ALA18009070 in MeOH, 5.4000 mg
Integral -1078.01 mJ
normalized -199.63 Jg^-1
Onset 213.17 °C
Peak Height 18.57 mW
Peak 222.80 °C
Extrapol. Peak 222.97 °C
Endset 228.98 °C
Peak Width 9.23 °C
mW
0
-10
-20
-30
-40
60
80
100
120
140
160
180
200
220
240
260
280
300
320
340°C

FIG. 26

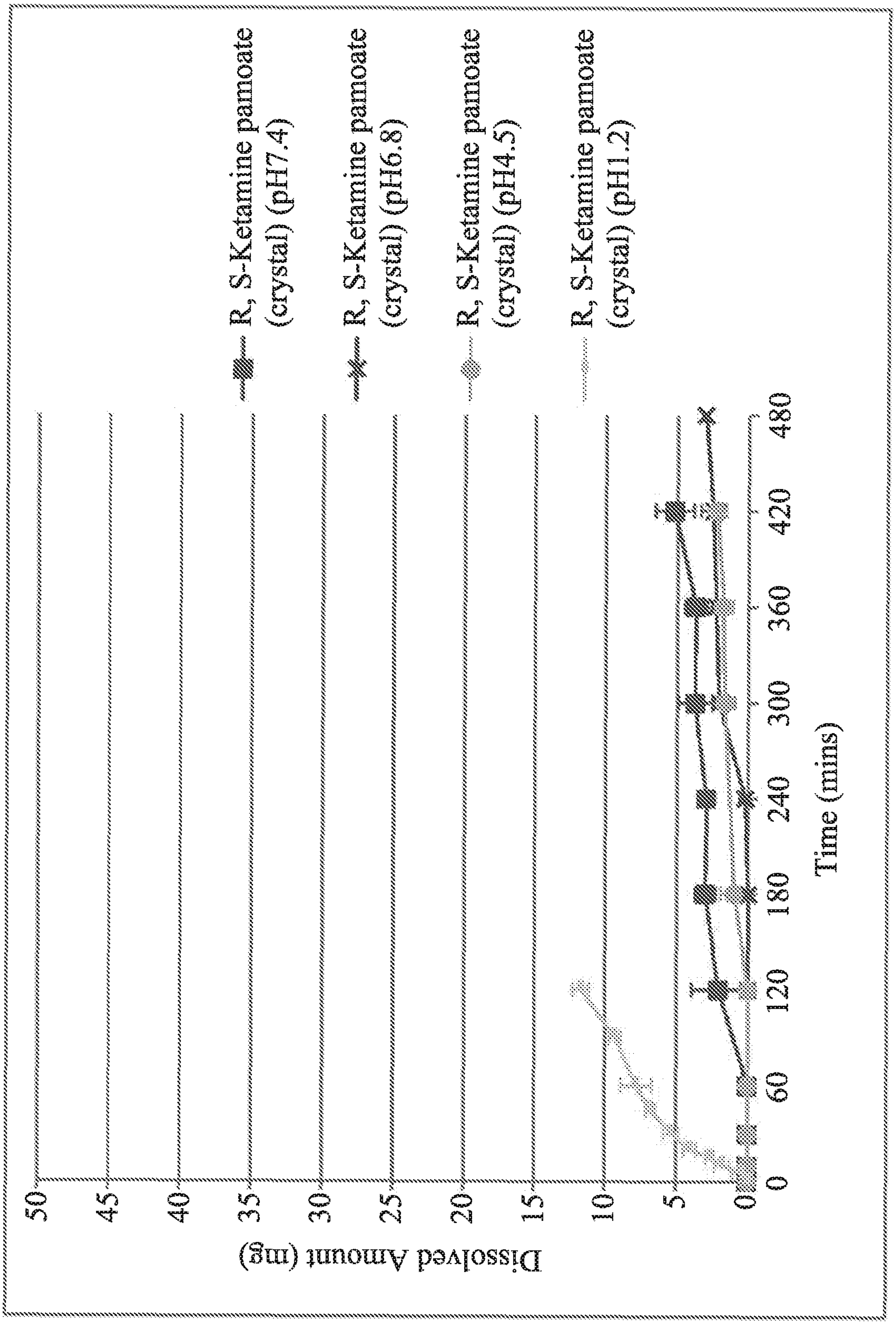

Dissolved Amount (mg)
0
5
10
15
20
25
30
35
40
45
50
Time (mins)
0
60
120
180
240
300
360
420
480
R, S-Ketamine pamoate (crystal) (pH7.4)
R, S-Ketamine pamoate (crystal) (pH6.8)
R, S-Ketamine pamoate (crystal) (pH4.5)
R, S-Ketamine pamoate (crystal) (pH1.2)


FIG. 27

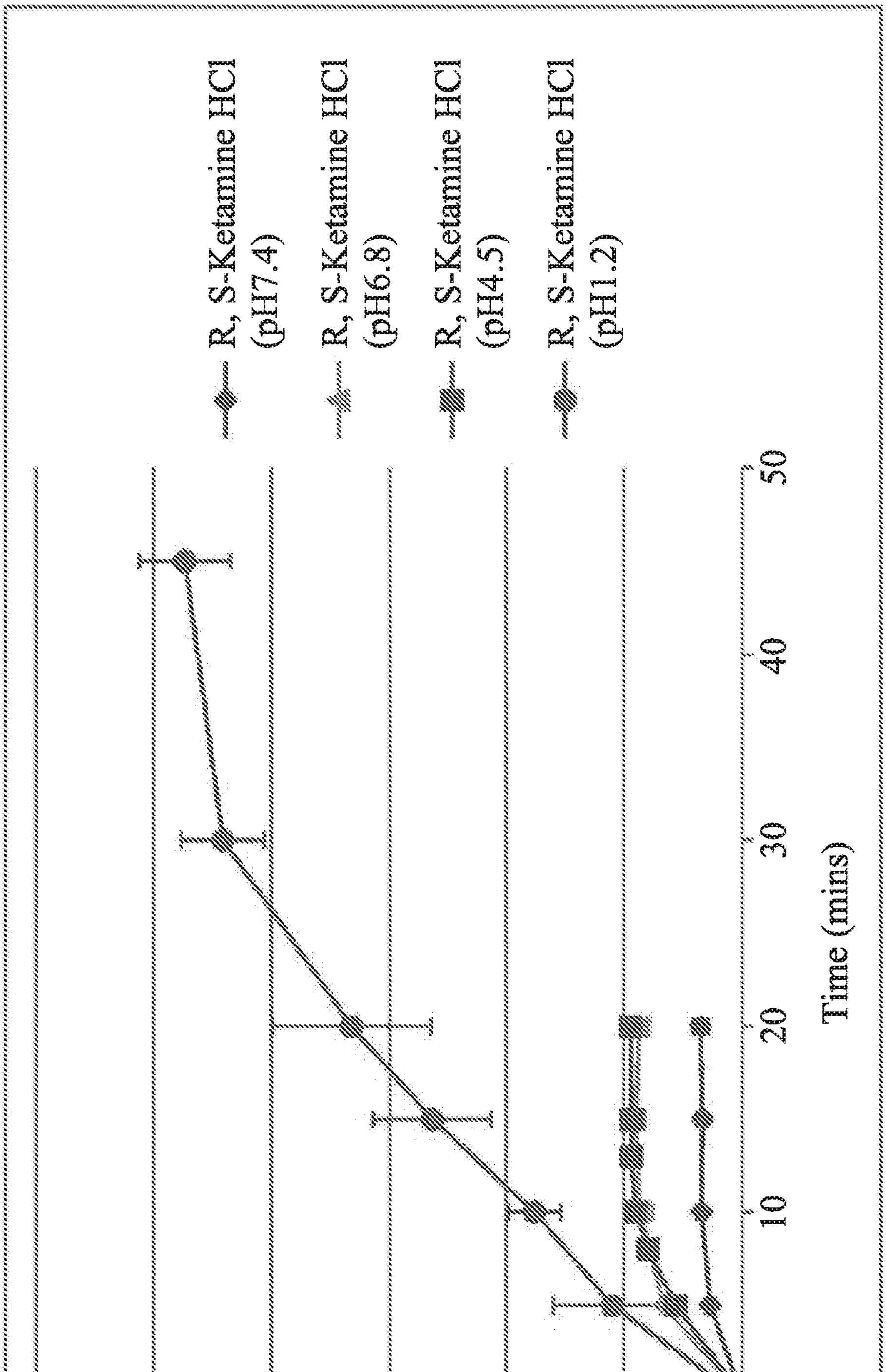
Dissolved Amount (mg)
300
250
200
150
100
50
0
0
10
20
30
40
50
Time (mins)
R, S-Ketamine HCl (pH7.4)
R, S-Ketamine HCl (pH6.8)
R, S-Ketamine HCl (pH4.5)
R, S-Ketamine HCl (pH1.2)

FIG. 28

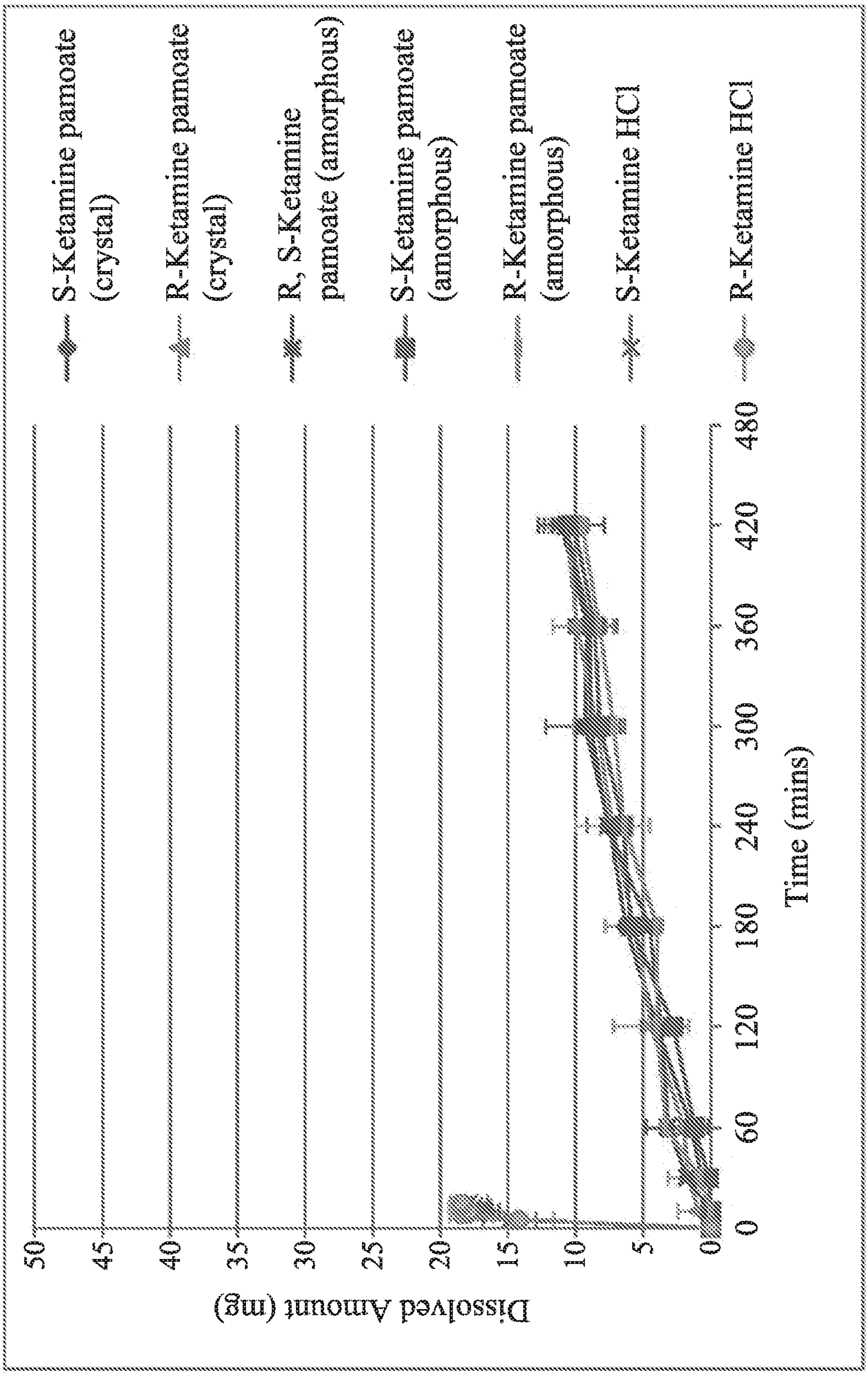
Dissolved Amount (mg)
0
5
10
15
20
25
30
35
40
45
50
0
60
120
180
240
300
360
420
480
Time (mins)
S-Ketamine pamoate (crystal)
R-Ketamine pamoate (crystal)
R, S-Ketamine pamoate (amorphous)
S-Ketamine pamoate (amorphous)
R-Ketamine pamoate (amorphous)
S-Ketamine HCl
R-Ketamine HCl

FIG. 29

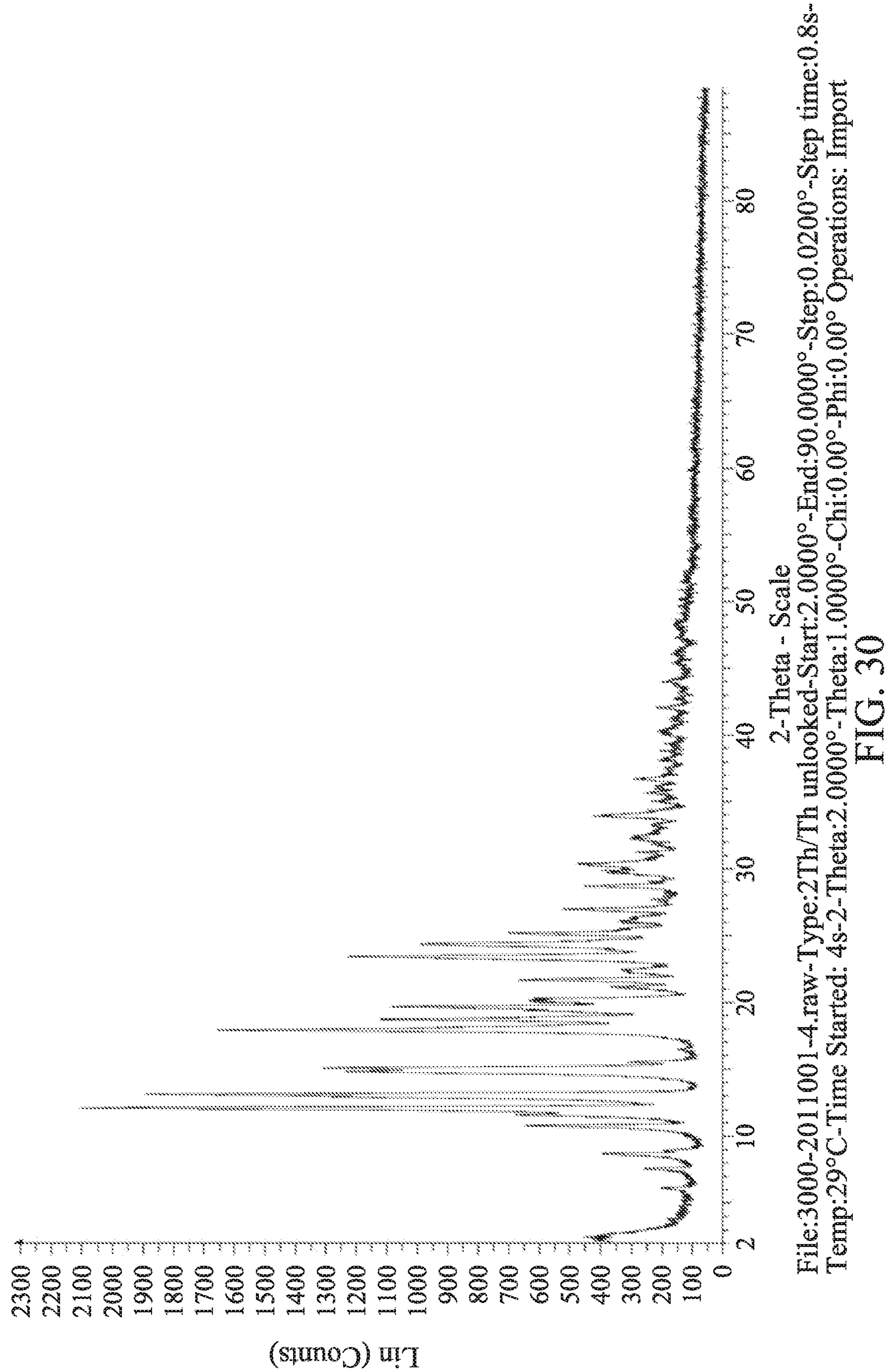
Lin (Counts)
2300
2200
2100
2000
1900
1800
1700
1600
1500
1400
1300
1200
1100
1000
900
800
700
600
500
400
300
200
100
0
2
10
20
30
40
50
60
70
80
2-Theta - Scale
File:3000-2011001-4.raw-Type:2Th/Th unlooked-Start:2.0000°-End:90.0000°-Step:0.0200°-Step time:0.8s-
Temp:29°C-Time Started: 4s-2-Theta:2.0000°-Theta:1.0000°-Chi:0.00°-Phi:0.00° Operations: Import

FIG. 30

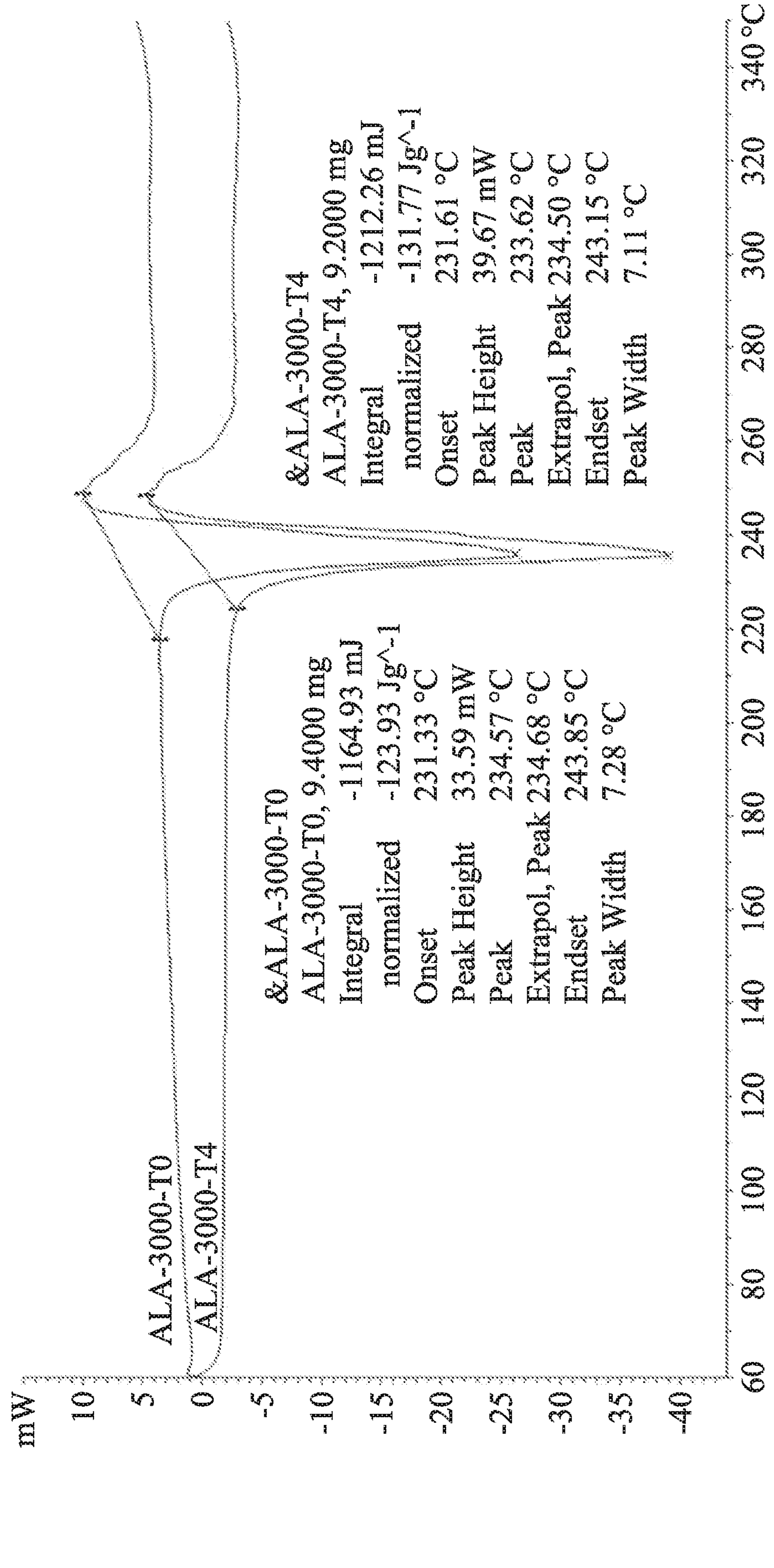
mW
10
5
0
-5
-10
-15
-20
-25
-30
-35
-40
60
80
100
120
140
160
180
200
220
240
260
280
300
320
340
°C
ALA-3000-T0
ALA-3000-T4
&ALA-3000-T0
ALA-3000-T0, 9.4000 mg
Integral -1164.93 mJ
normalized -123.93 Jg^-1
Onset 231.33 °C
Peak Height 33.59 mW
Peak 234.57 °C
Extrapol, Peak 234.68 °C
Endset 243.85 °C
Peak Width 7.28 °C
&ALA-3000-T4
ALA-3000-T4, 9.2000 mg
Integral -1212.26 mJ
normalized -131.77 Jg^-1
Onset 231.61 °C
Peak Height 39.67 mW
Peak 233.62 °C
Extrapol, Peak 234.50 °C
Endset 243.15 °C
Peak Width 7.11 °C

FIG. 31

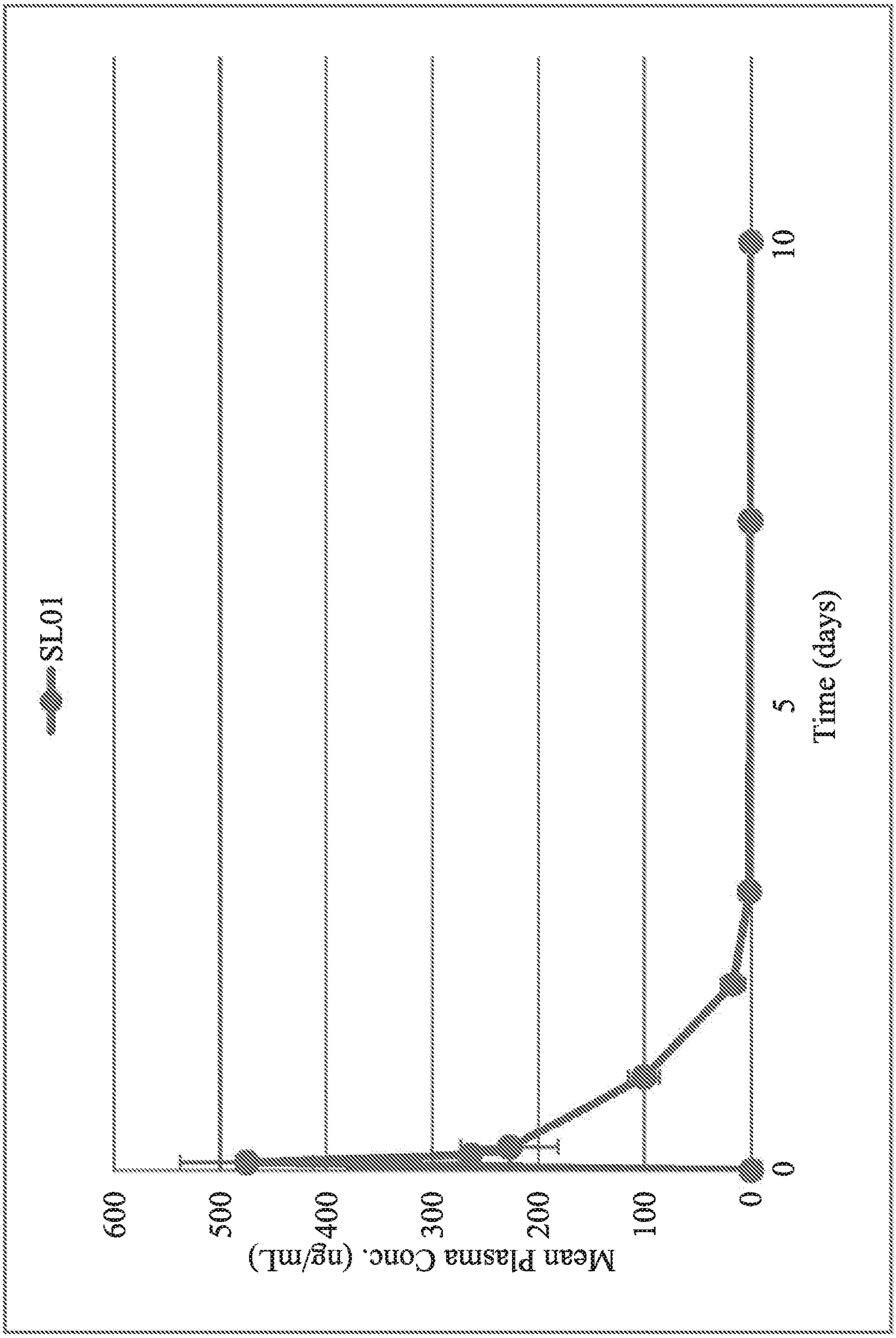
SL01
Mean Plasma Conc. (ng/mL)
600
500
400
300
200
100
0
0
5
10
Time (days)

FIG. 32

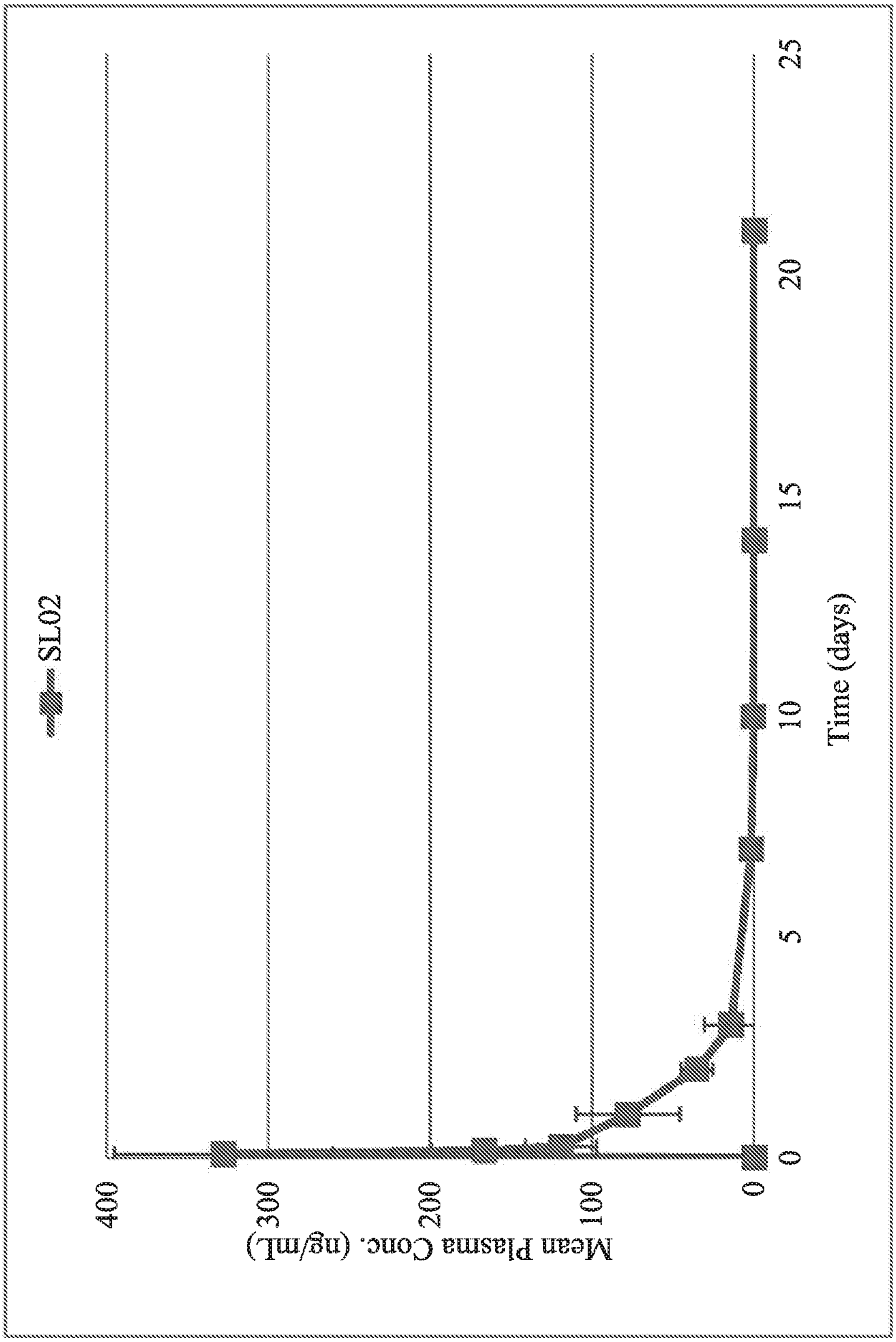
SL02
Mean Plasma Conc. (ng/mL)
400
300
200
100
0
Time (days)
0
5
10
15
20
25

FIG. 33

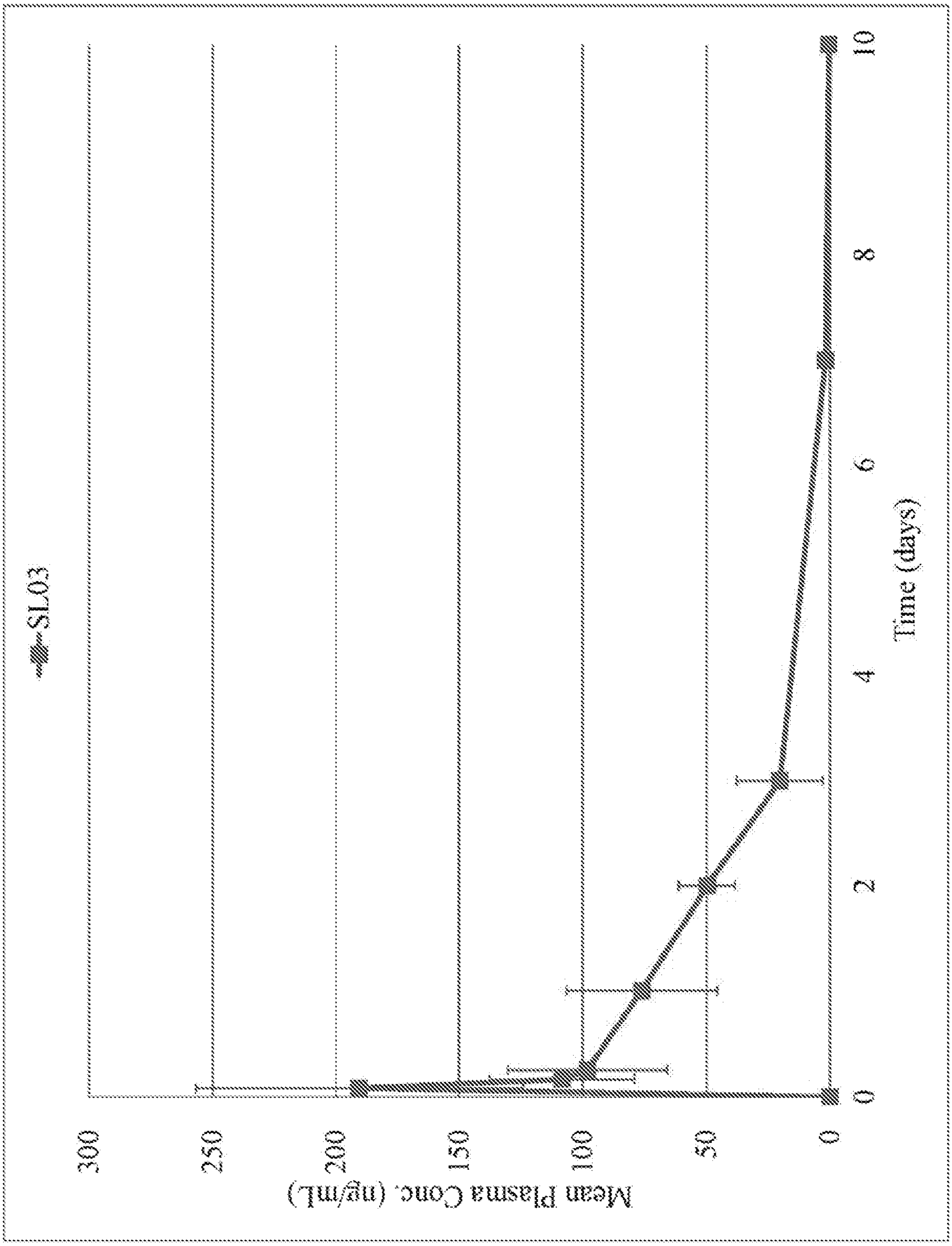
SL03
Mean Plasma Conc. (ng/mL)
300
250
200
150
100
50
0
0
2
4
6
8
10
Time (days)

FIG. 34

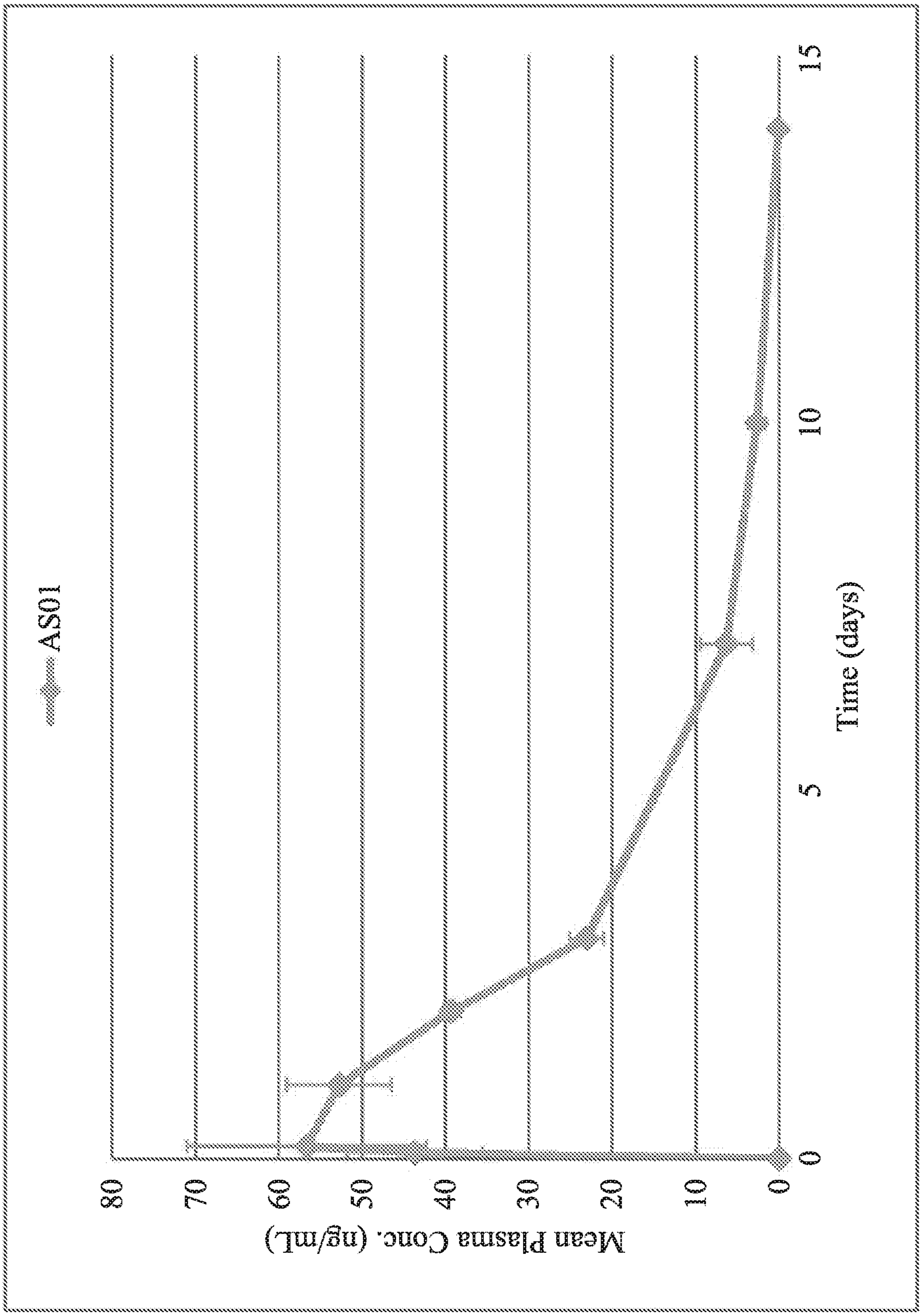
AS01
Mean Plasma Conc. (ng/mL)
80
70
60
50
40
30
20
10
0
0
5
10
15
Time (days)

FIG. 35

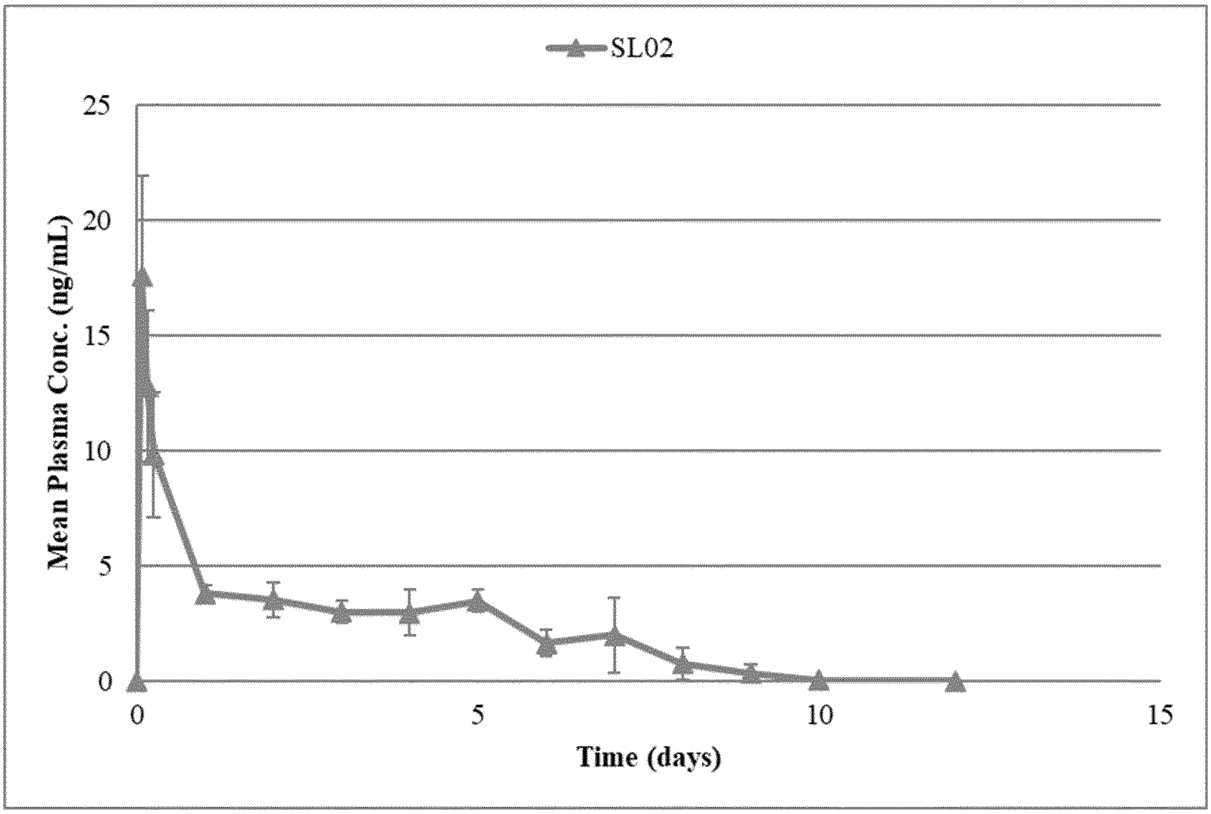


FIG. 36

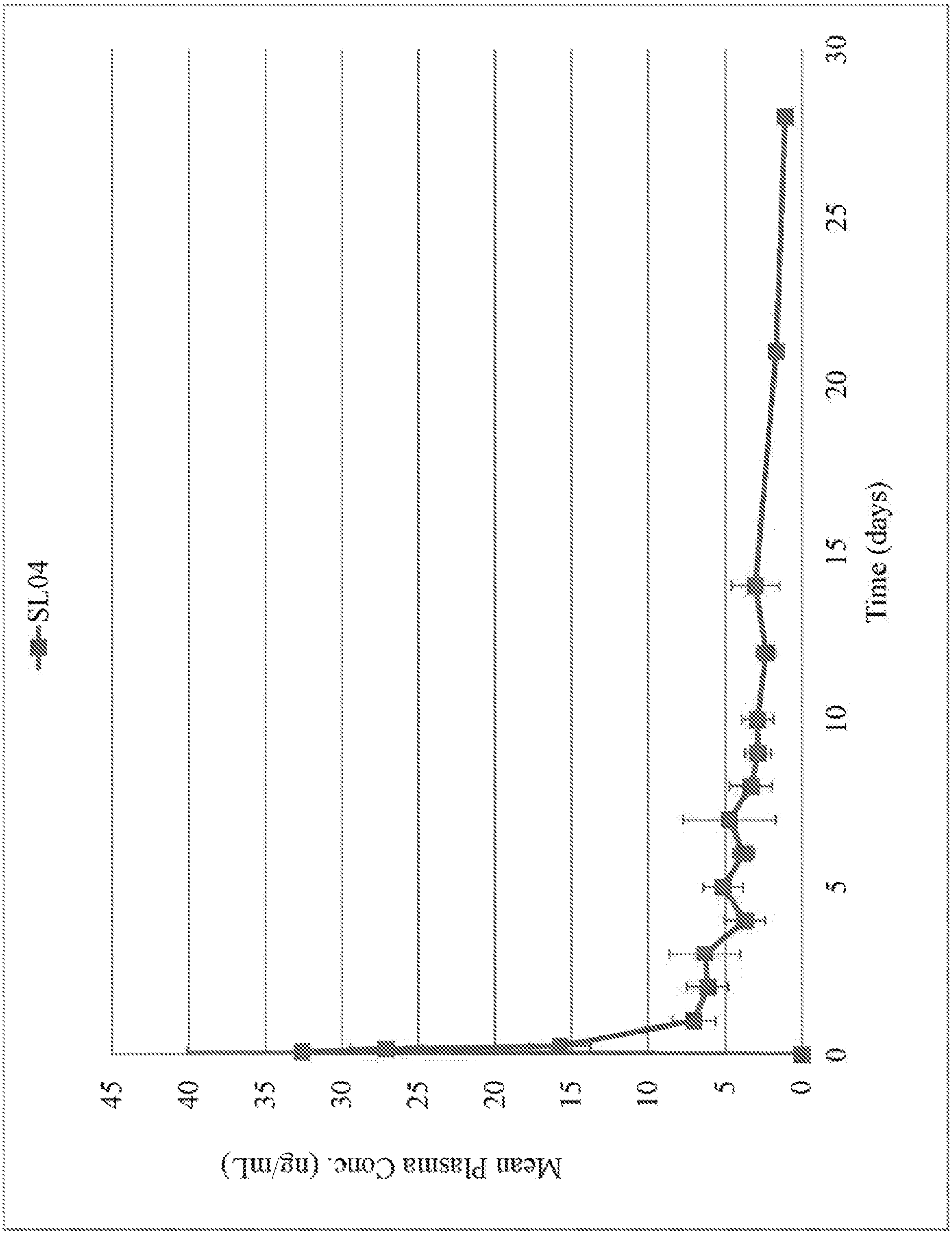
SL04
Mean Plasma Conc. (ng/mL)
45
40
35
30
25
20
15
10
5
0
0
5
10
15
20
25
30
Time (days)

FIG. 37

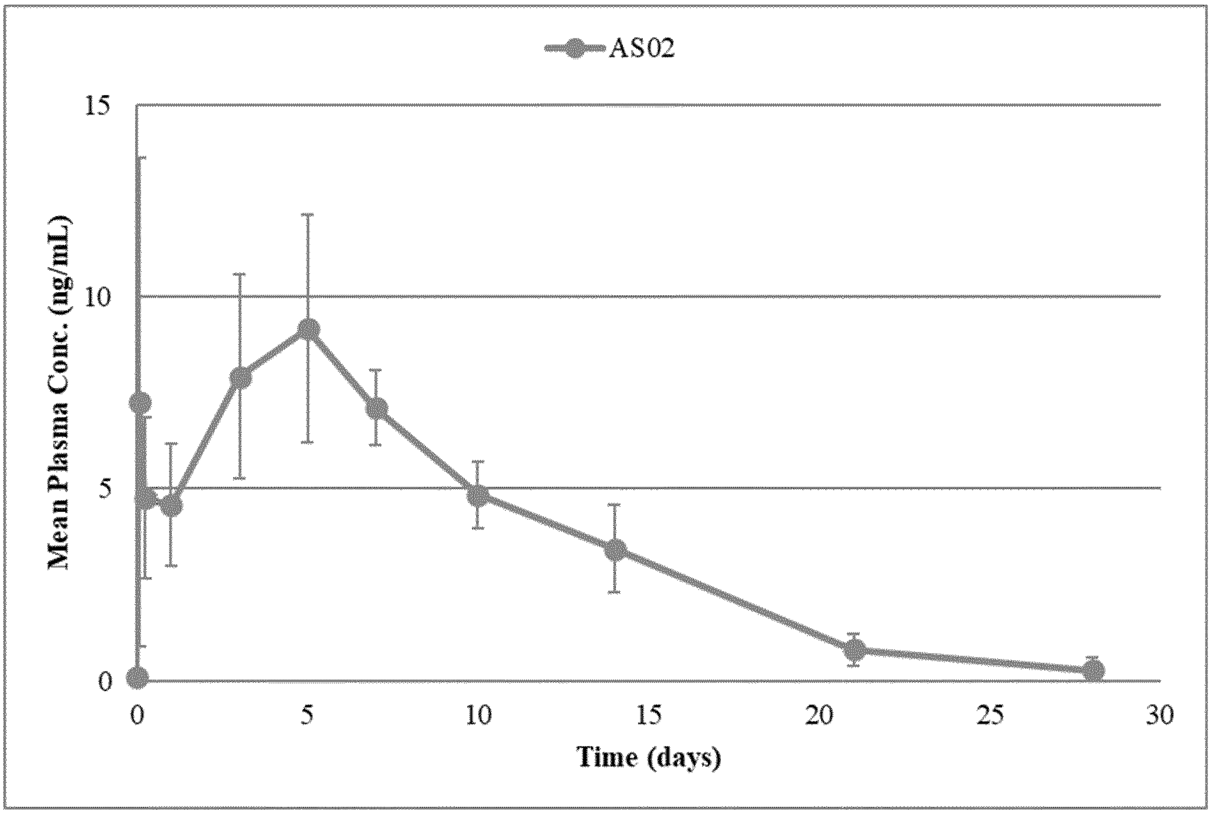


FIG. 38

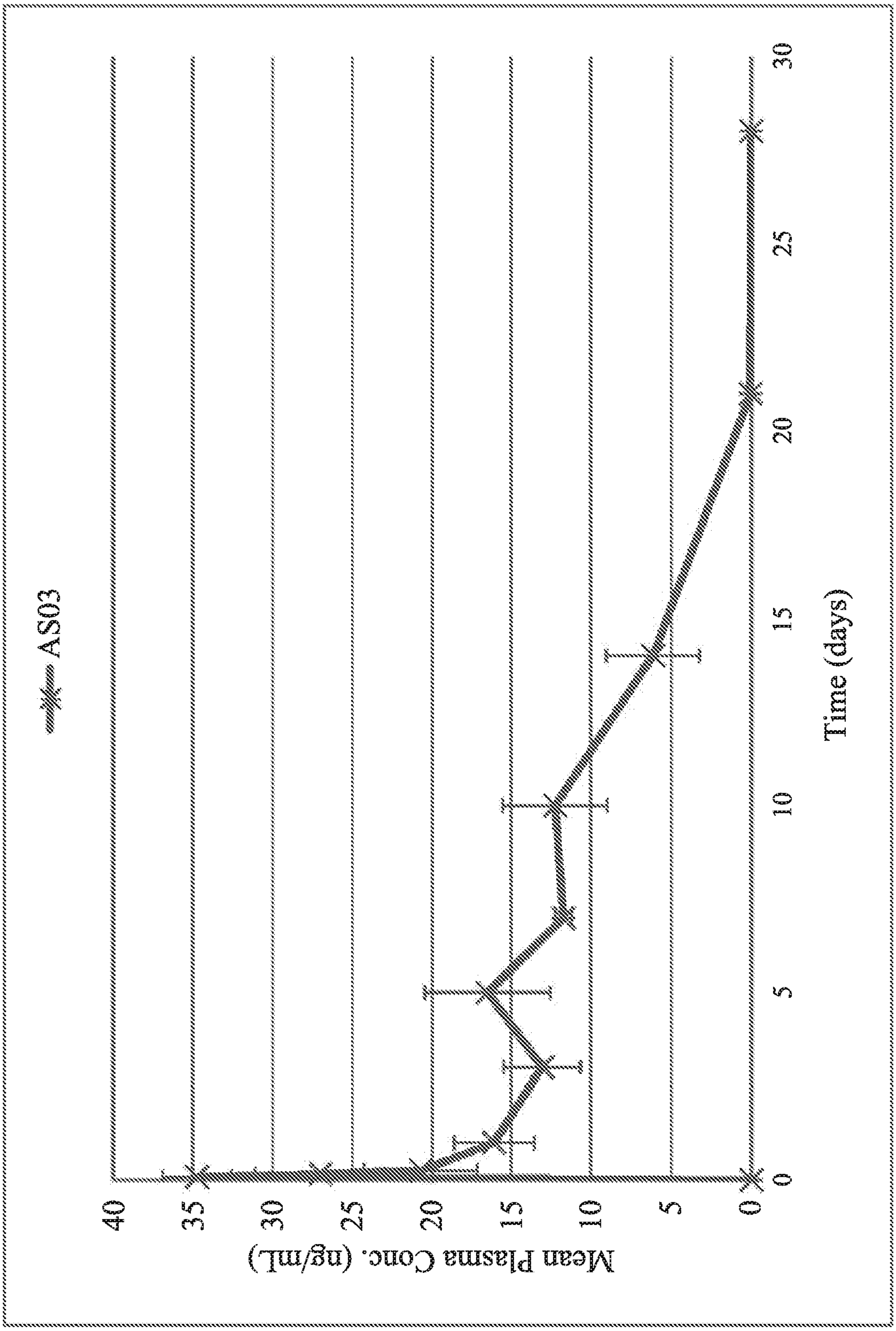
AS03
Mean Plasma Conc. (ng/mL)
0
5
10
15
20
25
30
35
40
Time (days)
0
5
10
15
20
25
30

FIG. 39

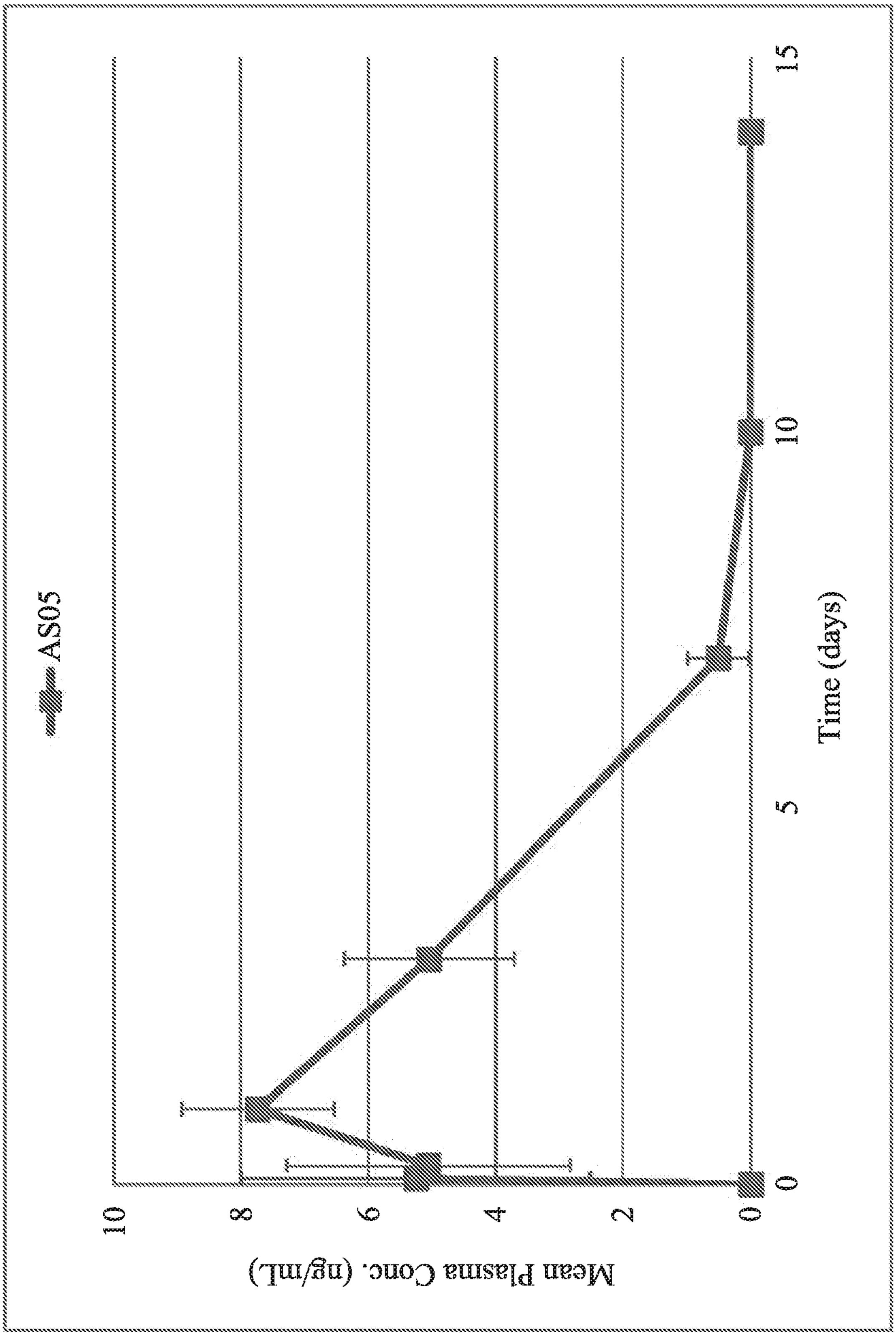


FIG. 40

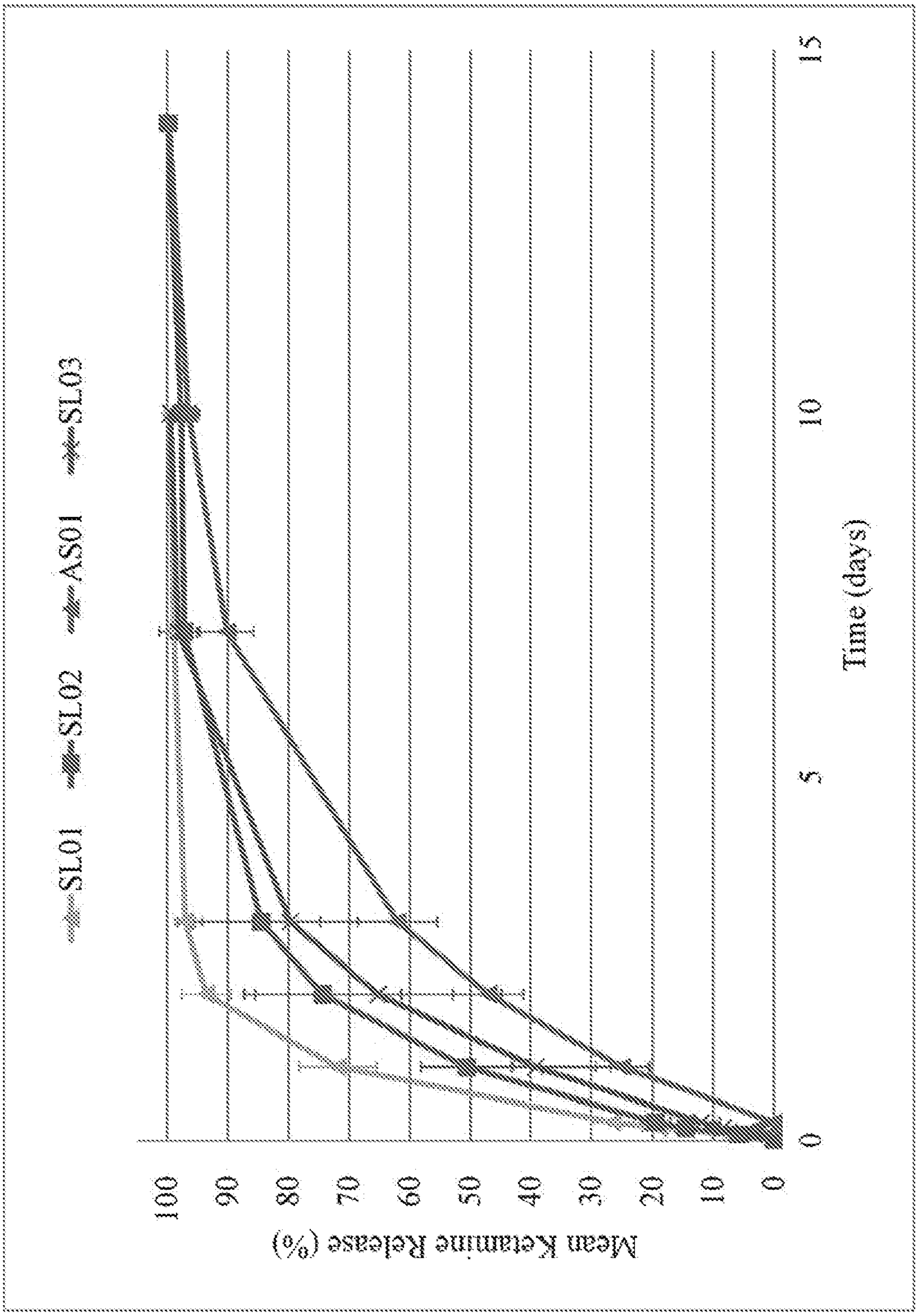
SL01
SL02
AS01
SL03
Mean Ketamine Release (%)
100
90
80
70
60
50
40
30
20
10
0
0
5
10
15
Time (days)

FIG. 41

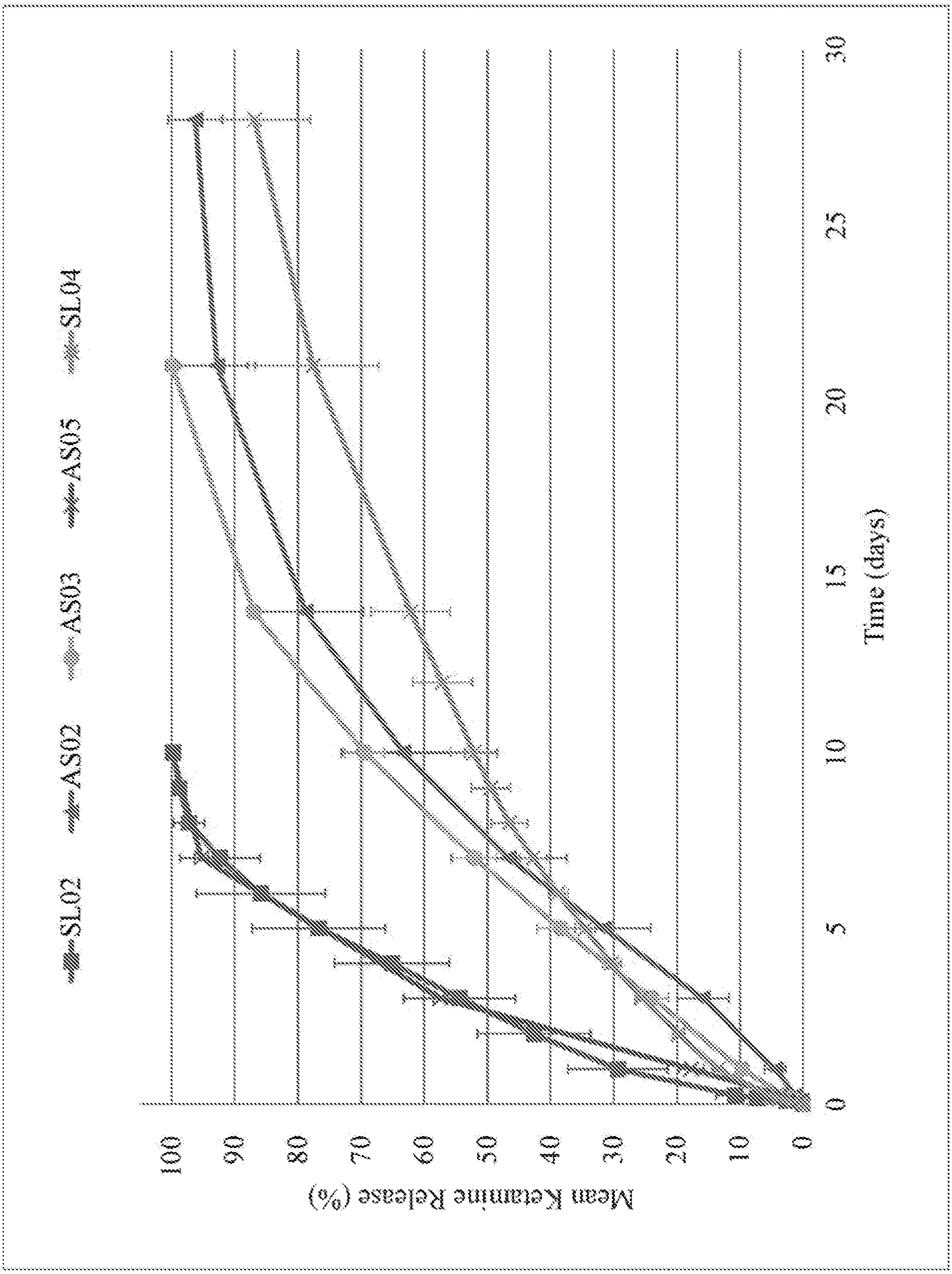
SL02
AS02
AS03
AS05
SL04
Mean Ketamine Release (%)
100
90
80
70
60
50
40
30
20
10
0
Time (days)
0
5
10
15
20
25
30

FIG. 42

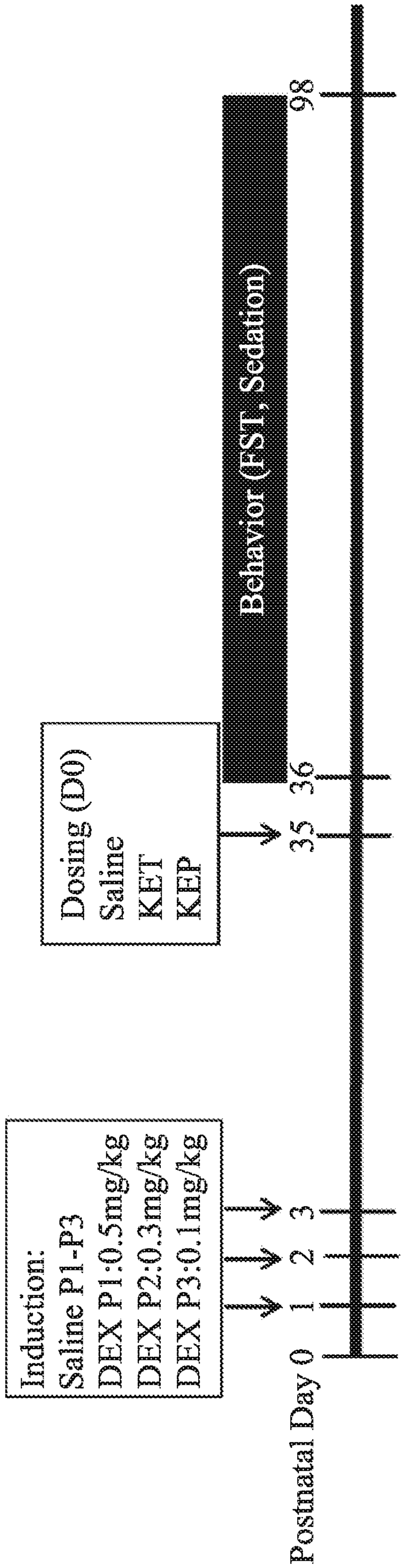
Induction:
Saline P1-P3
DEX P1:0.5mg/kg
DEX P2:0.3mg/kg
DEX P3:0.1mg/kg
Postnatal Day 0
1
2
3
Dosing (D0)
Saline
KET
KEP
35
36
Behavior (FST, Sedation)
98

FIG. 43

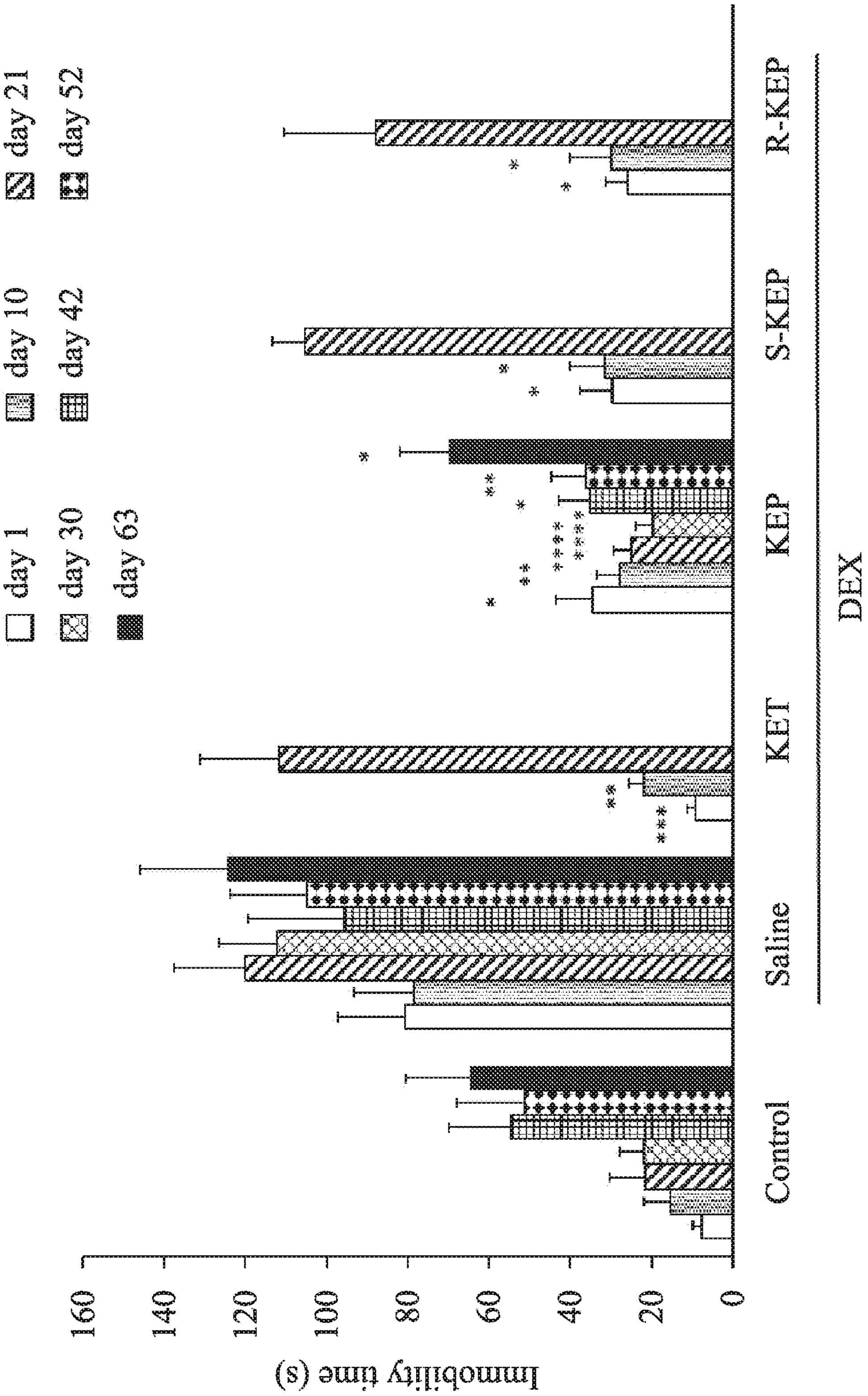
day 1
day 10
day 21
day 30
day 42
day 52
day 63
Immobility time (s)
0
20
40
60
80
100
120
140
160
Control
Saline
KET
KEP
S-KEP
R-KEP
DEX

FIG. 44

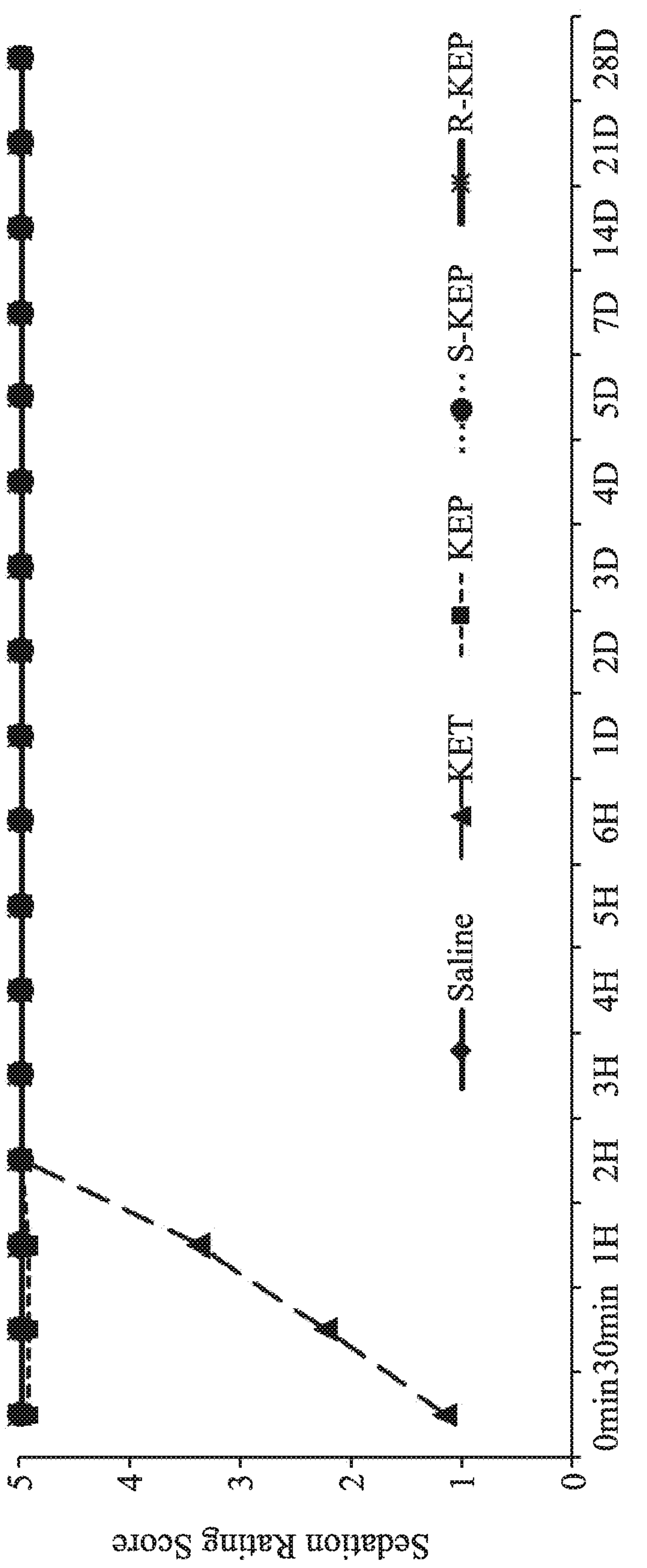
Sedation Rating Score
0
1
2
3
4
5
0min
30min
1H
2H
3H
4H
5H
6H
1D
2D
3D
4D
5D
7D
14D
21D
28D
Saline
KET
KEP
S-KEP
R-KEP

FIG. 45

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018122626 A1
- WO 2019186357 A1
- WO 2005016261 A2
- WO 2017003935 A1
- WO 2019073408 A1


### Non-patent literature cited in the description


- **HAN et al.** *Int. J. Pharm.*, 2020, vol. 581, 119291
- **HAN, FELICITY Y. et al.** Sustained-release ketamine-loaded nanoparticles fabricated by sequential nanoprecipitation. *International Journal of Pharmaceutics*, 2020, 119291
- **WAY, WALTER L.** Ketamine - its pharmacology and therapeutic uses.. *Anesthesiology: The Journal of the American Society of Anesthesiologists*, 1982, vol. 56.2, 119-136